(19) Europäisches Patentamt
European Patent Office
Office européen des brevets

(11) EP 4 397 671 A1

(12) **EUROPEAN PATENT APPLICATION**
published in accordance with Art. 153(4) EPC

(43) Date of publication:
**10.07.2024 Bulletin 2024/28**

(21) Application number: **22864576.8**

(22) Date of filing: **30.08.2022**

(51) International Patent Classification (IPC):
***C07K 2/00*** *(2006.01)* ***A61K 47/54*** *(2017.01)*
***A61K 49/00*** *(2006.01)* ***C07K 16/00*** *(2006.01)*
***C09K 11/06*** *(2006.01)* ***G01N 33/533*** *(2006.01)*

(52) Cooperative Patent Classification (CPC):
**A61K 47/54; A61K 49/00; C07K 2/00; C07K 16/00; C09K 11/06; G01N 33/533**

(86) International application number:
**PCT/JP2022/032639**

(87) International publication number:
**WO 2023/032994 (09.03.2023 Gazette 2023/10)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **31.08.2021 JP 2021141996**

(71) Applicant: **FUJIFILM Corporation**
**Tokyo 106-8620 (JP)**

(72) Inventors:
- **HAMADA, Naoka**
  **Ashigarakami-gun, Kanagawa 258-8577 (JP)**
- **SHIROKANE, Kenji**
  **Ashigarakami-gun, Kanagawa 258-8577 (JP)**
- **YOSHIMITSU, Yuji**
  **Ashigarakami-gun, Kanagawa 258-8577 (JP)**

(74) Representative: **HGF**
**HGF Limited**
**1 City Walk**
**Leeds LS11 9DX (GB)**

(54) **COMPOUND AND LABELED BIOMATERIAL USING SAME**

(57) There are provided a compound having a phosphor moiety and a structure represented by General Formula (I), and a labeled biological substance.

$$*\left(A^1-B\right)_p A^2-*$$ General Formula (I)

In the formula, $A^1$ represents a structure represented by General Formula (a1),
$A^2$ represents a structure represented by General Formula (a2), and
B represents a structure represented by General Formula (b),

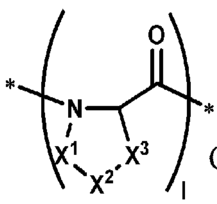
General Formula (a1)
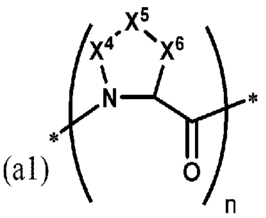
General Formula (a2)

EP 4 397 671 A1

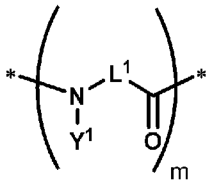

General Formula (b)

In the formulae, $X^1$ to $X^6$ represent -O-, -S-, >$NR^1$, or >$CR^2R^3$.

$R^1$ to $R^3$ represent a hydrogen atom, an alkyl group, an alkenyl group, an alkynyl group, an acyl group, an aryl group, a heteroaryl group, -$NR^9R^{10}$, -$OR^{11}$, or an anionic group.

$R^9$ to $R^{11}$ represent a hydrogen atom, an alkyl group, an alkenyl group, an alkynyl group, an acyl group, an aryl group, or a heteroaryl group.

$Y^1$ represents a hydrogen atom, an alkyl group, an alkenyl group, an alkynyl group, an aryl group, or a heteroaryl group.

$L^1$ represents a divalent linking group.

l, m, n, and p are an integer of 1 or more.

* represents a bonding site.

## Description

## BACKGROUND OF THE INVENTION

1. Field of the Invention

The present invention relates to a compound and a labeled biological substance using the compound.

2. Description of the Related Art

In order to observe in vivo changes in response to various stimuli (diseases, environmental changes, and the like), fluorescently labeled biological substances obtained by labeling a biological molecule (an antibody or the like) having a binding property to a target substance to be detected, with a fluorescent compound (a fluorescent dye), are often used.

For example, also in Western blotting (hereinafter, also abbreviated as WB) that detects a specific protein from a protein mixture, a fluorescence method in which the presence or absence or the abundance of the specific protein is detected using a fluorescently labeled antibody having a binding property to this protein is used.

In addition, in bioimaging technology for analyzing the dynamics and functions of biological molecules, cells, tissues, and the like in a living body, in vivo fluorescence imaging in which a specific portion of a living body visualized by fluorescence labeling is observed is used as one of the techniques for the living body observation.

In the fluorescence labeling, an organic fluorescent dye molecule is generally used, and the brightness (fluorescence intensity) is increased generally by using a fluorescently labeled biological substance to which a plurality of fluorescent dye molecules are bonded. However, since most of the organic dyes exhibiting fluorescence, such as a cyanine dye and a rhodamine dye, have an aromatic chromophore having high planarity, an interaction between the dyes easily occurs, and as a result, a decrease in the fluorescence intensity after labeling due to an interaction such as self-association between the dyes easily occurs. In addition, as the number of molecules (degree of fluorescence labeling: DOL) of the fluorescent dye per one molecule of the biological molecule increases, the fluorescence intensity due to self-association or the like tends to further decrease.

As a labeling substance that is used for the fluorescence labeling, a compound having a structure in which a long-chain molecule is bonded to a dye molecule is known. In addition, also in a field different from fluorescence labeling, similarly, a dye or the like is bonded by using a long-chain molecule to form a tag or a probe.

For example, "Synthesis of Polyproline Spacers between NIR Dye Pairs for FRET to Enhance Photoacoustic Imaging" discloses that a near-infrared fluorescent dye (NIRF dye) and a near-infrared quencher dye (NIRQ dye) are bonded to each other with a polyproline linker. According to the technique described in "Synthesis of Polyproline Spacers between NIR Dye Pairs for FRET to Enhance Photoacoustic Imaging", it is said that the distance between the NIRF dye and the NIRQ dye is adjusted with the above-described linker, whereby fluorescence is quenched by fluorescence resonance energy transfer (FRET), and the sensitivity of detection by photoacoustic imaging can be improved.

In addition, JP2017-512763A discloses an amphipathic nanoparticle having a target directivity that is specific to a biomarker (an in vivo substance that serves as a physiological indicator for a change in a disease state or the like), which is used for diagnosis and/or medical treatment, where the amphipathic nanoparticle contains a block copolymer (A) containing a hydrophilic and hydrophobic polymer and a block copolymer (B) containing a peptide that is decomposed by a proteolytic enzyme and containing a hydrophobic polymer. JP2017-512763A discloses that proline can be used as a component that constitutes a hydrophobic polymer and that the nanoparticle may contain a fluorescent substance.

JP2010-512400A discloses a compound for diagnostic and/or therapeutic application, where the compound contains a peptide capable of binding to fibrin and has a structure derived from proline in this peptide chain. According to the technique described in JP2010-512400A, it is said that this compound may include a fluorescent dye.

## SUMMARY OF THE INVENTION

An object of the present invention is to provide a dye compound that exhibits an excellent fluorescence intensity in both usage forms of an application to a solution and an application to a membrane, in a case where it is used as a labeling substance for a biological substance or the like. In addition, another object of the present invention is to provide a labeled biological substance obtained by bonding the compound to a biological substance.

That is, the above objects of the present invention have been achieved by the following means.

[1] A compound comprising:

a phosphor moiety; and

a structure represented by General Formula (I),

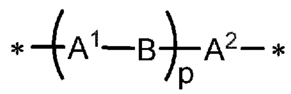

General Formula (I)

in the formula, $A^1$ represents a structure represented by General Formula (a1),
$A^2$ represents a structure represented by General Formula (a2), and
B represents a structure represented by General Formula (b),

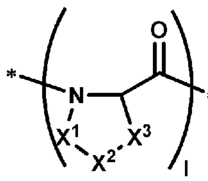

General Formula (a1)

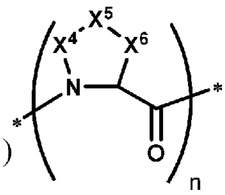

General Formula (a2)

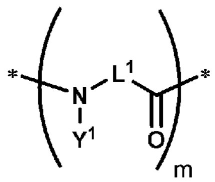

General Formula (b)

in the formulae, $X^1$ to $X^6$ represent -O-, -S-, >$NR^1$ or >$CR^2R^3$,
$R^1$ to $R^3$ represent a hydrogen atom, an alkyl group, an alkenyl group, an alkynyl group, an acyl group, an aryl group, a heteroaryl group, $-NR^9R^{10}$, $-OR^{11}$, or an anionic group,
$R^9$ to $R^{11}$ represent a hydrogen atom, an alkyl group, an alkenyl group, an alkynyl group, an acyl group, an aryl group, or a heteroaryl group,
$Y^1$ represents a hydrogen atom, an alkyl group, an alkenyl group, an alkynyl group, an aryl group, or a heteroaryl group,
$L^1$ represents a divalent linking group,
l, m, n, and p are an integer of 1 or more, and
* represents a bonding site.

[2] The compound according to [1], in which the compound is represented by General Formula (II),

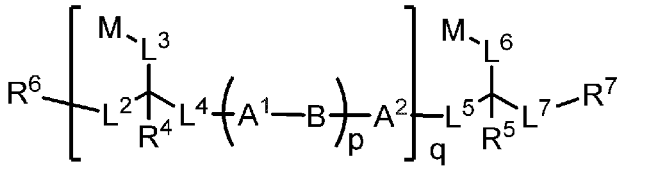

General Formula (II)

in the formula, $R^4$ and $R^5$ represent a hydrogen atom, an alkyl group, an alkenyl group, an alkynyl group, an aryl group, or a heteroaryl group,
$R^6$ and $R^7$ represent a hydrogen atom, a hydroxy group, a sulfanyl group, an alkyl group, an alkenyl group, an alkynyl group, an aryl group, an alkoxy group, an amino group, an acyl group, a heteroaryl group, an anionic group, a cationic group, or Q, where Q represents a carboxy group, a substituent capable of being bonded to a biological substance, or a substituent capable of being bonded to a solid support,
$L^2$ to $L^7$ represents a single bond or a divalent linking group,
M represents a phosphor moiety, a physiologically active substance moiety, a prodrug moiety, or a radioactive isotope-containing moiety,
q is an integer of 1 or more, and
$A^1$, $A^2$, B, and p have the same meanings as $A^1$, $A^2$, B, and p described above,
provided that at least one of M's represents a phosphor moiety.

[3] The compound according to [1] or [2], in which at least one of l or n described above is an integer of 3 or more.
[4] The compound according to any one of [1] to [3], in which the $L^1$ is a linking group in which the number of shortest-distance atoms that connect $>NY^1$ to $>C=O$ in General Formula (b) is 1 to 4.
[5] The compound according to any one of [1] to [4], in which the $L^1$ is a linking group including a ring structure.
[6] The compound according to [5], in which the ring structure is a group having a polyalkyleneoxy group as a substituent.
[7] The compound according to any one of [1] to [6], in which a ClogP value of the structure represented by General Formula (b) is 1.0 or less.
[8] The compound according to any one of [1] to [7], in which the B is a structure represented by General Formula (c),

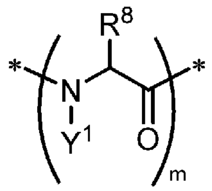

General Formula (c)

in the formula, $R^8$ represents a hydrogen atom or an alkyl group,
$Y^1$ and m respectively have the same meanings as $Y^1$ and m described above, and
* represents a bonding site.

[9] The compound according to [8], in which $R^8$ is an alkyl group having, as a substituent, any one of an anionic group, a cationic group, or a polyalkyleneoxy group.
[10] The compound according to any one of [1] to [9], in which the p is an integer of 1.
[11] The compound according to [9], in which the $R^8$ is an alkyl group having a polyalkyleneoxy group as a substituent.
[12] A labeled biological substance that is obtained by bonding the compound according to any one of [1] to [11] to a biological substance.
[13] The labeled biological substance according to [12], in which the biological substance is any one of a protein, an amino acid, a nucleic acid, a nucleotide, a sugar chain, or a phospholipid.

The compound according to the aspect of the present invention can exhibit an excellent fluorescence intensity in both usage forms of an application to a solution and an application to a membrane, in a case where it is used as a label for a biological substance or the like. In addition, the labeled biological substance according to the aspect of the present invention exhibits an excellent fluorescence intensity in both usage forms of an application to a solution and an application to a membrane.

## DESCRIPTION OF THE PREFERRED EMBODIMENTS

In the present invention, in a case where there is a plurality of substituents, linking groups, structural units, or the like (hereinafter, referred to as substituents or the like), which are represented by a specific symbol or Formula, or in a case where a plurality of substituents or the like are regulated at the same time, the substituents or the like may be the same or different from each other, unless otherwise specified. The same applies to the regulation of the number of substituents or the like. In addition, in a case where a plurality of substituents or the like come close to each other (particularly in a case where they are adjacent to each other), they may be linked to each other to form a ring, unless otherwise specified. In addition, unless otherwise specified, rings such as an alicyclic ring, an aromatic ring, and a heterocyclic ring may be fused to form a fused ring.

For example, in the present invention, a structure represented by General Formula (a1) described below means that l (l is an integer of 1 (one) or more) pieces of structures represented by General Formula (i) are connected. In this case, the l pieces of structures represented by General Formulae (i) may be the same or different from each other. It is noted that $X^1$ to $X^3$ in General Formula (i) respectively have the same meanings as $X^1$ to $X^3$ in General Formula (a1) described later. The same applies to a structure parenthesized in $( )_m$, a structure parenthesized in $( )_n$, and a structure parenthesized in $( )_p$, as well as a structure parenthesized in $[ ]_q$, where m pieces of structures may be the same or different from each other, n pieces of structures may be the same or different from each other, p pieces of structures may be the same or different from each other, and q pieces of structures may be the same or different from each other. In addition, in a case where a plurality of structures represented by General Formula (I) are included in a compound, a plurality of $A^1$'s may be the same or different from each other, a plurality of $A^2$'s may be the same or different from each other, and a plurality of B's may be the same or different from each other.

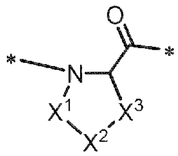

General Formula (i)

In the present invention, in a case where an E type and a Z type of a double bond are present in a molecule, any one of the E type or the Z type, or a mixture thereof may be used unless otherwise specified. In addition, unless otherwise specified, in a case where a chiral carbon atom or a chiral center is present in a compound, the steric conformation may be any of R or S in the R-S notation, and a mixture thereof may be may be allowed.

In the present invention, the denotation of a compound or substituent is meant to include not only the compound itself but also a salt thereof, and an ion thereof. For example, the dissociable anionic group such as the carboxy group, the sulfo group, and the phosphono group ($-P(=O)(OH)_2$) may have an ionic structure by a hydrogen ion being dissociated therefrom, or they may have a salt structure. That is, in the present invention, the "carboxy group" is meant to include a group of an ion or salt of a carboxylic acid, the "sulfo group" is meant to include a group of an ion or salt of a sulfonic acid, and the "phosphono group" is meant to include a group of an ion or salt of a phosphonic acid. The monovalent or polyvalent cation in forming the salt structure is not particularly limited. Examples thereof include an inorganic cation and an organic cation, and specific examples thereof include alkali metal cations such as $Na^+$, $Li^+$, and $K^+$, alkaline earth metal cations such as $Mg^{2+}$, $Ca^{2+}$, and $Ba^{2+}$, and organic ammonium cations such as a trialkylammonium cation and a tetraalkylammonium cation.

In a case of a salt structure, the kind of the salt may be one kind, two or more kinds thereof may be mixed, a salt-type group and a group having a free acid structure may be mixed in a compound, and a compound having a salt structure and a compound having a free acid structure compound may be mixed.

Any compound according to the embodiment of the present invention is a neutral compound. In the present invention, the fact that the compound is neutral means that the compound is electrically neutral. Specifically, the charge of the compound as a whole is adjusted to be 0 by a group having a charge or by a counterion in the compound. For example, in the cyanine dye represented by General Formula ($\alpha$), which is exemplified as an example of the dye that constitutes the phosphor moiety, the formal charge of the nitrogen atom to which $R^{42}$ is bonded is +1, and the dissociable group such as a sulfo group in another structure of the cyanine dye or the compound according to the embodiment of the present invention has an ionic structure such as a sulfonate ion to be paired with this formal charge, whereby the compound according to the embodiment of the present invention is to be a compound having a charge of 0 as a whole.

In each general formula pertaining to the cyanine dye, which is defined in the present invention, the positive charge possessed by the compound is specified and indicated, for convenience, as a structure of a specific nitrogen atom. However, since the cyanine dye defined in the present invention has a conjugated system, another atom other than the nitrogen atom actually may be capable of being positively charged, and thus any cyanine dye capable of adopting a structure represented by each general formula as one of the chemical structures is included in the cyanine dye represented by each general formula. This also applies to the negative charge.

In addition, it is meant to include those in which a part of the structure is changed within the scope that does not impair the effect of the present invention. Further, it is meant that a compound, which is not specified to be substituted or unsubstituted, may have any substituent within the scope that does not impair the effect of the present invention. The same applies to a substituent (for example, a group represented by "alkyl group", "methyl group", "methyl") and a linking group (for example, a group represented by "alkylene group", "methylene group", "methylene"). Among such substituents, a preferred substituent in the present invention is a substituent selected from a substituent group T described later.

In the present invention, in a case where the number of carbon atoms of a certain group is specified, this number of carbon atoms means the number of carbon atoms of the entire group thereof unless otherwise specified in the present invention or the present specification. That is, in a case where this group has a form that further has a substituent, it means the total number of carbon atoms, to which the number of carbon atoms of this substituent is included.

In addition, in the present invention, the numerical range represented by using "to" means a range including the numerical values before and after "to" as the lower limit value and the upper limit value, respectively.

The compound according to the embodiment of the present invention is a compound having a phosphor moiety and a structure represented by General Formula (I) described later. Although the details of the reason why the compound according to the embodiment of the present invention makes it possible to obtain a labeled biological substance exhibiting an excellent fluorescence intensity in any state of being in a solution, a membrane, or a dot blot are not clear, they can be conceived as follows.

The structure contained in the compound according to the embodiment of the present invention, which is represented by General Formula (I) described later, is a relatively rigid structure which has $A^1$ and $A^2$ that are structures consisting of a repeating unit (repetition number: 1 or more) containing a nitrogen-containing saturated 5-membered ring, such as a ring structure derived from proline, and in which B that is a structure consisting of a repeating unit

(repetition number: 1 or more) different from $A^1$ and $A^2$ links between $A^1$ and $A^2$ and furthermore $A^1$'s in a case where a plurality of $A^1$'s are present. It is conceived that due to having this structure having a relatively rigid structure represented by General Formula (I) and a structure having a phosphor moiety, the compound according to the embodiment of the present invention can effectively suppress the quenching due to the intramolecular association of phosphor moieties or the intermolecular association thereof in a case of having two or more phosphor moieties are contained. As a result, a labeled biological substance that is obtained by using the compound according to the embodiment of the present invention can exhibit an excellent fluorescence intensity in any usage form of a solution, a membrane, or a dot blot. Among the above, it is conceived that since the structure of B is provided in the labeled biological substance obtained by using the compound according to the embodiment of the present invention, the aggregation of $A^1$ and $A^2$ due to the hydrophobic interaction can be suppressed, and as a result, a more excellent fluorescence intensity can be exhibited in a state of being in a dot blot as compared with a case of using a compound that does not have the structure B in the structure represented by General Formula (I).

Hereinafter, the compound according to the embodiment of the present invention will be described in detail.

<Compound according to embodiment of present invention>

The compound according to the embodiment of the present invention has a phosphor moiety and a structure represented by General Formula (I).

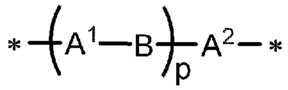

General Formula (I)

In the formula, $A^1$ represents a structure represented by General Formula (a1),

$A^2$ represents a structure represented by General Formula (a2), and
B represents a structure represented by General Formula (b),

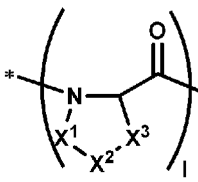

General Formula (a1)

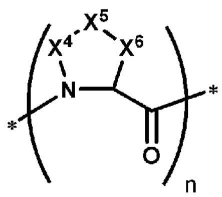

General Formula (a2)

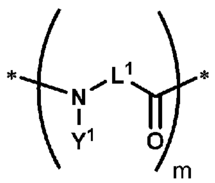

General Formula (b)

In the formulae, $X^1$ to $X^6$ represent -O-, -S-, >$NR^1$ or >$CR^2R^3$.

$R^1$ to $R^3$ represent a hydrogen atom, an alkyl group, an alkenyl group, an alkynyl group, an acyl group, an aryl group, a heteroaryl group, $-NR^9R^{10}$, $-OR^{11}$, or an anionic group.
$R^9$ to $R^{11}$ represent a hydrogen atom, an alkyl group, an alkenyl group, an alkynyl group, an acyl group, an aryl group, or a heteroaryl group.
$Y^1$ represents a hydrogen atom, an alkyl group, an alkenyl group, an alkynyl group, an aryl group, or a heteroaryl group.
$L^1$ represents a divalent linking group.
l, m, n, and p are an integer of 1 or more.
* represents a bonding site.

It is noted that as described above, the structure represented by General Formula (b) is a structure different from both the structure represented by General Formula (a1) and the structure represented by General Formula (a2). That is, although $L^1$ and $Y^1$ in General Formula (b) may be bonded to each other to form a ring, the ring to be formed is not allowed to be a 5-membered ring that has a nitrogen atom, $X^1$ to $X^3$, and a carbon atom in General Formula (a1)

as a ring-constituting atom and is not allowed to be a 5-membered ring that has a nitrogen atom, $X^4$ to $X^6$, and a carbon atom in General Formula (a2) as a ring-constituting atom.

It suffices that the phosphor moiety is contained in the compound according to the embodiment of the present invention, in a state of being covalently bonded, and the phosphor moiety may be bonded as a group that is contained in any substituent in the structure represented by General Formula (I) and may be bonded to the bonding site * in General Formula (I), directly or through a linking group, as a structure different from the structure represented by General Formula (I). Examples of the form in which the phosphor moiety is bonded to the bonding site * in General Formula (I) directly or through a linking group include a compound represented by General Formula (II) described later.

It suffices that at least one phosphor moiety is contained in the compound according to the embodiment of the present invention. It is preferable that two or more phosphor moieties are contained.

The description, the specific examples, and the like of the phosphor moiety in the compound represented by General Formula (II) described later can be applied to the phosphor moiety.

In the present invention, the structure represented by General Formula (a1) is preferably a structure represented by any one of General Formula (IA) or (IB) in consideration of stereoisomers. It is noted that $X^1$ to $X^3$ and l in the following general formula respectively have the same meanings as $X^1$ to $X^3$ and l in General Formula (a1). Similarly, the structure represented by General Formula (a2) is preferably a structure represented by any one of General Formula (IA) or (IB) in consideration of stereoisomers. In this case, $X^1$ to $X^3$ and l in General Formulae (IA) and (IB) respectively have the same meanings as $X^4$ to $X^6$ and n in General Formula (a2).

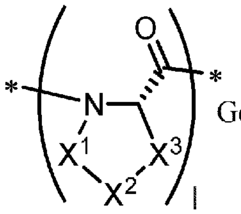 General Formula (IA) 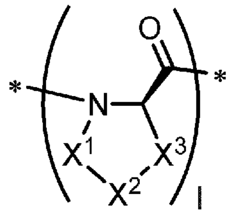 General Formula (IB)

$X^1$ to $X^6$ represent -O-, -S-, $>NR^1$ or $>CR^2R^3$. $R^1$ to $R^3$ represent a hydrogen atom, an alkyl group, an alkenyl group, an alkynyl group, an acyl group, an aryl group, a heteroaryl group, $-NR^9R^{10}$, $-OR^{11}$, or an anionic group.

The alkyl group, the alkenyl group, the alkynyl group, the acyl group, the aryl group, the heteroaryl group, and the anionic group, which can be adopted as $R^1$ to $R^3$, respectively have the same meanings as the alkyl group, the alkenyl group, the alkynyl group, the acyl group, the aryl group, the heteroaryl group, and the anionic group in the substituent group T which will be described later, and the same also applies to the preferred ranges thereof.

The alkyl group, the alkenyl group, the alkynyl group, the acyl group, the aryl group, and the heteroaryl group, which can be adopted as $R^1$ to $R^3$, may be unsubstituted or may have a substituent.

Examples of the substituent which may be contained in the alkyl group, the alkenyl group, the alkynyl group, the acyl group, the aryl group, and the heteroaryl group, as $R^1$ to $R^3$, include substituents in the substituent group T which will be described later. For example, a halogen atom is preferable.

In $-NR^9R^{10}$ and $-OR^{11}$, which can be adopted as $R^1$ to $R^3$, $R^9$ to $R^{11}$, represent a hydrogen atom, an alkyl group, an alkenyl group, an alkynyl group, an acyl group, an aryl group, or a heteroaryl group.

The alkyl group, the alkenyl group, the alkynyl group, the acyl group, the aryl group, and the heteroaryl group, which can be adopted as $R^9$ to $R^{11}$, respectively have the same meanings as the alkyl group, the alkenyl group, the alkynyl group, the acyl group, the aryl group, and the heteroaryl group in the substituent group T which will be described later, and the same also applies to the preferred ranges thereof.

The alkyl group, the alkenyl group, the alkynyl group, the acyl group, the aryl group, and the heteroaryl group, which can be adopted as $R^9$ to $R^{11}$, may be unsubstituted or may have a substituent.

Examples of the substituent which may be contained in the alkyl group, the alkenyl group, the alkynyl group, the acyl group, the aryl group, and the heteroaryl group, as $R^9$ to $R^{11}$, include substituents in the substituent group T which will be described later. For example, a halogen atom is preferable.

$R^9$ to $R^{11}$ are preferably a hydrogen atom or an alkyl group.

$R^1$ to $R^3$ are preferably a hydrogen atom, an alkyl group, $-NR^8R^9$, $-OR^{10}$, or an anionic group, more preferably a hydrogen atom, an alkyl group, $-NR^8R^9$, or $-OR^{10}$, and still more preferably a hydrogen atom.

Regarding $X^1$ to $X^3$, it is preferable that at least any one thereof is $>CR^2R^3$, it is more preferable that at least two thereof are $>CR^2R^3$, and it is still more preferable that all thereof are $>CR^2R^3$.

Regarding $X^4$ to $X^6$, it is preferable that at least any one thereof is $>CR^2R^3$, it is more preferable that at least two thereof are $>CR^2R^3$, and it is still more preferable that all thereof are $>CR^2R^3$.

It is noted that $X^1$ to $X^6$ may have or may not have the phosphor moiety.

The alkyl group, the alkenyl group, the alkynyl group, the aryl group, and the heteroaryl group, which can be

adopted as $Y^1$, respectively have the same meanings as the alkyl group, the alkenyl group, the alkynyl group, the aryl group, and the heteroaryl group in the substituent group T which will be described later, and the same also applies to the preferred ranges thereof.

Examples of the substituent which may be contained in the alkyl group, the alkenyl group, the alkynyl group, the aryl group, and the heteroaryl group, as $Y^1$, include substituents in the substituent group T which will be described later. For example, a halogen atom is preferable.

$Y^1$ is preferably a hydrogen atom.

The divalent linking group $L^1$ is preferably a linking group obtained by combining one or two or more among groups of an alkylene group, an alkenylene group, an alkynylene group, a divalent ring group, -O-, >$NR^{a1}$, >C=O, >S=O, $-S(=O)_2-$, and >P(=O)$OR^{a2}$. $R^{a1}$ and $R^{a2}$ represent a hydrogen atom or a monovalent substituent.

The alkylene group that can constitute $L^1$ has the same meaning as the group in which one hydrogen atom is further removed from the alkyl group selected from the substituent group T, which is described later, and the same applies to the preferred one thereof. The alkylene moiety (that is, a moiety excluding the substituent moiety contained in the alkylene group) preferably has 1 to 30 carbon atoms, more preferably has 1 to 20 carbon atoms, and still more preferably has 1 to 15 carbon atoms.

The alkenylene group that can constitute $L^1$ has the same meaning as the group in which one hydrogen atom is further removed from the alkenyl group selected from the substituent group T, which is described later, and the same applies to the preferred one thereof. The alkenylene moiety (that is, a moiety excluding the substituent moiety contained in the alkenylene group) preferably has 2 to 30 carbon atoms, more preferably has 2 to 20 carbon atoms, and still more preferably has 2 to 15 carbon atoms.

The alkynylene group that can constitute $L^1$ has the same meaning as the group in which one hydrogen atom is further removed from the alkynyl group selected from the substituent group T, which is described later, and the same applies to the preferred one thereof. The alkynylene moiety (that is, a moiety excluding the substituent moiety contained in the alkynylene group) preferably has 2 to 30 carbon atoms, more preferably has 2 to 20 carbon atoms, and still more preferably has 2 to 15 carbon atoms.

Each of the alkylene group, the alkenylene group, and the alkynylene group, which can constitute $L^1$, may be linear or may be branched.

The divalent cyclic group that can constitute $L^1$ has the same meaning as the cycloalkylene group, the cycloalkenylene group, the arylene group, the heteroarylene group, or the divalent aliphatic heterocyclic group, which is a group obtained by further removing one hydrogen atom from a ring group (that is, a cycloalkyl group, a cycloalkenyl group, an aryl group, or a heterocyclic group) in the substituent group T which will be described later, and the same applies to the preferred range thereof.

The arylene group that can constitute $L^1$ is such that the arylene moiety (that is, a moiety excluding the substituent moiety contained in the arylene group) preferably has 6 to 22 carbon atoms, more preferably has 6 to 18 carbon atoms, and still more preferably has 6 to 14 carbon atoms. The arylene group is preferably a phenylene group or a group obtained by removing two hydrogen atoms from phenanthrene.

The heteroarylene group that can constitute $L^1$ is such that the heteroarylene moiety (that is, a moiety excluding the substituent moiety contained in the heteroarylene group) preferably has 2 to 16 carbon atoms, more preferably has 2 to 12 carbon atoms, and still more preferably has 2 to 8 carbon atoms. The heteroarylene group is preferably a 5- or 6-membered heteroarylene group having >NH as a ring-constituting atom, and it is more preferably a divalent group obtained by removing two hydrogen atoms from a pyrrole ring, an imidazole ring, or a triazole ring.

The divalent ring group that can constitute $L^1$ is preferably an arylene group or a heteroarylene group.

The monovalent substituent which can be adopted as $R^{a1}$ and $R^{a2}$ are preferably an alkyl group, an alkenyl group, an alkynyl group, an aryl group, or a heteroaryl group. The alkyl group, the alkenyl group, the alkynyl group, the aryl group, and the heteroaryl group, which can be adopted as $R^{a1}$ and $R^{a2}$, respectively have the same meanings as the alkyl group, the alkenyl group, the alkynyl group, the aryl group, and the heteroaryl group in the substituent group T which will be described later, and the same also applies to the preferred ranges thereof. These substituents may be further substituted with a group selected from the substituent group T.

$R^{a1}$ and $R^{a2}$ are preferably a hydrogen atom or an alkyl group and more preferably a hydrogen atom.

The divalent linking group $L^1$ may be unsubstituted or may have a substituent. That is, the alkylene group, the alkenylene group, the alkynylene group, the divalent ring group, >$NR^{a1}$, and >P(=O)$OR^{a2}$, which can constitute $L^1$ in the preferred form may also be unsubstituted or may have a substituent

Examples of the substituent which may be contained in $L^1$ include substituents in the substituent group T described later, and it is preferably, for example, $-NR^{c1}R^{c2}$ ($R^{c1}$ and $R^{c2}$ represent a hydrogen atom or an alkyl group, where a hydrogen atom is preferable), an aryl group (preferably a phenyl group), a heteroaryl group (preferably a 5- or 6-membered heteroaryl group containing >NH as a ring-constituting atom, and more preferably an imidazole-yl group or an indole-yl group), a halogen atom, a hydroxy group, an alkoxycarbonyl group, an acyloxy group, a carbamoyl group, an acylamino group, a guanidino group (preferably $-NHC(=NH)NH_2$), an alkylthio group, a phenol group, a sulfanyl group,

an anionic group, a cationic group, or a polyalkyleneoxy group, or a group obtained by combining two or more of these substituents.

Among the substituents which may be contained in $L^1$, preferred examples of the group obtained by combining two or more of those described above include an aryl group, a heteroaryl group, a hydroxy group, a carbamoyl group, an acylamino group, an anionic group, or a group obtained by combining two or more kinds of anionic groups or polyalkyleneoxy groups, where an aryl group substituted with an anionic group, a cationic group, or a hydroxy group, a heteroaryl group substituted with an anionic group, a cationic group, or a hydroxy group, a carbamoyl group having a polyalkyleneoxy group as a substituent, or an acylamino group having a polyalkyleneoxy group as a substituent is more preferable.

It is noted that in the carbamoyl group having a polyalkyleneoxy group as a substituent, the polyalkyleneoxy group may be directly bonded to the carbamoyl group or may be bonded to the carbamoyl group through a linking group that is obtained by removing one hydrogen atom from a carbamoyl group or an acylamino group, or the like. Similarly, in the acylamino group having a polyalkyleneoxy group as a substituent, the polyalkyleneoxy group may be directly bonded to the acylamino group or may be bonded to the carbamoyl group through a linking group that is obtained by removing one hydrogen atom from an acylamino group or an acylamino group, or the like.

In the linking group obtained by combining one or two or more among an alkylene group, an alkenylene group, an alkynylene group, a divalent ring group, -O-, >$NR^{a1}$, >C=O, >S=O, -$S(=O)_2$-, and >P(=O)$OR^{a2}$, which can constitute $L^1$, the kind of group to be combined is not particularly limited as long as the group to be combined has a reasonable chemical structure.

In the linking group obtained by combining one or two or more among an alkylene group, an alkenylene group, an alkynylene group, a divalent ring group (a cycloalkylene group, a cycloalkenylene group, an arylene group, a heteroarylene group, or a divalent aliphatic heterocyclic group), -O-, >$NR^{a1}$, >C=O, >S=O, -$S(=O)_2$-, and >P(=O)$OR^{a2}$, which can constitute $L^1$, the kind of groups to be combined is not particularly limited; however, it is, for example, preferably 1 to 6, more preferably 1 to 4, and still more preferably 1 or 2. It is noted that in a case of counting, as one kind, each of the alkylene group, the alkenylene group, the alkynylene group, the cycloalkylene group, the cycloalkenylene group, the arylene group, the heteroarylene group, the divalent aliphatic heterocyclic group, -O-, >$NR^{a1}$, >C=O, >S=O, -$S(=O)_2$-, and >P(=O)$OR^{a2}$, the maximum number of kinds is 14.

The linking group obtained by combining two or more among an alkylene group, an alkenylene group, an alkynylene group, a divalent ring group, -O-, >$NR^{a1}$, >C=O, >S=O, -$S(=O)_2$-, and >P(=O)$OR^{a2}$, which can constitute $L^1$, is more preferably alkylene-arylene-alkylene, alkylene-heteroarylene-alkylene, alkylene-O-P(=O)$OR^{a2}$-alkylene, a repetition of alkylene-O- (-$(LL-O)_h$- described later), or heteroarylene-C(=O)-NH-alkylene-C(=O)-NH-heteroarylene-C(=O)-NH -alkylene-NH-.

The divalent linking group $L^1$ is preferably an alkylene group, an arylene group, or a heteroarylene group, or a group obtained by combining two or more thereof, more preferably an alkylene group, an arylene group, a heteroarylene group, alkylene-arylene-alkylene, or an alkylene-heteroarylene-alkylene, still more preferably an alkylene group, an arylene group, or a heteroarylene group, and particularly preferably an alkylene group. The divalent linking group $L^1$ is also preferably a group obtained by combining at least one of an alkylene group, an alkenylene group, an alkynylene group, or a divalent ring group, and at least one of -O-, >$NR^{a1}$, >C=O, or >P(=O)$OR^{a2}$, and it is more preferably alkylene-O-P(=O)$OR^{a2}$-alkylene, a repetition of alkylene-O- (-$(LL-O)_h$- described later), or heteroarylene-C(=O)-NH-alkylene-C(=O)-NH-heteroarylene-C(=O)-NH -alkylene-NH-.

It is noted that from the viewpoint of adjusting the Clog P value of the structure represented by General Formula (b) to a preferred range described later, $L^1$ also preferably has a specific substituent, and specifically, it preferably has -$NH_2$, a 5- or 6-membered heteroaryl group containing >NH as a ring-constituting atom, a hydroxy group, -C(=O)$NH_2$, -NHC(=NH)$NH_2$, an anionic group, a cationic group, or a polyalkyleneoxy group, and it more preferably has an anionic group, a cationic group, or a polyalkyleneoxy group.

The divalent linking group $L^1$ is preferably a linking group in which the number of shortest-distance atoms that connect >NY' to >C=O in General Formula (b) is 1 to 8, more preferably a linking group in which the number thereof is 1 to 6, and still more preferably a linking group in which the number thereof is 1 to 4. From the viewpoint of suppressing the association of the phosphor moieties, the above-described number of shortest-distance atoms in $L^1$ is preferably 1 or 2 and more preferably 1.

Regarding the divalent linking group $L^1$, "the number of shortest-distance atoms that connect >NY' to >C=O in General Formula (b)" means the number of atoms that constitute the shortest chain connecting >NY' to >C=O. For example, in a structure represented by General Formula (c), the number of atoms that constitute the shortest chain connecting >$NY^1$ to >C=O is "1". Details of General Formula (c) will be described in detail later.

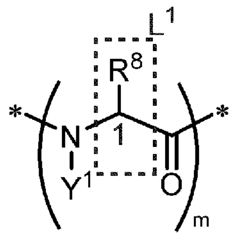

General Formula (c)

It is preferable that the $L^1$ is a linking group including a ring structure. Examples of the form in which $L^1$ is a linking group including a ring structure include (i) a form having a ring structure in the shortest chain that connects >NY’ to >C=O and (ii) a form having a ring structure at a position other than the shortest chain that connects >$NY^1$ to >C=O. Examples of the ring structure in the form of above (i) include the divalent ring group which can constitute $L^1$ described above. The form of the above (ii) corresponds to a form in which $L^1$ has a ring structure as a substituent, and specific examples thereof include an example in which $R^8$ in General Formula (c) described later has a ring structure.

Among the above, the form of the above (i) is preferable.

Regarding the structure represented by General Formula (b), two or more structures can be adopted depending on m and p (m, p, and q in General Formula (II) described later). Among the structures represented by General Formula (b) in the compound according to the embodiment of the present invention, the proportion of the number of structures in which $L^1$ is the above-described group including a ring structure is preferably 20% or more and more preferably 40% or more. The upper limit of the ratio is not particularly limited and may be 100% or less. It is noted that it is also preferable that all $L^1$'s in the structure represented by General Formula (b) are a group including a ring structure.

The ring structure is preferably a group having a polyalkyleneoxy group as a substituent, where the polyalkyleneoxy group may be directly bonded to the ring structure or may be bonded to through a linking group. Examples of the linking group include a group obtained by further removing a hydrogen atom from the group selected from the above-described substituents which may be contained in $L^1$, where an alkylene group is preferable.

It is preferable that the B has a structure represented by General Formula (c). The structure represented by General Formula (c) corresponds to a structure in which the number of shortest-distance atoms in $L^1$ in General Formula (b) (the number of shortest-distance atoms that connect >$NY^1$ to >C=O in General Formula (b) is 1.

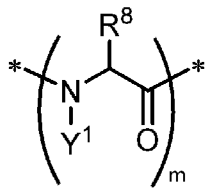

General Formula (c)

In the formula, $R^8$ represents a hydrogen atom or an alkyl group. $Y^1$ and m respectively have the same meanings as $Y^1$ and m in General Formula (b).

**[0078]** * represents a bonding site.

The alkyl group which can be adopted by $R^8$ has the same meaning as the alkyl group in the substituent group T which will be described later, and the same applies to the preferred range thereof.

The alkyl group which can be adopted by $R^8$ may be unsubstituted or may have a substituent.

Examples of the substituent which may be contained in the alkyl group as $R^8$ include substituents in the substituent group T described later, and the preferred description of the substituent which may be contained in $L^1$ described above can be applied thereto.

The substituent which may be contained in the alkyl group as $R^8$ is preferably an aryl group (preferably a phenyl group), a heteroaryl group (preferably, an imidazole-yl group or an indole-yl group), a hydroxy group, an amino group, (preferably $-NH_2$), a carbamoyl group, an acylamino group, a guanidino group (preferably $-NHC(=NH)NH_2$), an alkylthio group, a phenol group, a sulfanyl group, an anionic group (preferably a sulfo group, a phosphono group, or a phosphonooxy group), a cationic group, or a polyalkyleneoxy group, or a group obtained by combining two or more thereof.

To the group obtained by combining two or more of those described above among the substituent which may be contained in the alkyl group as $R^8$, the description pertaining to the group obtained by combining two or more of those described above, among the above-described substituents which may be contained in $L^1$, can be preferably applied.

It is preferable that $R^8$ is an alkyl group having, as a substituent, any one of an anionic group, a cationic group, or a polyalkyleneoxy group, where the anionic group, the cationic group, or the polyalkyleneoxy group may be directly added to the alkyl group or may be bonded through a linking group. Examples of the linking group include a group

obtained by further removing a hydrogen atom from the group selected from the above-described substituents which may be contained in the alkyl group as $R^8$, where a carbamoyl group or an acylamino group, or a group obtained by combining these is preferable.

Similar to $L^1$, $R^8$ preferably has a specific substituent from the viewpoint of adjusting the Clog P value of the structure represented by General Formula (c) to a preferred range described later, and also in terms of being contained in $L^1$ described above, the specific group has the same meaning as that in the description of the preferred specific substituent.

Among the above, from the viewpoint of increasing the fluorescence intensity in a state of being in a solution, $R^8$ is preferably an alkyl group having an anionic group as a substituent, and from the viewpoint of increasing the fluorescence intensity in a case of an application to a solution and an application to a membrane (in a dry state), $R^8$ is preferably an alkyl group having a polyalkyleneoxy group as a substituent.

(ClogP Value)

The Clog P value of the structure represented by General Formula (b) is preferably 1.0 or less, more preferably 0.0 or less, and still more preferably -1.0 or less.

Here, the "Clog P value" shall be calculated using ChemDraw (registered trade name) Professional (ver. 16.0.1.4) manufactured by PerkinElmer Informatics. Specifically, the ClogP value of the structure represented by General Formula (b) shall be a value obtained by rounding off the second decimal place in the ClogP value that is calculated the structure represented by General Formula (b) is drawn with ChemDraw and a structure (a structure that constitutes a constitutional repeating unit) in a range surrounded by parentheses is selected, in a case where the structure represented by General Formula (b) is drawn with ChemDraw and a structure (a structure that constitutes a constitutional repeating unit) in a range surrounded by parentheses is selected.

Regarding the structure represented by General Formula (b), two or more structures can be adopted depending on m and p (m, p, and q in General Formula (II) described later). Among the structures represented by General Formula (b) in the compound according to the embodiment of the present invention, the proportion of the number of structures that satisfy the ClogP value is preferably 20% or more and more preferably 40% or more. The upper limit of the ratio is not particularly limited and may be 100% or less. It is noted that it is also preferable that all of the structures represented by General Formula (b) are structures that satisfy the Clog P value.

The descriptions pertaining to the preferred range of the ClogP value of the structure represented by General Formula (b) and the proportion of the structure satisfying the ClogP value can be also applied to the preferred range of the ClogP value of the structure represented by General Formula (c) described above and the proportion of the structure satisfying the ClogP value, respectively.

Specific examples of the structure represented by General Formula (b) are shown below together with the Clog P values thereof. However, the present invention is not limited to these structures.

In the following specific examples, * indicates a bonding site.

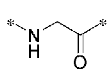
CLogP: -1.2

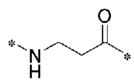
CLogP: -0.9

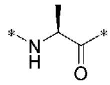
CLogP: -0.7

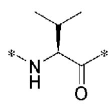
CLogP: 0.2

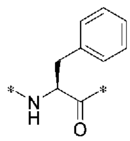
CLogP: 0.7

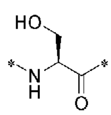
CLogP: -1.9

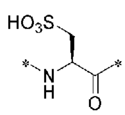
CLogP: -3.2

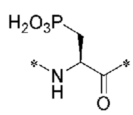
CLogP: -2.3

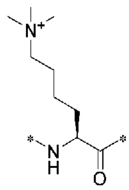
CLogP: -5.6

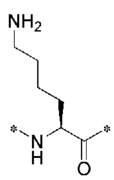
CLogP: -1.0

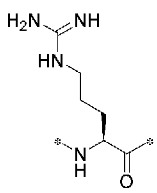
CLogP: -2.4

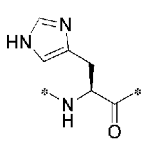
CLogP: -1.7

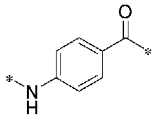
CLogP: 0.2

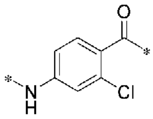
CLogP: 1.0

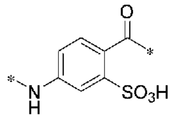
CLogP: -2.6

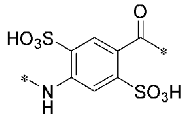
CLogP: -5.4

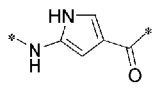
CLogP: -0.4

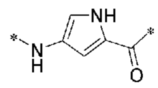
CLogP: -0.4

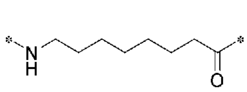
CLogP: 1.1

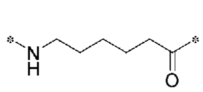
CLogP: 0.0

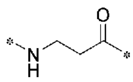
CLogP: -0.9

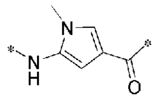
CLogP: 0.1

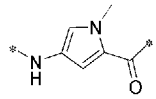
CLogP: 0.1

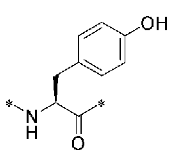
CLogP: 0.0

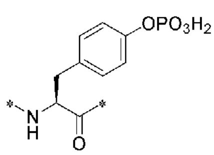
CLogP: -1.9

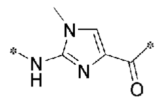
CLogP: -0.4

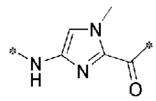
CLogP: -0.4

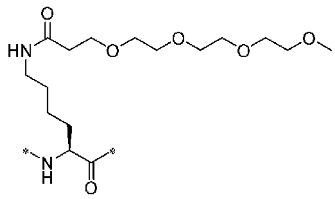
CLogP: -1.5

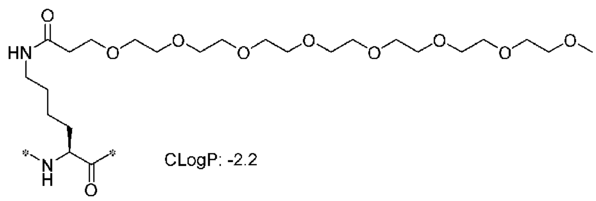
CLogP: -2.2

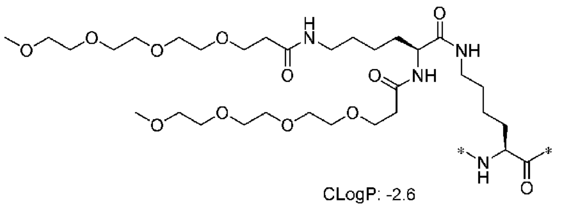
CLogP: -2.6

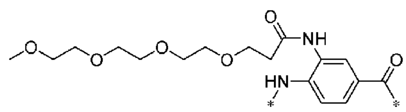
CLogP: -0.5

l, m, n, and p are an integer of 1 or more. All the numbers in the preferred ranges of l, m, n, and p described below are "integers".

l is preferably 2 or more and more preferably 5 or more. The upper limit of l is not particularly limited; however, it is preferably 30 or less, more preferably 25 or less, still more preferably 20 or less, and particularly preferably 15 or less, among which 10 or less is preferable.

m is preferably 1 to 4, more preferably 1 or 2, and still more preferably 1.

n is preferably 2 or more and more preferably 5 or more. The upper limit of n is not particularly limited; however, it is preferably 30 or less, more preferably 25 or less, still more preferably 20 or less, and particularly preferably 15 or less, among which 10 or less is preferable.

p is preferably 1 to 4 and more preferably 1 or 2, and it still more preferably 1 from the viewpoint of increasing the activity of the labeled biological substance to be obtained. It is noted that p is read as the maximum number which can be read in the compound.

From the viewpoint of suppressing the intramolecular and/or intermolecular association of the phosphor moieties and further improving the fluorescence intensity, it is preferable that at least one of l or n is an integer of 3 or more, and it is more preferable that both l and n are an integer of 3 or more.

In a case where two or more $A^1$'s are present in the compound, the proportion of the number of $A^1$'s in which l is an integer of 3 or more among the $A^1$'s in the compound of the present invention is not particularly limited as long as the above-described effect can be obtained; however, the proportion thereof, is preferably 40% or more, more preferably 60% or more, and still more preferably 80% or more. The upper limit of the ratio is not particularly limited and may be 100% or less. It is noted that it is also preferable that all of $A^1$'s in the compound according to the embodiment of the present invention are $A^1$'s in which l is an integer of 3 or more.

The same applies in a case where two or more $A^2$'s are present in the compound and applies to the description in the case where two or more $A^1$'s are present in the compound by reading $A^1$ as $A^2$ and reading l as n.

In the compound according to the embodiment of the present invention, it is conceived that even in a case where m is 2 or more, the association of the phosphor moieties is suppressed, whereby high fluorescence performance is exhibited in a case where the rigidity of the entire structure of General Formula (I) is maintained due to the structures of $A^1$ and $A^2$ in General Formula (I) (a case where an aromatic ring is contained or the like is preferable, for example, in a case where the structure of B is an $\alpha$-amino acid, where the case is not particularly limited).

The compound according to the embodiment of the present invention is also preferably a compound represented by General Formula (II).

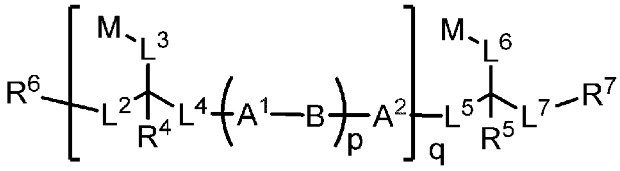

General Formula (II)

$R^4$ and $R^5$ represent a hydrogen atom, an alkyl group, an alkenyl group, an alkynyl group, an aryl group, or a heteroaryl group.

The alkyl group, the alkenyl group, the alkynyl group, the aryl group, and the heteroaryl group, which can be adopted as $R^4$ and $R^5$, respectively have the same meanings as the alkyl group, the alkenyl group, the alkynyl group, the aryl group, and the heteroaryl group in the substituent group T which will be described later, and the same also applies to the preferred ranges thereof.

The substituent which can be adopted as $R^4$ and $R^5$ may have a substituent, and examples of such a substituent include substituents in the substituent group T described later. For example, a halogen atom is preferable.

$R^4$ and $R^5$ are preferably a hydrogen atom.

$R^4$ and $R^5$ are often a hydrogen atom in a case where an amino acid is used as a raw material; however, the substituents in $R^4$ and $R^5$ do not greatly contribute to the exhibition of the excellent fluorescence intensity by the compound according to the embodiment of the present invention, and thus $R^4$ and $R^5$ may be another substituent (an alkyl group, an alkenyl group, an alkynyl group, an aryl group, or a heteroaryl group) other than the hydrogen atom.

$R^6$ and $R^7$ represent a hydrogen atom, a hydroxy group, a sulfanyl group, an alkyl group, an alkenyl group, an alkynyl group, an aryl group, an alkoxy group, an amino group, an acyl group, a heteroaryl group, an anionic group, a cationic group, $-(L-O)_tR^E$, or Q.

The alkyl group, the alkenyl group, the alkynyl group, the aryl group, the alkoxy group, the amino group, the acyl group, the heteroaryl group, the anionic group, the cationic group, and $-(L-O)_tR^E$, which can be adopted as $R^6$ or $R^7$, respectively have the same meanings as the alkyl group, the alkenyl group, the alkynyl group, the aryl group, the alkoxy group, the amino group, the acyl group, the heteroaryl group, the anionic group, the cationic group, and $-(L-O)_tR^E$, and the same also applies to the preferred ranges thereof.

The alkyl group, the alkenyl group, the alkynyl group, the aryl group, the alkoxy group, the amino group, the acyl group, and the heteroaryl group, which can be adopted as $R^6$ or $R^7$, may be unsubstituted or may have a substituent.

Examples of the substituent which may be contained in the alkyl group, the alkenyl group, the alkynyl group, the aryl group, the alkoxy group, the amino group, the acyl group, and the heteroaryl group, as $R^6$ or $R^7$, include a group obtained by combining one or two or more of substituents in the substituent group T described later, where an alkyl group, an acyl group, an alkoxy group, an amino group, a carbamoyl group, an acylamino group, a halogen atom, $-(L-O)_tR^E$, an anionic group, a cationic group, or Q, or a substituent obtained by combining two or more of these substituents is preferable, and an acyl group, an amino group, a carbamoyl group, an acylamino group, a halogen atom, $-(L-O)_tR^E$, a phosphono group, a phosphonooxy group, or Q, or a substituent obtained by combining two or more of these substituents is more preferable.

Q, which can be adopted as $R^6$ or $R^7$, represents a carboxy group, a substituent capable of being bonded to a biological substance, or a substituent capable of being bonded to a solid support.

As the substituent capable of being bonded to a biological substance, the description of the substituent capable of being bonded to a biological substance described later can be applied, and as the substituent capable of being bonded to a solid support, the description of the substituent capable of being bonded to a solid support described later can be applied.

$R^6$ is preferably an acyl group or $-C(=O)(L-O)_tR^E$ and more preferably an acyl group. It is noted that $R^6$ can be a group obtained by combining an alkyl group, and a phosphono group and/or a phosphonooxy group.

$R^7$ is preferably a group having a structure represented by *-NH-alkylene-C(=O)- or a group having a structure represented by $\text{*-NH-(LL-O)}_{h}$-. * means a bonding site to $L^7$. $-(LL-O)_h-$ has the same meaning as $-(LL-O)_h-$ described

later, except that the side on which $L^7$ is bonded is specified.

(Group having structure represented by *-NH-alkylene-C(=O)-)

The alkylene group in the structure represented by *-NH-alkylene-C(=O)- is preferably $>CHR^8$ in General Formula (c) described above. However, $R^8$ is preferably a hydrogen atom. In addition, a structure represented by -NH-alkylene-C(=O)- may be repeated, and the repetition number thereof (hereinafter, the repetition number is also referred to as w) is preferably 1 to 15, more preferably 1 to 10, and still more preferably 1 to 5.

$R^7$ preferably has a form which is a structure represented by $*\text{-}[NHCH_2C(=O)]_w\text{-}**$ (w is 1 to 5), where a terminal on a side represented by ** is a hydroxy group.

In a case where the terminal on the side represented by ** is Q, $R^7$ also preferably has a form in which $*\text{-}[NHCH_2C(=O)]_w\text{-}**$ and Q are linked by a linking group. In this case, the linking group is not particularly limited; however, examples thereof include >C=O, $>NR^{b1}$ ($R^{b1}$ is as described later and is preferably a hydrogen atom or an alkyl group), or an alkylene group, or a group obtained by combining two or more thereof, and preferred examples thereof include $-NR^{b1}$-alkylene. Such $R^7$ may have, for example, a form which is a structure represented by $*\text{-}[NHCH_2C(=O)]_w\text{-}NHCH_2\text{-}**$ (w is 1 to 5), where a terminal on a side represented by ** is Q.

(Group having structure represented by $*\text{-}NH\text{-}(LL\text{-}O)_h\text{-}$)

In a case where $R^7$ has a substituent of which a terminal is Q, a form in which $*\text{-}NH\text{-}(LL\text{-}O)_h\text{-}$ and Q are linked by a linking group is also preferable. In this case, the linking group is not particularly limited; however, examples thereof include >C=O, $>NR^{b1}$ ($R^{b1}$ is as described later and is preferably a hydrogen atom or an alkyl group), or an alkylene group, or a group obtained by combining two or more thereof, and preferred examples thereof include an alkylene group. Such $R^7$ may have, for example, a form of $*\text{-}NH\text{-}(LL\text{-}O)_h\text{-}C_2H_4\text{-}Q$.

It is preferable that at least one of $R^6$ or $R^7$ has Q. The form in which $R^6$ has Q includes a form of a case where $R^6$ is Q and a form in which a linking group is bonded to Q (a form in which Q is bonded through a linking group), and the form in which $R^7$ has Q includes a form of a case where $R^7$ is Q and a form in which a linking group is bonded to Q (a form in which Q is bonded through a linking group).

In a case where at least one of $R^6$ or $R^7$ has Q, $R^6$ having Q and $R^7$ having Q preferably have a peptide structure and are more preferably a combination of a peptide structure and an NHS ester structure. In a case where at least one of $R^6$ or $R^7$ has Q, $R^6$ having Q and $R^7$ having Q can also have a form having $-(LL\text{-}O)_h\text{-}$ described later or can be also a combination of $-(LL\text{-}O)_h\text{-}$ and an NHS ester structure. A more specific structure thereof is as described above.

$L^2$ to $L^7$ represent a single bond or a divalent linking group and are preferably a single bond or a linking group obtained by combining one or two or more among groups of an alkylene group, an alkenylene group, an alkynylene group, an arylene group, a heteroarylene group, -O-, -S-, $>NR^{b1}$, >C=O, >S=O, $-S(=O)_2-$, and $>P(=O)OR^{b2}$. $R^{b1}$ and $R^{b2}$ represent a hydrogen atom or a monovalent substituent.

The alkylene group that can constitute $L^2$ to $L^7$ has the same meaning as the group in which one hydrogen atom is further removed from the alkyl group selected from the substituent group T, which is described later, and the same applies to the preferred one thereof.

The alkenylene group which can be adopted as $L^2$ to $L^7$ has the same meaning as the group in which one hydrogen atom is further removed from the alkenyl group selected from the substituent group T, which is described later, and the same applies to the preferred one thereof.

The alkynylene group which can be adopted as $L^2$ to $L^7$ has the same meaning as the group in which one hydrogen atom is further removed from the alkynyl group selected from the substituent group T, which is described later, and the same applies to the preferred one thereof.

The arylene group that can constitute $L^2$ to $L^7$ has the same meaning as the group in which one hydrogen atom is further removed from the aryl group selected from the substituent group T, which is described later, and the same applies to the preferred one thereof.

The heteroarylene group that can constitute $L^2$ to $L^7$ is synonymous with the group in which one hydrogen atom is further removed from the heteroaryl group selected from the substituent group T, which is described later, and the same applies to the preferred one thereof.

The alkylene group, the alkenylene group, the alkynylene group, the arylene group, and the heteroarylene group, which can constitute $L^2$ to $L^7$, may be an unsubstituted group or a group having a substituent.

The substituent which may be contained in the alkylene group, the alkenylene group, the alkynylene group, the arylene group, and the heteroarylene group, which can constitute $L^2$ to $L^7$, is not particularly limited, and examples thereof include a group selected from the substituent group T described later, where an alkyl group, an alkenyl group, an alkynyl group, an aryl group, an alkoxy group, a heteroaryl group, an anionic group, a cationic group, an amino group, an acyl group, a carbamoyl group, an acylamino group, $-(L\text{-}O)_tR^E$, or a halogen atom is preferable.

In addition, the number of substituents which may be contained in the alkylene group, the alkenylene group, the alkynylene group, the arylene group, and the heteroarylene group, which can constitute $L^2$ to $L^7$, is not particularly limited as long as it can be adopted as a structure. The number thereof can be set to at least one or more, the upper limit thereof is not particularly limited, and for example, all hydrogen atoms in the alkylene group, the alkenylene group, the alkynylene group, the arylene group, and the heteroarylene group may be substituted with a substituent.

The monovalent substituent which can be adopted as $R^{b1}$ in $>NR^{b1}$ and $R^{b2}$ in $>P(=O)OR^{b2}$, where each of $>NR^{b1}$ and $>P(=O)OR^{b2}$ can constitute $L^2$ to $L^7$, is preferably an alkyl group, an alkenyl group, an alkynyl group, an aryl group, a heteroaryl group, or $-(L-O)_gR^E$, which has the same meaning as the alkyl group, an alkenyl group, an alkynyl group, an aryl group, a heteroaryl group, and $-(L-O)_gR^E$ in the substituent group T which will be described later, and the same applies to the preferred range thereof.

$R^{b1}$ and $R^{b2}$ are preferably a hydrogen atom or an alkyl group and more preferably a hydrogen atom.

In the linking group obtained by combining two or more of an alkylene group, an alkenylene group, an alkynylene group, an arylene group, a heteroarylene group, -O-, -S-, $>NR^{b1}$, >C=O, >S(=O), $>S(=O)_2-$, and $>P(=O)OR^{b2}$, which can constitute $L^3$ to $L^6$, the kind of the group to be combined is not particularly limited as long as the group to be combined has a reasonable chemical structure; however, it is preferably 2 to 6 kinds and more preferably 2 to 4 kinds. It is noted that in a case of counting, as one kind, each of the alkylene group, the alkenylene group, the alkynylene group, -O-, -S-, $>NR^{b1}$, >C=O, >S=O, $-S(=O)_2-$, and $>P(=O)OR^{b2}$, the maximum number of kinds is 12.

In the linking group obtained by combining two or more of an alkylene group, an alkenylene group, an alkynylene group, an arylene group, a heteroarylene group, -O-, -S-, $>NR^{b1}$, >C=O, >S=O, $>S(=O)_2-$, and $>P(=O)OR^{b2}$, which can constitute $L^3$ to $L^6$, the number of groups to be combined is not particularly limited; however, for example, the following number of groups to be combined is preferable.

In $L^2$, the number of groups to be combined is not particularly limited; however, preferred examples thereof include 1 to 10 groups, where 1 to 7 groups are more preferable, and 1 to 3 groups are still more preferable. It is noted that the number thereof is also preferably 1.

In $L^3$, and $L^6$, the number of groups to be combined is not particularly limited; however, preferred examples thereof include 1 to 10 groups, where 1 to 7 groups are more preferable, and 1 to 5 groups are still more preferable.

In $L^4$, the number of groups to be combined is not particularly limited; however, preferred examples thereof include 1 to 10 groups, where 1 to 7 groups are more preferable, and 1 to 5 groups are still more preferable.

In $L^5$, the number of groups to be combined is not particularly limited; however, preferred examples thereof include 1 to 10 groups, where 1 to 7 groups are more preferable, and 1 to 5 groups are still more preferable.

In $L^7$, the number of groups to be combined is not particularly limited; however, preferred examples thereof include 1 to 20 groups, where 1 to 18 groups are more preferable, and 1 to 10 groups are still more preferable. It is noted that the number thereof is also preferably 1.

For example, in the compound (1) - NHS of Example 1, $L^4$ is a group obtained by combining two pieces of >C=O, one $>NR^{a1}$ ($R^{a1}$ is a hydrogen atom), and one alkylene group ($-C_2H_4-$), and "the kind of group to be combined" is 3 kinds, and "the number of groups to be combined" is 4 groups.

(i) $L^2$ and $L^7$

Among the above-described divalent linking groups that can constitute $L^2$ to $L^7$, $L^2$ is more preferably a divalent linking group in which the number of combinations is 1, still more preferably $>NR^{b1}$, >C=O, an arylene group, an alkylene group, -O-, or -S-, particularly preferably $>NR^{b1}$, >C=O, an arylene group, or an alkyl group, and among the above, particularly preferably >NH or >C=O.

Among the above-described divalent linking groups that can constitute $L^2$ to $L^7$, $L^7$ is more preferably a divalent linking group in which the number of combinations is 1, still more preferably >C=O, $>NR^{b1}$, or an arylene group, and particularly preferably >C =O or $>NR^{b1}$.

(ii) $L^3$ and $L^6$

$L^3$ and $L^6$ are more preferably a group obtained by combining one or two or more of an alkylene group, an alkenylene group, an alkynylene group, an arylene group, a heteroarylene group, -O-, -S-, >C=O, and $>NR^{b1}$, and are still more preferably a group represented by * $-L^x-L^y-$**.

$L^x$ is a single bond or a group obtained by combining one or two or more of an alkylene group, an alkenylene group, an alkynylene group, an arylene group, and a heteroarylene group, and $L^y$ is a single bond, -O, -, -S-, >C=O, or $>NR^{b1}$. * represents a bonding site to a carbon atom to which $L^2$ and $L^4$ are bonded or a carbon atom to which $L^5$ and $L^7$ are bonded, and ** represents a bonding site to M. However, in a case where $L^y$ is a single bond, $L^x$ is a heteroarylene group or a group consisting of a group obtained by combining one or two or more of an alkylene group, an alkenylene group, an alkynylene group, and an arylene group, which are located on the * side, and a heteroarylene group which is

located on the ** side.

Among the groups represented by * $-L^x-L^y-$**, $L^3$ and $L^6$ are preferably a group in which $L^y$ is a single bond, -S-, >C=O, or >$NR^{b1}$, more preferably a group in which $L^y$ is >C=O or >$NR^{b1}$, and still more preferably a group in which $L^x$ is an alkylene group and $L^y$ is >C=O or >$NR^{b1}$.

(iii) $L^4$ and $L^5$

$L^4$ is more preferably a single bond, >C =O, >$NR^{b1}$, an alkylene group, an alkenylene group, an alkynylene group, an arylene group, or a heteroarylene group, or a combination of at least one of an alkylene group, an alkenylene group, an alkynylene group, an arylene group, or a heteroarylene group, and >$NR^{b1}$ and >C=O, still more preferably a group in which $-C(=O)-NR^{b1}-$ and >C =O are linked by at least one of an alkylene group, an alkenylene group, or an alkynylene group, or >C=O, and particularly preferably a group that links $-C(=O)-NR^{b1}-$ and >C=O with an alkylene group, or >C=O, among which >C=O is preferable.

$L^5$ is more preferably a single bond, >C =O, >$NR^{b1}$, an alkylene group, an alkenylene group, an alkynylene group, an arylene group, or a heteroarylene group, or a combination of at least one of an alkylene group, an alkenylene group, an alkynylene group, an arylene group, or a heteroarylene group, and >$NR^{b1}$ and >C=O, still more preferably a group in which $-NR^{b1}-C(=O)-$ and >$NR^{b1}$ are linked by at least one of an alkylene group, an alkenylene group, or an alkynylene group, or >$NR^{b1}$, and particularly preferably a group in which $-NR^{b1}-C(=O)-$ and >$NR^{b1}$ are linked by an alkylene group, or >$NR^{b1}$, among which >$NR^{b1}$ is preferable.

It is noted that the left and right sides of "$-C(=O)-NR^{b1}-$" in the description pertaining to $L^4$ described above and "$-NR^{b1}-C(=O)-$" in the description pertaining to $L^5$ described above shall be the same as the left and right sides of the paper surface in General Formula (II).

It is noted that in the compound represented by General Formula (II), it suffices that the number of shortest-distance atoms in the linking chain that connects M to a carbon atom to which $L^2$ and $L^4$ are bonded or a carbon atom to which $L^5$ and $L^7$ are bonded is, for example, 1 to 60, where 1 to 40 is preferable. In a case where M is a phosphor moiety, the above-described number of shortest-distance atoms means the number of shortest-distance atoms in the linking chain that connects a conjugated structural moiety for exhibiting fluorescence in the phosphor moiety M to a carbon atom to which $L^2$ and $L^4$ are bonded or a carbon atom to which $L^5$ and $L^7$ are bonded.

It is noted that in the compound represented by General Formula (II), it is also preferable that a structure represented by $-(CH_2-CH_2-O)_b-$ described later (b is also as described later) is provided at any portion of the linking chain represented by "-linking group ZZZ-$L^3$-" in the structure represented by "the conjugated structural moiety of M - the linking group ZZZ-$L^3$- described later" and any portion of the linking chain represented by "-linking group ZZZ-$L^6$-" in the structure represented by "the conjugated structural moiety of M - the linking group ZZZ-$L^6$- described later".

In the compound represented by General Formula (II), adjacent groups may be bonded to each other to form a ring. Examples of the combination of adjacent groups which may be bonded to each other to form a ring include a combination of $L^2$ and $L^3$, a combination of $L^2$ and $L^4$, a combination of $L^3$ and $R^4$, a combination of $L^5$ and $L^6$, a combination of $L^5$ and $L^7$, or a combination of $L^6$ and $R^5$.

The above-described ring which may be formed by bonding adjacent groups to each other may be any one of an aromatic ring or an aliphatic ring or may be any one of a hydrocarbon ring or a hetero ring, and it is preferably a 5- or 6-membered ring.

The preferred examples of the aliphatic ring include a cyclopentane ring, a cyclohexane ring, or a 5-membered ring having a carbon atom, a nitrogen atom, and $X^1$ to $X^3$ as ring-constituting atoms, which is described in the structure represented by General Formula (a1) described above, where a 5-membered ring having a carbon atom, a nitrogen atom, and $X^1$ to $X^3$ as ring-constituting atoms, which is described in the structure represented by General Formula (a1) described above, is more preferable.

The aromatic ring is preferably a benzene ring or a nitrogen-containing aromatic heterocyclic ring, more preferably a benzene ring or a nitrogen-containing aromatic heterocyclic ring in which the ring-constituting atom is composed of a carbon atom and a nitrogen atom, and still more preferably a benzene ring or a pyridine ring.

These rings may have a substituent, and the substituent which may be contained is not particularly limited and is selected from the substituent group T.

The ring formed by the combination of $L^3$ and $R^4$ or the combination of $L^6$ and $R^5$ may be any one of the above-described aliphatic ring or aromatic ring, where the above-described aliphatic ring is preferable.

The ring formed by a combination of $L^2$ and $L^3$, a combination of $L^2$ and $L^4$, a combination of $L^5$ and $L^6$, or a combination of $L^5$ and $L^7$, may be any one of the above-described aliphatic ring or aromatic ring, where a 5-membered ring having a carbon atom, a nitrogen atom, and $X^1$ to $X^3$ as ring-constituting atoms, which is described in the structure represented by General Formula (a1) described above, or a benzene ring is preferable.

For example, the following structures surrounded by broken lines in General Formula (II)

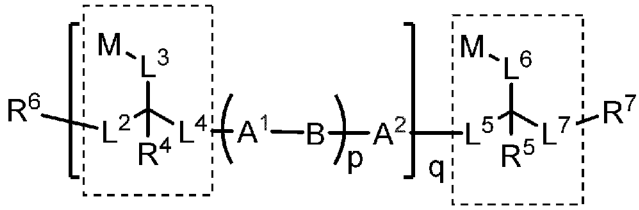

General Formula (II)

can be set to a structure including the following structure including a ring structure. In the following structure, * indicates a connecting portion.

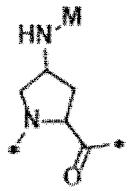

q is an integer of 1 or more. The upper limit value thereof is not particularly limited and can be set to, for example, an integer of 30 or less, and it is preferably an integer of 20 or less, more preferably an integer of 10 or less, and still more preferably an integer of 8 or less.

$A^1$, $A^2$, B, and p respectively have the same meanings as $A^1$, $A^2$, B, and p in General Formula (I) described above.

M represents a phosphor moiety, a physiologically active substance moiety, a prodrug moiety, or a radioactive isotope-containing moiety.

However, at least one of M's represents a phosphor moiety. In a case where there are a plurality of M's, it is preferable that at least two of M's are phosphor moieties of which light absorption characteristics are equivalent to each other.

In the compound according to the embodiment of the present invention, the upper limit value of the number of the phosphor moieties M is not particularly limited and can be set to, for example, 20 or less, and it is preferably 18 or less and more preferably 16 or less.

The phosphor moiety which can be adopted as M (hereinafter, also referred to as the phosphor moiety M) can be used without particular limitation as long as it is a structural moiety consisting of an organic compound that exhibits fluorescence. In addition, the phosphor moiety M may be a structural moiety in which a structural moiety consisting of an organic compound exhibiting fluorescence further has a linking group. Such a linking group is not particularly limited; however, examples thereof include a linking group ZZZ described later. For example, in the compound represented by General Formula (II), preferred examples of the compound include a compound in which the phosphor moiety M is bonded to $L^3$ or $L^6$ by this linking group ZZZ.

Examples of the phosphor moiety M include a structural moiety consisting of at least one dye of a xanthene dye, a rhodamine dye, a coumarin dye, a cyanine dye, a pyrene dye, an oxazine dye, a squarylium dye, a pyridyloxazole dye, or a pyrromethene dye, which is preferable.

As the xanthene dye, the rhodamine dye, the coumarin dye, the cyanine dye, the pyrene dye, the oxazine dye, the squarylium dye, the pyridyloxazole dye, and the pyrromethene dye, dyes that are generally known as these dyes can be used without particular limitation.

In a case where the compound according to the embodiment of the present invention has two or more phosphor moieties, it is preferable that the respective phosphor moieties are phosphor moieties of which light absorption characteristics are equivalent to each other from the viewpoint of providing a structure in which FRET does not occur. The phrase "phosphor moieties of which light absorption characteristics are equivalent to each other" means those that satisfy a relationship in which a difference in the maximum absorption wavelength between the respective phosphor moieties in the absorption spectra is within 15 nm.

In the present invention, the compound is more preferably such that all the phosphor moieties contained in the compound satisfy a relationship in which a difference between the maximum absorption wavelength on the lowest wavelength side and the maximum absorption wavelength on the highest wavelength side among the maximum absorption wavelengths in the absorption spectra of the respective phosphor moieties is within 15 nm.

As a compound having two phosphor moieties in the compound, the compound that causes the FRET phenomenon is known as described above. In this compound, a phosphor moiety I (an energy donor) that is excited with excitation light and another phosphor moiety II (an energy acceptor) that receives energy from the phosphor moiety I are designed such that in addition to satisfying a specific requirement such as the distance between the phosphor

moieties, the difference in the maximum absorption wavelength between the respective absorption spectra generally exceeds 15 nm. In such a compound, the energy is transferred to the phosphor moiety II instead of emitting fluorescence from the phosphor moiety I, and thus the fluorescence intensity of the compound is decreased.

The chemical structures of the above-described phosphor moieties of which the light absorption characteristics are equivalent to each other are not particularly limited as long as the above-described difference in maximum absorption wavelength is satisfied, and they preferably have the same structure in terms of the main skeleton of the phosphor moiety. However, the steric conformation, chain length, and the like of the substituent may be different from each other, and in a case where an anionic group or a cationic group is contained, a counter ion thereof may be different from each other. The difference in the maximum absorption wavelength is preferably within 10 nm and more preferably within 5 nm.

It is noted that the absorption spectrum of the phosphor moiety is a spectrum that is obtained measuring, with a spectrophotometer, a simple body of a phosphor constituting the phosphor moiety, which is diluted with a PBS buffer solution.

As one aspect, the phosphor moiety which can be adopted as M is preferably a structural moiety consisting of a pyrromethene dye. Examples of the pyrromethene dye include a dipyrromethene boron complex. As the dipyrromethene boron complex, it is possible to use a fluorescent compound (a dipyrromethene boron complex) represented by General Formula (1) or (4) described in WO2019/230963A, or a compound (a dipyrromethene boron complex) represented by General Formula (1) described in WO2021/100814A, and the description thereof is incorporated into the present specification by reference.

It is noted that the incorporation is made such that the dye that constitutes the phosphor moiety M does not have a substituent capable of being bonded to a biological substance.

As another aspect, the phosphor moiety which can be adopted as M is preferably a structural moiety consisting of a cyanine dye, and it is more preferably a structural moiety consisting of a cyanine dye represented by General Formula ($\alpha$).

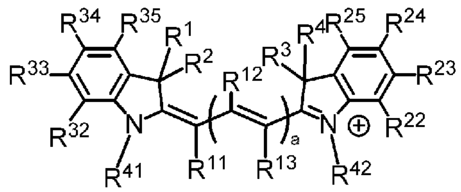

General Formula ($\alpha$)

In the formula, $R^1$ to $R^4$ represent an alkyl group or $-(CH_2-CH_2-O)_b-R^{21}$. b is 1 to 50, and $R^{21}$ represents an alkyl group.

$R^{11}$ to $R^{13}$ represent a hydrogen atom, an alkyl group, an alkoxy group, an aryloxy group, an alkylthio group, an arylthio group, an amino group, or a halogen atom, where adjacent groups may be bonded to each other to form a 5-membered or 6-membered ring.

$R^{22}$ to $R^{25}$ and $R^{32}$ to $R^{35}$ represent a hydrogen atom, an alkyl group, an alkoxy group, an aryl group, a sulfo group, a sulfamoyl group, a carboxy group, an alkoxycarbonyl group, an aryloxycarbonyl group, an acyloxy group, a carbamoyl group, an acylamino group, a nitro group, or a halogen atom.

$R^{41}$ and $R^{42}$ represent an alkyl group or $-(CH_2-CH_2-O)_b-R^{21}$. $R^{21}$ and b respectively have the same meanings as $R^{21}$ and b described above. $R^{41}$ and $R^{42}$ may be bonded to each other to form a ring.

a is an integer of 1 to 3.

In a case where one hydrogen atom is removed from any of $R^1$ to $R^4$, $R^{11}$ to $R^{13}$, $R^{22}$ to $R^{25}$, $R^{32}$ to $R^{35}$, $R^{41}$, or $R^{42}$ described above, a monovalent structural moiety is provided.

However, the cyanine dye represented by Formula ($\alpha$) is neutral.

Depending on the length of the methine chain having a repetition number of 2a + 3, which is connected by a conjugated double bond, the cyanine dyes represented by General Formula ($\alpha$) respectively have an excitation absorption wavelength in a wavelength range of 520 to 600 nm (in the vicinity of 585 nm) in a case of a = 1, in a wavelength range of 620 to 700 nm (in the vicinity of 685 nm) in a case of a = 2, and in a wavelength range of 740 to 830 nm (in the vicinity of 785 nm) in a case of a = 3. As a result, each of the compounds according to the embodiment of the present invention which has, as the phosphor moiety M, a structural moiety consisting of a cyanine dye represented by General Formula ($\alpha$) can be used as a compound exhibiting an excellent fluorescence intensity, in the fluorescence labeling in which a light source having any wavelength in a wavelength range of about 500 to 800 nm (for example, in the vicinity of 600 nm, in the vicinity of 700 nm, or in the vicinity of 800 nm) matching with the absorption excitation wavelength of the compound is used as an excitation light source.

In multicolor WB, a plurality of luminescence colors are detected in the range from the visible range to the near infrared range. As a result, it is necessary to select wavelengths so that the absorption and luminescence waveforms

of a plurality of dyes have a suitable wavelength relationship so that crosstalk does not occur due to mutual interference in a case where the dyes are excited to emit light. Ideally, it should be adjusted so that only one dye emits light at one excitation light and the other dyes do not emit light. From this point of view, two kinds of excitation light sources having wavelengths separated to some extent, for example, in the vicinity of 700 nm and in the vicinity of 800 nm, are used for luminescence in the near infrared range of the multicolor WB.

As compared with the detection by visible light excitation, the fluorescence detection by near-infrared light excitation can suppress the autofluorescence of the membrane, that is, the background fluorescence, and thus it is easy to increase the signal to noise ratio (the S/N ratio) and it is possible to detect a target protein with high sensitivity. As a result, in recent years, there has been an increasing need for fluorescence detection WB using luminescence in the near infrared range in the analytical research on the trace amount of proteins.

However, in the near infrared range, the fluorescence quantum yield of the fluorescent dye is generally low, and thus it is difficult to obtain a high signal amount. Among the compounds according to the embodiment of the present invention which has, as the phosphor moiety M, a structural moiety consisting of a cyanine dye represented by General Formula ($\alpha$), the compound in which a = 2 or 3 can be used as a compound that exhibits an excellent fluorescence intensity even in the multicolor WB having the above-described two kinds of excitation light sources in the vicinity of 700 nm and in the vicinity of 800 nm, and in particular, it can exhibit an excellent fluorescence intensity even with respect to a request for observing and detecting proteins with higher sensitivity, as compared with the fluorescence labeling using cyanine dyes in the related art.

(i) $R^1$ to $R^4$

$R^1$ to $R^4$ represent an alkyl group or $-(CH_2-CH_2-O)_b-R^{21}$.

The alkyl group which can be adopted as $R^1$ to $R^4$ has the same meaning as the alkyl group in the substituent group T which will be described later.

The unsubstituted alkyl group preferably has 1 to 6 carbon atoms, more preferably has 1 to 4 carbon atoms, and still more preferably has 1 or 2 carbon atoms.

In a case where the alkyl group has a substituent, the alkyl group moiety of the alkyl group having a substituent preferably has 1 to 10 carbon atoms, more preferably has 1 to 8 carbon atoms, still more preferably has 2 to 6 carbon atoms, and particularly preferably has 2 to 5 carbon atoms. In addition, the number of atoms that constitute the longest chain of the alkyl group having a substituent is preferably 3 to 35, more preferably 3 to 25, still more preferably 3 to 15, and particularly preferably 3 to 11.

In the present invention, the "number of carbon atoms of the alkyl group moiety of the alkyl group having a substituent" means the number of carbon atoms excluding the substituent moiety contained in the alkyl group.

In the present invention, the "number of atoms that constitute the longest chain of the alkyl group having a substituent" means the number of atoms including the substituent moiety (that is, the number of atoms obtained by subtracting the number of atoms of the molecular chain that does not constitute the longest chain, from the number of total atoms). It is noted that in a case where a substituent having a dissociative hydrogen atom such as a sulfo group or a carboxy group constitutes the longest chain, the calculation is carried out including the hydrogen atom regardless of the presence or absence of dissociation. In addition, the number of atoms in the substituent moiety capable of being bonded to a biological substance described later is not included.

Examples of the substituent which may be contained in the alkyl group which can be adopted as $R^1$ to $R^4$ include an alkoxy group, a carboxy group, an alkoxycarbonyl group, an acyloxy group, a carbonyl group, an acylamino group, a sulfo group, a phosphono group, and $-(CH_2-CH_2-O)_b-R^{21}$, as well as a group consisting of a combination of these substituents.

The alkyl group having a substituent, which can be adopted as $R^1$ to $R^4$, is not particularly limited as long as it is the above-described alkyl group having a substituent.

The alkyl group which can be adopted as $R^1$ to $R^4$ is preferably an unsubstituted alkyl group.

($-(CH_2-CH_2-O)_b-R^{21}$)

In $-(CH_2-CH_2-O)_b-R^{21}$ which can be adopted as $R^1$ to $R^4$, b is 1 to 50, and $R^{21}$ represents an alkyl group.

b means an average repetition number (simply, also referred to as a repetition number), and it is preferably 1 to 24, more preferably 1 to 12, still more preferably 1 to 10, particularly preferably 4 to 10, and most preferably 4 to 8.

The average repetition number can be calculated from the average integrated value obtained by subjecting a compound to $^1H$-NMR measurement. The average repetition number defined in the present invention means a number that is obtained by rounding off the first decimal place of the average repetition number calculated according to the above method.

To the alkyl group as $R^{21}$, the description of the alkyl group which can be adopted as $R^1$ to $R^4$ described above

can be applied.

The $-(CH_2-CH_2-O)_b-R^{21}$ which can be adopted as $R^1$ to $R^4$ and $-(CH_2-CH_2-O)_b-R^{21}$ which can be adopted as a substituent by an alkyl group as $R^1$ to $R^4$ are preferably an alkyl group of $-(CH_2-CH_2-O_b$-unsubstituted.

From the viewpoint of further improving the fluorescence intensity of the fluorescent dye itself, it is preferable that at least one of $R^1$, ..., or $R^4$ includes a structure represented by $-(CH_2-CH_2-O)_b-$, and it is more preferable at least one of $R^1$ or $R^2$ and at least one of $R^3$ or $R^4$ include a structure represented by $-(CH_2-CH_2-O)_b-$.

It is still more preferable that the entire phosphor moiety M in the compound according to the embodiment of the present invention is a structural moiety consisting of a cyanine dye represented by General Formula ($\alpha$), where at least one of $R^1$ or $R^2$ and at least one of $R^3$ or $R^4$ includes a structure represented by $-(CH_2-CH_2-O)_b-$.

It is preferable that the structure represented by $-(CH_2-CH_2-O)_b-$ is introduced by employing $-(CH_2-CH_2-O)_b-R^{21}$ as $R^1$ to $R^4$.

The b in $-(CH_2-CH_2-O)_b-$ described above has the same meaning as the b in $-(CH_2-CH_2-O)_b-R^{21}$ described above.

Since the substituents of $R^1$ to $R^4$ protrude in a direction perpendicular to the cyanine dye skeleton (plane), it is presumed that in a case of including a structure represented by $-(CH_2-CH_2-O)_b-$ as this substituent, the fused ring portion is difficult to undergo the $\pi$-$\pi$ interaction (the effect of suppressing the association is strengthened), and thus the decrease in the fluorescence intensity due to the association can be suppressed.

(ii) $R^{11}$ to $R^{13}$

$R^{11}$ to $R^{13}$ each independently represent a hydrogen atom, an alkyl group, an alkoxy group, an aryloxy group, an alkylthio group, an arylthio group, an amino group, or a halogen atom. Adjacent groups may be bonded to each other to form a 5- or 6-membered ring.

The alkyl group, the alkoxy group, the aryloxy group, the alkylthio group, the arylthio group, the amino group, and the halogen atom, which can be adopted as $R^{11}$ to $R^{13}$, respectively have the same meanings as the alkyl group, the alkoxy group, the aryloxy group, the alkylthio group, the arylthio group, the amino group, and the halogen atom in the substituent group T described later, and the same applies to the preferred ranges thereof.

Examples of the substituent which may be contained in the alkyl group, the alkoxy group, the aryloxy group, the alkylthio group, the arylthio group, and the amino group, as $R^{11}$ to $R^{13}$, include the substituents in the substituent group T described below.

Among $R^{11}$ to $R^{13}$, the 5- or 6-membered ring formed by bonding adjacent groups to each other may be either aromatic or aliphatic, and it is preferably aliphatic. In addition, it is preferable to form a 6-membered ring. The number of the above-described 5- or 6-membered rings in the compound is not particularly limited; however, it is preferably 1 or 2 and more preferably 1.

In a case of taking a case of a = 3 as an example, preferred examples of the structure having a ring formed by bonding adjacent groups among $R^{11}$ to $R^{13}$ include the following structures. It is noted that in the following examples, $R^{11}$ to $R^{13}$ that do not form a ring structure are a hydrogen atom, and the ring structure is described as a structure that does not have a substituent, which are not limited thereto. It is noted that, hereinafter, the structure beyond the wavy line will be omitted.

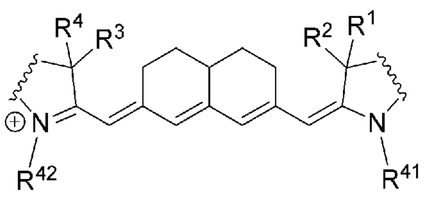

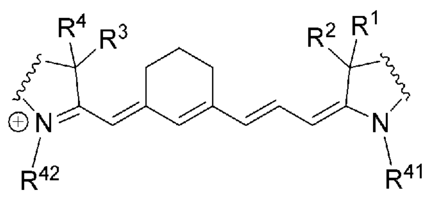

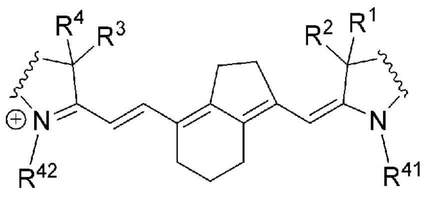

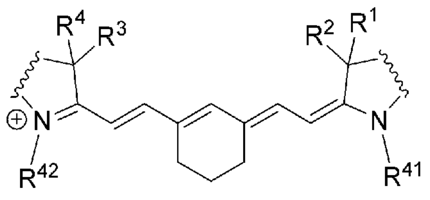

$R^{11}$ and $R^{13}$ possessed by the carbon atom bonded to the indolenine ring are preferably a hydrogen atom.

$R^{12}$, and $R^{13}$ other than those described are preferably a hydrogen atom or an alkyl group.

Among $R^{11}$ to $R^{13}$, adjacent groups in $R^{12}$ and $R^{13}$ other than $R^{11}$ and $R^{13}$ possessed by the carbon atom bonded to the indolenine ring (that is, adjacent groups of $R^{13}$ and $R^{12}$ other than $R^{13}$ possessed by the carbon atom bonded to the indolenine ring) are preferably bonded to each other to form a 5- or 6-membered ring and more preferably to form a 6-membered ring. In addition, it is preferable that the 5- or 6-membered ring is formed at the central portion of the bond that connects the indoline ring and the indolenine ring. The ring formed in the central portion of the bond connecting the indoline ring and the indolenine ring means a ring containing carbon atoms as ring-constituting atoms so that the numbers of bonded atoms from the indoline ring and the indolenine ring are the same.

In a case where one hydrogen atom is removed from any of $R^1$ to $R^4$, $R^{11}$ to $R^{13}$, $R^{22}$ to $R^{25}$, $R^{32}$ to $R^{35}$, $R^{41}$, or $R^{42}$ described above, the phosphor moiety M is a monovalent structural moiety.

Specifically, a monovalent structural moiety is provided in a case where one hydrogen atom is removed from a substituent which can be adopted as $R^1$ to $R^4$, $R^{11}$ to $R^{13}$, $R^{22}$ to $R^{25}$, $R^{32}$ to $R^{35}$, $R^{41}$, or $R^{42}$, or a hydrogen atom which can be adopted as $R^{11}$ to $R^{13}$, $R^{22}$ to $R^{25}$, or $R^{32}$ to $R^{35}$ is removed to provide a monovalent structural moiety which has a bonding site on the carbon atom to which $R^{11}$ to $R^{13}$, $R^{22}$ to $R^{25}$, or $R^{32}$ to $R^{35}$ has been bonded.

Among the above, it is preferable that the phosphor moiety M is to be a monovalent structural moiety by removing one hydrogen atom from the ring formed by the bonding of $R^{41}$ and $R^{42}$ described above.

(iii) $R^{22}$ to $R^{25}$ and $R^{32}$ to $R^{35}$

$R^{22}$ to $R^{25}$ and $R^{32}$ to $R^{35}$ represent a hydrogen atom, an alkyl group, an alkoxy group, an aryl group, a sulfo group, a sulfamoyl group, a carboxy group, an alkoxycarbonyl group, an aryloxycarbonyl group, an acyloxy group, a carbamoyl group, an acylamino group, a nitro group, or a halogen atom. Regarding this $R^{22}$ to $R^{25}$ and $R^{32}$ to $R^{35}$, adjacent groups may be bonded to each other to form a fused ring.

The alkyl group, the alkoxy group, the aryl group, the sulfo group, the sulfamoyl group, the carboxy group, the alkoxycarbonyl group, the aryloxycarbonyl group, the acyloxy group, the carbamoyl group, the acylamino group, the nitro group, and the halogen atom, which can be adopted as $R^{22}$ to $R^{25}$ and $R^{32}$ to $R^{35}$, respectively have the same meanings as the alkyl group, the alkoxy group, the aryl group, the sulfo group, the sulfamoyl group, the carboxy group, the alkoxycarbonyl group, the aryloxycarbonyl group, the acyloxy group, the carbamoyl group, the acylamino group, the nitro group, and the halogen atom in the substituent group T which will be described later.

The fused ring formed by bonding adjacent groups among $R^{22}$ to $R^{25}$ and $R^{32}$ to $R^{35}$ to each other is not particularly limited. However, examples thereof include a naphthalene ring. From the viewpoint of suppressing association, it is preferable that adjacent groups among $R^{22}$ to $R^{25}$ and $R^{32}$ to $R^{35}$ are not bonded to each other and do not form a fused ring.

From the viewpoint of improving water solubility and suppressing association, it is preferable that at least one of $R^{22}$, ..., or $R^{25}$ and at least one of $R^{32}$, ..., or $R^{35}$ have a hydrophilic group, and it is more preferable that at least one hydrophilic group is contained per the number of rings of one, to which $R^{22}$ to $R^{25}$ are bonded and rings to which $R^{32}$ to $R^{35}$ are bonded. For example, in a case where adjacent groups among $R^{22}$ to $R^{25}$ and $R^{32}$ to $R^{35}$ are bonded to each other to form a naphthalene ring as a fused ring, the number of rings to which $R^{22}$ to $R^{25}$ are bonded is two, and the number of rings to which $R^{32}$ to $R^{35}$ are bonded to each other is two, which means that it is more preferable that at least two of $R^{22}$ to $R^{25}$ and at least two of $R^{32}$ to $R^{35}$ have a hydrophilic group. The upper limit value thereof is not particularly limited as long as it is allowed in terms of structure, and it can be appropriately adjusted in accordance with the number of hydrophilic groups in the compound as a whole, which will be described later.

The hydrophilic group is not particularly limited; however, examples thereof include an alkoxy group having a substituent, a carboxy group, a sulfo group, and a phosphono group, where a sulfo group is preferable.

$R^{22}$ to $R^{25}$ and $R^{32}$ to $R^{35}$ are preferably a hydrogen atom, an alkyl group, a sulfo group, a nitro group, or a halogen atom, more preferably a hydrogen atom, an alkyl group, a sulfo group, or a halogen atom, and still more preferably a hydrogen atom, an alkyl group, or a sulfo group.

(iv) $R^{41}$ and $R^{42}$

$R^{41}$ and $R^{42}$ represent an alkyl group or $-(CH_2-CH_2-O)_b-R^{21}$. $R^{21}$ and b respectively have the same meanings as $R^{21}$ and b described above.

Examples of the substituent which may be contained in the alkyl groups as $R^{41}$ and $R^{42}$ include an alkoxy group, a carboxy group, an alkoxycarbonyl group, an acyloxy group, a carbamoyl group, an acylamino group, a sulfo group, and a phosphono group, as well as a group consisting of a combination of these substituents.

The alkyl group which can be adopted as $R^{41}$ and $R^{42}$ has the same meaning as the alkyl group in the substituent group T which will be described later.

The unsubstituted alkyl group preferably has 1 to 6 carbon atoms, more preferably has 1 to 4 carbon atoms, and still more preferably has 1 to 3 carbon atoms.

The alkyl group moiety of the alkyl group having a substituent preferably has 1 to 10 carbon atoms, more preferably has 1 to 8 carbon atoms, still more preferably has 1 to 7 carbon atoms, even still more preferably has 1 to 6 carbon atoms, and even further still more preferably has 1 to 5 carbon atoms. In addition, the number of atoms that constitute the longest chain of the alkyl group having a substituent is preferably 3 to 14, more preferably 3 to 12, and still more preferably 3 to 10.

The alkyl group having a substituent, which can be adopted as $R^{41}$ and $R^{42}$, is preferably an alkyl group having, as a substituent, at least one of an alkoxy group, a carboxy group, a sulfo group, or a phosphono group, and more preferably an alkyl group having, as a substituent, at least one of a carboxy group or a sulfo group, from the viewpoint of further improving water solubility. It is noted that it may be an alkyl group having a substituent consisting of a combination of the above-described preferred substituents (the alkoxy group, the carboxy group, the sulfo group, and the phosphono group) and a group other than these substituents.

In addition, the form of the alkyl group having a substituent, which can be adopted by $R^1$ to $R^4$, can be also preferably applied.

To $-(CH_2-CH_2-O)_b-R^{21}$ which can be adopted as $R^{41}$ and $R^{42}$, the description of $-(CH_2-CH_2-O)_b-R^{21}$ in $R^1$ to $R^4$ can be preferably applied.

$R^{41}$ and $R^{42}$ may be bonded to each other to form a ring.

Among the cyanine dyes represented by General Formula (α), preferred examples of the structure in which $R^{41}$ and $R^{42}$ are bonded to each other to form a ring include a cyanine dye represented by General Formula (β).

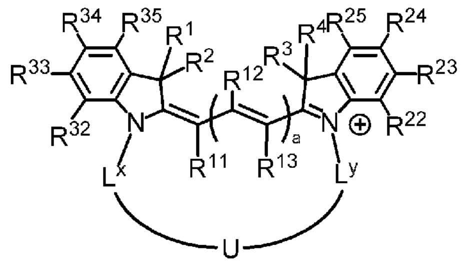

General Formula (β)

In the formula, $L^x$ to $L^y$ represent an alkylene group or $-(CH_2-CH_2-O)_b$-alkylene-*. * represents a bonding position to U.

The linking group U represents a divalent linking group having 1 to 100 atoms.

$R^1$ to $R^4$, $R^{11}$ to $R^{13}$, $R^{22}$ to $R^{25}$, $R^{32}$ to $R^{35}$, b, and a respectively have the same meanings as $R^1$ to $R^4$, $R^{11}$ to $R^{13}$, $R^{22}$ to $R^{25}$, $R^{32}$ to $R^{35}$, b, and a in General Formula (α), and the same also applies to the preferred ranges thereof unless otherwise specified.

At least one of $R^1$, $R^2$, $R^3$, $R^4$, $L^x$, or U includes a structure represented by-$(CH_2-CH_2-O)_b$-. m has the same meaning as m described above.

However, the cyanine dye represented by Formula (β) is neutral.

The alkylene group which can be adopted as $L^x$ and $L^y$ corresponds to an alkylene group obtained by removing one hydrogen atom or one substituent from an alkyl group having a substituent which can be adopted as $R^{41}$ and $R^{42}$.

For the number of carbon atoms of the alkylene group moiety of the alkylene group which can be adopted as $L^x$ to $L^y$, the description of the number of carbon atoms of the alkyl group moiety of the alkyl group having a substituent, as $R^{41}$ and $R^{42}$, can be preferably applied.

The $-(CH_2-CH_2-O)_b$-alkylene-* which can be adopted as $L^x$ and $L^y$ corresponds to $-(CH_2-CH_2-O)_b$-alkylene obtained by removing one hydrogen atom or one substituent from the alkyl group as $R^{21}$, among the $-(CH_2-CH_2-O)_b-R^{21}$ which can be adopted as $R^{41}$ and $R^{42}$ ($R^{21}$ represents an alkyl group having a substituent).

In the $-(CH_2-CH_2-O)_b$-alkylene-* which can be adopted as $L^x$ and $L^y$, b is preferably 1 to 10 and more preferably 1 to 8, and as the number of carbon atoms of the alkylene group moiety, the description of the number of carbon atoms of the alkyl group moiety of the alkyl group having a substituent in $R^{41}$ to $R^{42}$ can be preferably applied.

From the viewpoint of further improving the fluorescence intensity, it is preferable that both $L^x$ and $L^y$ include a structure represented by $-(CH_2-CH_2-O)_b-$.

The total number of atoms constituting the linking group U is 1 to 100, and it is preferably 10 to 90, more preferably 20 to 90, and still more preferably 30 to 80.

The linking group U is preferably a divalent linking group formed by bonding three or more selected from an alkylene group, -O-, $-NR^{50}-$, -COO-, $-CONR^{50}-$, and $-SO_2NR^{50}-$, $R^{50}$ represents a hydrogen atom or an alkyl group.

The number of carbon atoms in the alkylene moiety of the alkylene group which can be adopted as the linking group U is preferably 1 to 10, more preferably 1 to 8, still more preferably 1 to 7, particularly preferably 1 to 6, and most preferably 1 to 5.

In the present invention, "the number of carbon atoms in the alkylene moiety of the alkylene group" means the number of carbon atoms excluding the substituent moiety contained in the alkylene group.
As the alkyl group which can be adopted as $R^{50}$, the description of the alkyl group as $R^1$ to $R^4$ can be preferably applied,
**[0248]** $R^{50}$ is preferably a hydrogen atom.
The number of the above-described alkylene group, -O-, $-NR^{50}-$, -COO-, $-CONR^{50}-$, and $-SO_2NR^{50}-$, constituting the linking group U, is preferably 3 to 11, more preferably 3 to 7, still more preferably 3 to 5, and particularly preferably 3.
In the linking group U, the connecting portion to $L^x$ to $L^y$ is preferably -O-, $-NR^{50}-$, -COO-, $-CONR^{50}-$, or $-SO_2NR^{50}-$. That is, the linking group U is preferably bonded to the alkylene groups of $L^x$ to $L^y$ through -O-, $-NR^{50}-$, -COO-, $-CONR^{50}-$, or $-SO_2NR^{50}-$, which constitutes the linking group U. In the linking group U, it is more preferable that connecting portions to $L^x$ to $L^y$ are -O-, $-NR^{50}-$, -COO-, $-CONR^{50}-$, or $-SO_2NR^{50}-$, where the linking group U is a divalent linking group in which the connecting portions are connected to each other through an alkylene group.
It is preferable that the linking group U is to be a monovalent structural moiety, that is, a phosphor moiety M, by removing one hydrogen atom therefrom. In the linking group U, examples of the position where one hydrogen atom is removed include an alkylene group and an alkyl group as $R^{50}$, where an alkylene group is preferable.
In a case where in the linking group U, one hydrogen atom is removed in the alkylene group or the alkyl group as $R^{50}$, one hydrogen atom may be directly removed from the alkylene group or the alkyl group as $R^{50}$, and the linking group ZZZ may be bonded to the alkylene group or the alkyl group as $R^{50}$, thereby the linking group ZZZ serving as a bonding site.
Examples of the linking group ZZZ include an alkylene group, an alkenylene group, an alkynylene group, an arylene group, a heteroarylene group, -O-, -S-, >C=O, $-NR^{60}-$, >S=O, $-S(=O)_2-$, $>P(=O)OR^{70}$, -COO-, $-CONR^{60}-$, and $-(CH_2-CH_2-O)_p-$, as well as a group consisting of a combination of these substituents. The number of those to be combined is not particularly limited; however, it can be set to, for example, 2 to 20, and it is preferably 2 to 7 and more preferably 2 to 5.
$R^{60}$ and $R^{70}$ are a hydrogen atom or an alkyl group and are preferably a hydrogen atom. To the alkyl group which can be adopted as $R^{60}$ or $R^{70}$, the description of the alkyl group as $R^{50}$ can be preferably applied.
p represents the repetition number, and it is preferably 1 to 10, more preferably 1 to 8, and still more preferably 1 to 4.

(v) a

a is an integer of 1 to 3, where it is preferably an integer of 2 or 3.
In the cyanine dye represented by General Formula ($\alpha$), it is preferable that at least one of $R^1$, $R^2$, $R^3$, $R^4$, $R^{41}$, or $R^{42}$ includes a structure represented by $-(CH_2-CH_2-O)_b-$. b has the same meaning as b described above. It is conceived that this makes it possible for the compound according to the embodiment of the present invention, which has a structural moiety consisting of a cyanine dye represented by General Formula ($\alpha$), to have a proper hydrophilicity and a proper excluded volume effect, whereby a labeled biological substance to be obtained can exhibit an excellent fluorescence intensity.
In addition, from the viewpoint that sufficient hydrophilicity is imparted as the compound according to the embodiment of the present invention, the cyanine dye represented by General Formula ($\alpha$) is such that the number of hydrophilic groups per one molecule of the cyanine dye represented by General Formula ($\alpha$) is preferably 2 or more, more preferably 2 to 8, still more preferably 2 to 6, and particularly preferably 3 to 6.
To the hydrophilic group, the description of the hydrophilic group which can be adopted by $R^{22}$ to $R^{25}$ and $R^{32}$ to $R^{35}$ described above can be applied.
The position of the hydrophilic group is not particularly limited unless specified otherwise, and examples of the group having the hydrophilic group preferably include $R^{22}$ to $R^{25}$, $R^{32}$ to $R^{35}$ $R^{41}$, or $R^{42}$.
It is noted that in the structure represented by General Formula ($\alpha$) or ($\beta$), one hydrogen atom may be removed from any substituent to provide a monovalent structural moiety (the phosphor moiety M); however, it is preferable that, for example, one hydrogen atom is removed from the linking group U to provide a monovalent structural moiety.
The physiologically active substance moiety which can be adopted as M can be used without particular limitation as long as it is a structural moiety consisting of a physiologically active substance. Examples of the physiologically active substance include a vitamin, a coenzyme, a hormone, an antibiotic, a neurotransmitter, and a cytokine. More specific examples thereof are calicheamicin, doxorubicin, daunorubicin, mitomycin C, bleomycin, cyclocytidine, vincristine, vinblastine, methotrexate, cisplatin or a derivative thereof, auristatin or a derivative thereof, maytansine or a derivative thereof, taxol or a derivative thereof, and camptothecin or a derivative thereof, which are described in paragraph [0095] of JP2021-020956A, and the description of paragraphs [0095] to [0099] of JP2021-020956A can be applied thereto.
The prodrug moiety which can be adopted as M can be used without particular limitation as long as it is a structural moiety consisting of a compound that is metabolized in vivo to be changed to a physiologically active substance. To the prodrug, for example, the description (a prodrug form of 2-pyrrolinodoxorubicin) in paragraph [0003] of

JP2020-105187A can be applied.

The radioactive isotope-containing moiety which can be adopted as M can be used without particular limitation as long as it is a structural moiety containing a radioactive isotope that is capable of being used in the medical field. Examples of the radioactive isotope include iodine 131, indium 111, yttrium 90, lutetium 177, and copper 64, which are not limited thereto. The description of paragraph [0225] of JP2021-11483A can be applied thereto. Examples of the structural moiety containing a radioactive isotope include a structural moiety in which the radioactive isotope is bonded or coordinated to a nitrogen atom of an amino group or a tertiary amine, a sulfanyl group, an aryl group, a heteroaryl group, or the like. Examples of the structural moiety in which a nitrogen atom of a tertiary amine is coordinated to the radioactive isotope) include a structural moiety in which 1,4,7,10-tetraazacyclododecane-1,4,7,10-tetraacetic acid (DOTA) or the like is coordinated to the radioactive isotope to form a complex, and examples of the structural moiety in which a sulfanyl group is coordinated to the radioactive isotope include a structural moiety consisting of a complex such as copper (II) diacetylbis(N(4)-methylthiosemicarbazonate).

In a case where a compound represented by General Formula (II) among the compounds according to the embodiment of the present invention is obtained by using a peptide synthesis method, the structure is, in general, such that the C-terminal structure is on the right side of the paper surface, and the N-terminal structure is on the left side of the paper surface.

The compound according to the embodiment of the present invention preferably contains at least one substituent represented by Q, that is, at least one of a carboxy group, a substituent capable of being bonded to a biological substance, or a substituent capable of being bonded to a solid support.

The compound according to the embodiment of the present invention can be bonded to a biological substance by the carboxy group or a substituent capable of being bonded to a biological substance described later, whereby a targeted labeled biological substance can be obtained. It is noted that a substituent capable of being bonded to a biological substance can be easily derived from a carboxy group by a conventional method.

In addition, the compound according to the embodiment of the present invention can be bonded to a solid support such as microparticles by the carboxy group or a substituent capable of being bonded to a solid support described later, whereby a targeted labeled microparticles can be obtained. The microparticle is not particularly limited; however, examples thereof include small particles that are useful for bonding to the compound according to the embodiment of the present invention, including a glass bead, a non-polymer bead such as a magnetic bead, and a polymer bead. In a certain embodiment, the microparticle includes a polystyrene bead. The small particle is not particularly limited as long as it has a size that is commonly used in the fluorescence labeling; however, the average particle diameter thereof is generally 10 nm to 10 μm. It is noted that a substituent capable of being bonded to a solid support can be easily derived from a carboxy group by a conventional method.

In the present invention, for convenience, the substituent capable of being bonded to a biological substance and the substituent capable of being bonded to a solid support do not include a carboxy group, where "the substituent capable of being bonded to a biological substance" includes a substituent capable of being bonded to a biological substance, which is derived from a carboxy group, and "the substituent capable of being bonded to a solid support" includes a substituent capable of being bonded to a solid support, which is derived from a carboxy group. However, as described above, it is also possible to carry out bonding to a biological substance or a solid support by a carboxy group.

In the compound according to the embodiment of the present invention, a position where the substituent represented by Q is provided is not particularly limited; however, the substituent represented by Q is preferably provided in a structure other than the structure represented by General Formula (I) and the phosphor moiety and more preferably provided in at least one of $R^6$ or $R^7$ in the compound represented by General Formula (II).

It suffices that the number of substituents represented by Q in the compound according to the embodiment of the present invention is at least 1 or more in total, and it is preferably 1 to 3, more preferably 1 or 2, and still more preferably 1, from the viewpoint of the quantification of the target substance to be detected.

In addition, from the viewpoint of imparting sufficient hydrophilicity as a compound, the compound according to the embodiment of the present invention also preferably has an anionic group be described later at a position other than the phosphor moiety, and for example, it preferably has one or more anionic groups, more preferably 1 to 8 anionic groups, and still more preferably 1 to 6 anionic groups.

The position of the anionic group is not particularly limited unless specified otherwise, and examples of the group having the anionic group preferably include $L^1$ in General Formula (b).

Specific examples of the compound according to the embodiment of the present invention will be shown below; however, the present invention is not limited to these compounds. In the following specific examples, the sulfo group may adopt a salt structure in which a hydrogen ion is dissociated. Dye indicates a phosphor moiety. The following exemplary compound has an aspect in which an NHS ester structure (N-hydroxysuccinimide ester) is provided, at the terminal, as a substituent capable of being bonded to a biological substance.

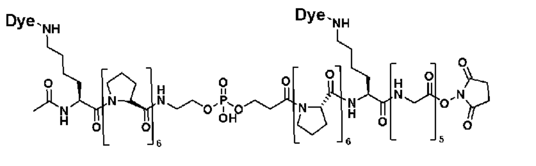

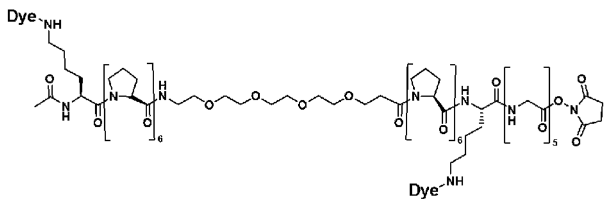

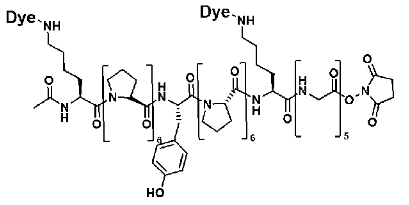

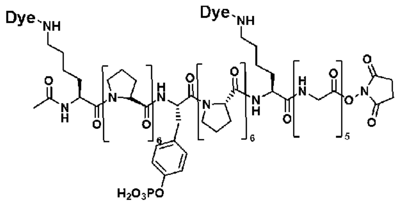

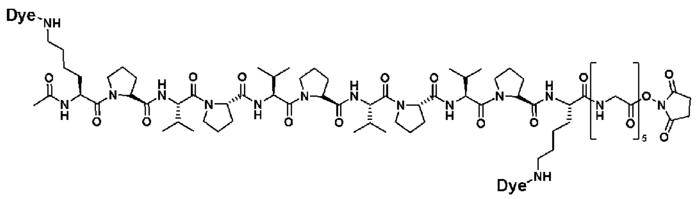

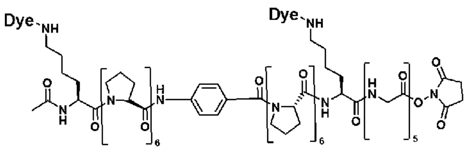

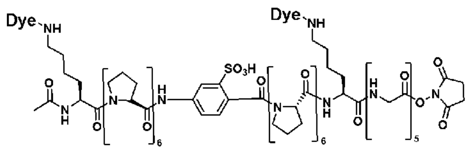

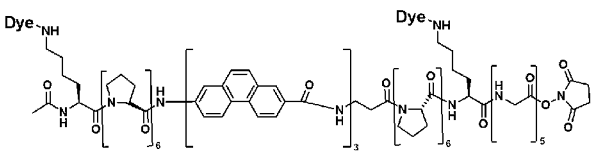

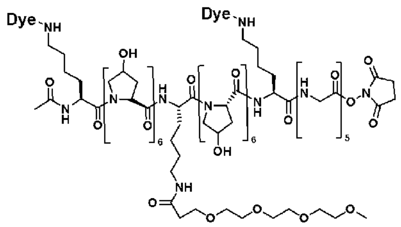

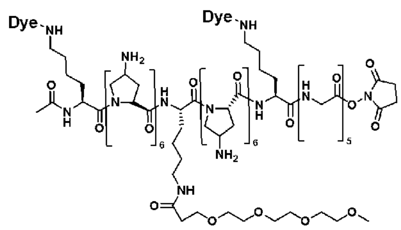

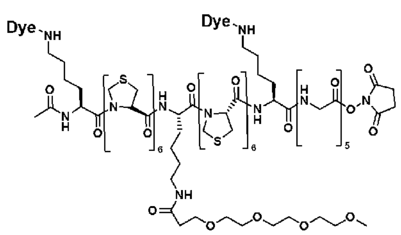

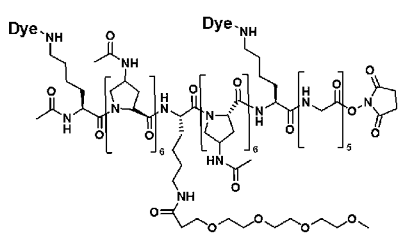

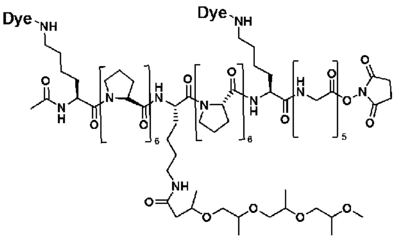

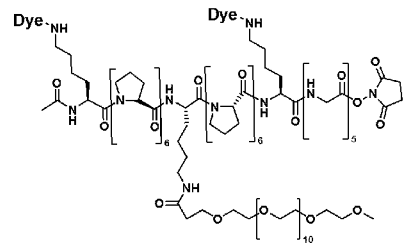

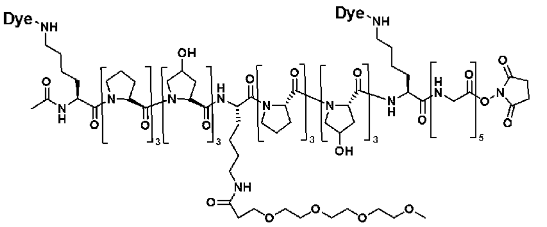

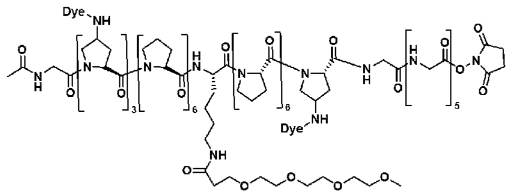

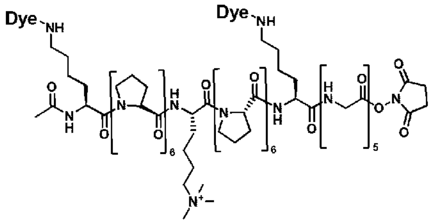

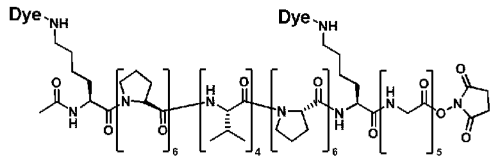

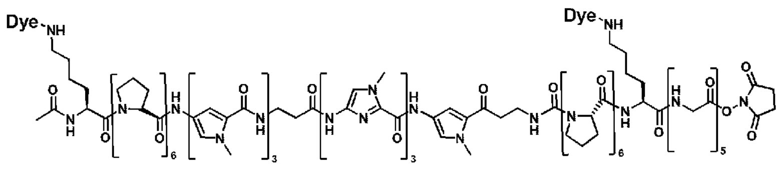

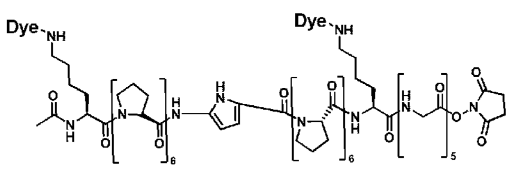

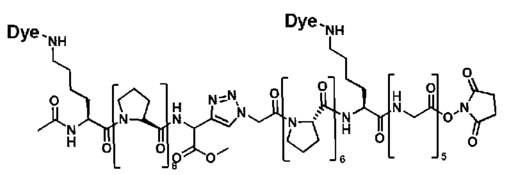

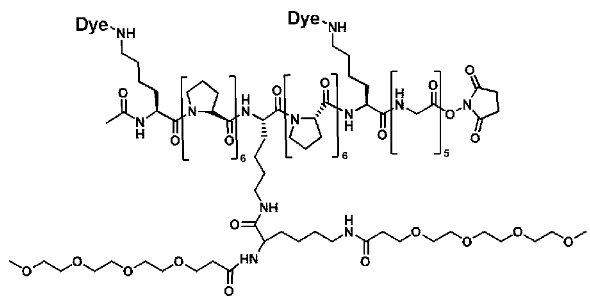

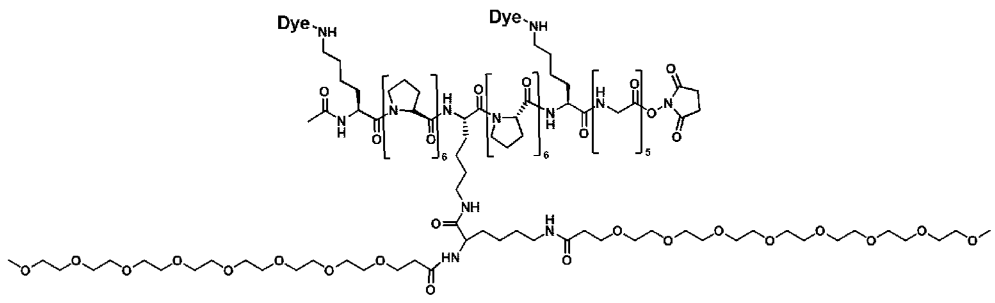

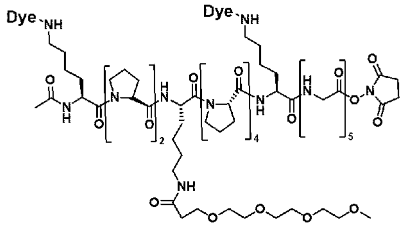

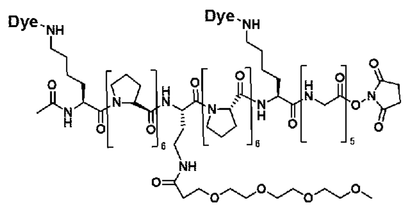

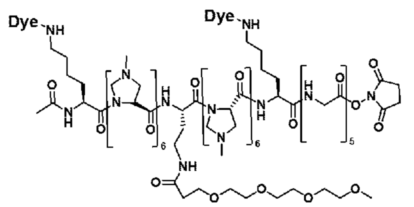

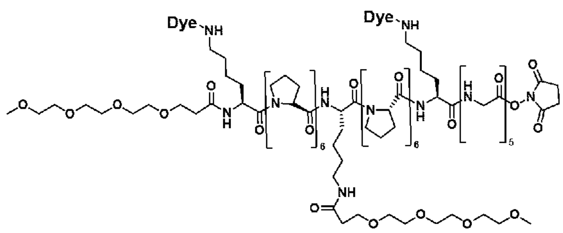

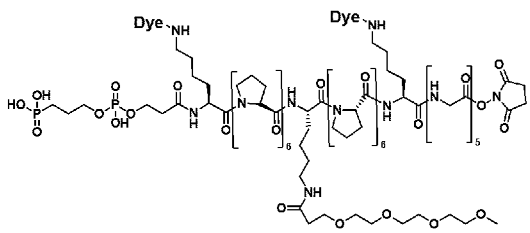

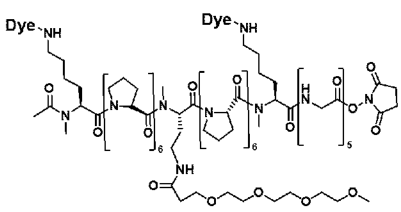

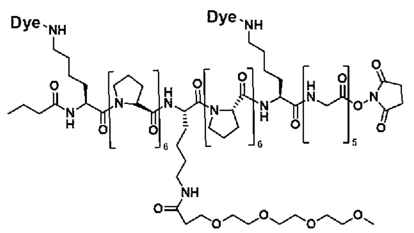

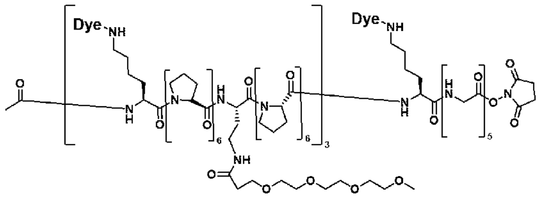

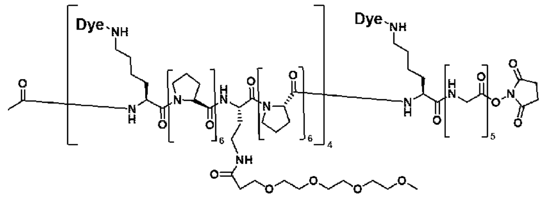

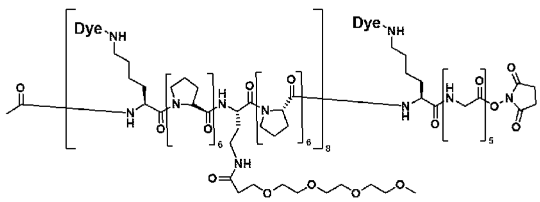

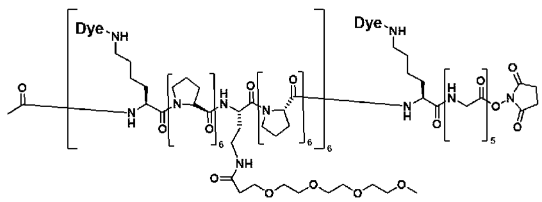

Dye =

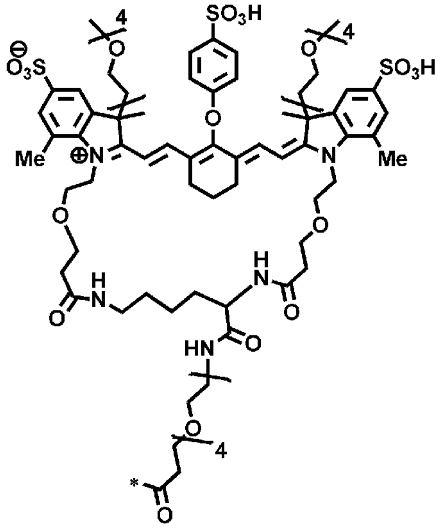

Preferred examples of the form according to the present invention include a compound that satisfies the following form I among the compounds represented by General Formula (II).

(Form I)

$A^1$ (structure represented by General Formula (a1)): At least one of $X^1$, ..., or $X^3$ is $>CR^2R^3$, and l is an integer of 2 to 30. ($R^2$ and $R^3$ are preferably a hydrogen atom, an alkyl group, $-NR^8R^9$, $-OR^{10}$, or an anionic group.)

$A^2$ (structure represented by General Formula (a2)): At least one of $X^4$, ..., or $X^6$ is $>CR^2R^3$, and n is an integer of 2 to 30. ($R^2$ and $R^3$ are preferably a hydrogen atom, an alkyl group, $-NR^8R^9$, $-OR^{10}$, or an anionic group.)

B (structure represented by General Formula (b)): $Y^1$ is a hydrogen atom, $L^1$ is a linking group in which the number of shortest-distance atoms that connect >NY' to >C=O is 1 to 4, and m is an integer of 1 to 4.

$R^4$ and $R^5$: Hydrogen atoms
$R^6$: An acyl group or $-C(=O)(L-O)_tR^E$, or a group obtained by combining an alkyl group, and a phosphono group and/or a phosphonooxy group
$R^7$: A group having structure represented by *-NH-alkylene-C(=O)-, or a group having structure represented by *-NH(LL-O)$_h$- (* means a bonding site to $L^7$)
$L^2$: $>NR^{b1}$, >C=O, an arylene group, an alkylene group, -O-, or -S-
$L^3$ and $L^6$: A group obtained by combining one or two or more of an alkylene group, an alkenylene group, an alkynylene group, an arylene group, a heteroarylene group, -O-, -S-, >C=O, and $>NR^{b1}$
$L^4$ and $L^5$: A single bond, >C =O, $>NR^{b1}$, an alkylene group, an alkenylene group, an alkynylene group, an arylene group, or a heteroarylene group, or a combination of at least one of an alkylene group, an alkenylene group, an alkynylene group, an arylene group, or a heteroarylene group, and $>NR^{b1}$ and >C=O
$L^7$: >C=O, $>NR^{b1}$, or an arylene group
Phosphor moiety in M: A structural moiety consisting of at least one dye of a xanthene dye, a rhodamine dye, a coumarin dye, a cyanine dye, a pyrene dye, an oxazine dye, a squarylium dye, a pyridyloxazole dye, or a pyrromethene dye
p: An integer of 1 to 4
q: An integer of 1 to 20

In these forms, the preferred descriptions pertaining to $A^1$, $A^2$, B, $R^4$ to $R^7$, $L^2$ to $L^7$, M, p, and q described above can be applied to $A^1$, $A^2$, B, $R^4$ to $R^7$, $L^2$ to $L^7$, M, p, and q in General Formula (II).

The compound according to the embodiment of the present invention can be bonded to a biological substance such as a protein (including a peptide), an amino acid, a nucleic acid, a nucleotide, a sugar chain, or a lipid, by at least one substituent capable of being bonded to a biological substance, where the substituent is contained in the compound, and the compound can be used as a labeled biological substance.

The substituent capable of being bonded to a biological substance can be used without particular limitation as long as it is a group for acting (including adhering) or bonding to a biological substance, and examples thereof include the substituents described in WO2002/026891A.

Specific examples of "the substituent capable of being bonded to a biological substance" include the following structures.

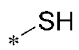
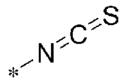
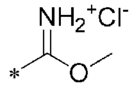
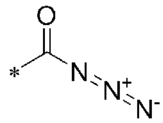
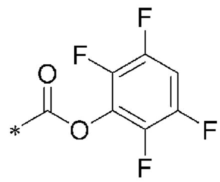
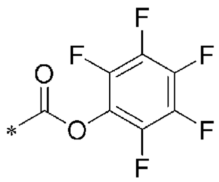

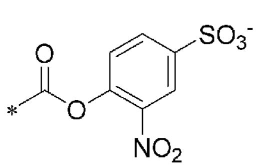
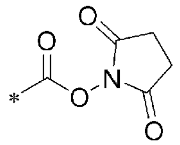
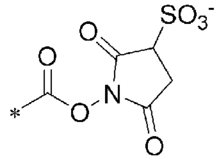
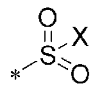
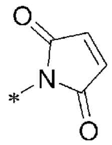

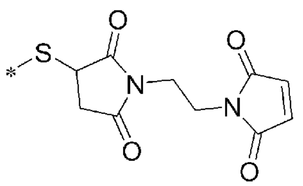
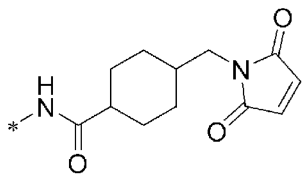
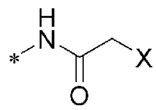
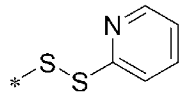

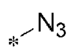
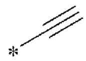
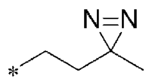

X means a halogen atom such as an iodine atom or a bromine atom. * represents a bonding site.

In addition to the above, it is possible to use a peptide structure (a polyamino acid structure), a long-chain alkyl group, or the like can be used as the "substituent capable of being bonded to a biological substance".

Among them, preferred examples thereof include an N-hydroxysuccinimide ester structure (an NHS ester structure), a succinimide structure, a maleimide structure, an azide group, an acetylene group, a peptide structure (a polyamino acid structure), a long-chain alkyl group (preferably having 12 to 30 carbon atoms), and a quaternary ammonium group.

Among the compounds according to the embodiment of the present invention, specific examples of the compound having at least one substituent capable of being bonded to a biological substance also include, as a specific example, in addition to the above-described exemplary compound (the compound having an NHS ester structure) according to the embodiment of the present invention, a form in which the NHS ester structure is appropriately replaced with the above-described another substituent, which is capable of being bonded to a biological substance. It is noted that the present invention is not limited to these compounds. For example, in the specific examples thereof, a group having a dissociative hydrogen atom such as a carboxy group or a sulfo group may adopt a salt structure by a hydrogen atom being dissociated therefrom.

The compound according to the embodiment of the present invention can be bonded to a solid support such as the above-described microparticles by a substituent capable of being bonded to at least one solid support, the substituent being contained in the compound, and it can be used as a solid support reagent.

The substituent that can be bonded to a solid support can be used without particular limitation as long as it is a group for acting on (including adhering to) or bonding to a solid support, and preferred examples thereof include an N-hydroxysuccinimide ester structure (an NHS ester structure), a succinimide structure, a maleimide structure, an azide

group, an acetylene group, a peptide structure (a polyamino acid structure), a long-chain alkyl group (preferably having 12 to 30 carbon atoms), and a quaternary ammonium group. Among them, preferred examples thereof include an N-hydroxysuccinimide ester structure (an NHS ester structure), a succinimide structure, and a maleimide structure. As the specific structure of "the substituent capable of being bonded to a solid support", the specific structure described as "the substituent capable of being bonded to a biological substance" described above can be used.

Among the compounds according to the embodiment of the present invention, specific examples of the compound having at least one substituent capable of being bonded to a solid support also include, as a specific example, in addition to the above-described exemplary compound (the compound having an NHS ester structure) according to the embodiment of the present invention, a form in which the NHS ester structure is appropriately replaced with the above-described substituent capable of being bonded to a solid support. It is noted that the present invention is not limited to these compounds. For example, in the specific examples thereof, a group having a dissociative hydrogen atom such as a carboxy group or a sulfo group may adopt a salt structure by a hydrogen atom being dissociated therefrom.

The compound according to the embodiment of the present invention can be synthesized according to a conventional method. The structure represented by General Formula (I) can be synthesized, for example, according to a conventional peptide synthesis method, or it can also be synthesized according to solid phase peptide synthesis. As the solid phase peptide synthesis method, a method that uses an automatic peptide synthesizer, which is described in WO2018/174078A, can also be preferably applied. The phosphor moiety, the physiologically active substance moiety, the prodrug moiety, and the radioactive isotope-containing moiety can also be synthesized based on a conventional method and introduced into the compound according to the embodiment of the present invention.

A compound having a substituent capable of being bonded to a biological substance can also be synthesized by a conventional method. For example, Bioconjugate Techniques (Third Edition, written by Greg T. Hermanson) can be referred to.

«Labeled biological substance»

The labeled biological substance according to the embodiment of the present invention is a substance in which the compound according to the embodiment of the present invention, is bonded to a biological substance. Since the compound according to the embodiment of the present invention has fluorescence and exhibits an excellent fluorescence intensity, it can be preferably used for a labeled biological substance. The bond between the compound according to the embodiment of the present invention and a biological substance may have a form in which the compound according to the embodiment of the present invention and the biological substance are directly bonded or a form of being linked via a linking group.

Preferred examples of the biological substance include a protein (including a peptide), an amino acid, a nucleic acid, a nucleotide, a sugar chain, and a lipid. Preferred examples of the protein include an antibody, and preferred examples of the lipid include a phospholipid, a fatty acid, and sterol, where a phospholipid is more preferable.

Among the above biological substances, the clinically useful substance is not particularly limited; however, examples thereof include immunoglobulins such as immunoglobulin (Ig) G, IgM, IgE, IgA, and IgD; blood plasma proteins such as complement, C-reactive protein (CRP), ferritin, $\alpha_1$ microglobulin, $\beta_2$ microglobulin, and antibodies thereof; tumor markers such as $\alpha$-fetoprotein, carcinoembryonic antigen (CEA), prostate acid phosphatase (PAP), carbohydrate antigen (CA) 19-9, and CA-125, and antibodies thereof; hormones such as luteinizing hormone (LH), follicle-stimulating hormone (FSH), human ciliated gonadotropin (hCG), estrogen, and insulin, and antibodies thereof; and viral infection-related substances of viruses such HIV and ATL, hepatitis B virus (HBV)-related antigens (HBs, HBe, and HBc), human immunodeficiency virus (HIV), adult T-cell leukemia (ATL), and antibodies thereof.

The examples thereof further include bacteria such as Corynebacterium diphtheriae, Clostridium botulinum, mycoplasma, and Treponema pallidum, and antibodies thereof; protozoa such as Toxoplasma, Trichomonas, Leishmania, Trypanosoma, and malaria parasites, and antibodies thereof; embryonic stem (ES) cells such as ELM3, HM1, KH2, v6.5, v17.2, v26.2 (derived from mice, 129, 129/SV, C57BL/6, and BALB/c), and antibodies thereof; antiepileptic drugs such as phenytoin and phenobarbital; cardiovascular drugs such as quinidine and digoxin; anti-asthma drugs such as theophylline; drugs such as antibiotics such as chloramphenicol and gentamicin, and antibodies thereof; and enzymes, extracellular toxins (for example, styrelidine O), and the like, and antibodies thereof. In addition, antibody fragments such as Fab'2, Fab, and Fv can also be used.

Examples of the specific form in which the compound according to the embodiment of the present invention (hereinafter, also abbreviated as the compound (A)) and the biological substance interact with each other to be bonded include the forms described below;

i) a non-covalent bond (for example, hydrogen bond, ionic bond including chelate formation) or covalent bond between a peptide in the compound (A) and a peptide in the biological substance,

ii) a Van der Waals force between a long-chain alkyl group in the compound (A) and a lipid bilayer, a lipid, or the

like in the biological substance,

iii) an amide bond formed by reacting an N-hydroxysuccinimide ester (NHS ester) in the compound (A) with an amino group in the biological substance,

iv) a thioether bond formed by reacting a maleimide group in the compound (A) with a sulfanyl group (-SH) in the biological substance, and

v) a formation of a triazole ring, which is formed by the Click reaction between an azide group in the compound (A) and an acetylene group in the biological substance, or the Click reaction between an acetylene group in the compound (A) and an azide group in the biological substance.

However, in the form of the i) described above, the peptide in the compound of the present invention is not particularly limited as long as it is a peptide that is capable of forming a non-covalent bond or a covalent bond with a peptide in the biological substance, and preferred examples of the position where such a peptide is provided include $R^6$ or $R^7$ in General Formula (II).

In addition to the forms i) to v) described above, the bonding can be formed, for example, in the form described in Lucas C. D. de Rezende and Flavio da Silva Emery. A Review of the Synthetic Strategies for the Development of BODIPY Dyes for Conjugation with Proteins, Orbital: The Electronic Journal of Chemistry, 2013, Vol 5, No. 1, p. 62-83. In addition, the method described in the same document can be appropriately referred to for the preparation of the labeled biological substance according to the embodiment of the present invention.

Among the compounds according to the embodiment of the present invention, the labeled biological substance according to the embodiment of the present invention, which is obtained from a compound having a substituent capable of being bonded to a biological substance and a biological substance that is bonded to the compound by an interaction includes the compound in which a moiety other than the substituent capable of being bonded to a biological substance is replaced with the compound according to the embodiment of the present invention, and a product thereof, in the description of the compound example and the product in paragraph 0038 of JP2019-172826A. However, the present invention is not limited to these labeled biological substances and the like.

<Reagent containing labeled biological substance>

In the reagent containing the labeled biological substance according to the embodiment of the present invention, the form of the labeled biological substance according to the embodiment of the present invention, for example, a solution form dissolved in an aqueous medium such as saline or a phosphate buffer solution, and a solid form such as a fine particle powder or a freeze-dried powder, is not particularly limited and can be appropriately selected depending on the purpose of use.

For example, in a case where the labeled biological substance according to the embodiment of the present invention is used as a fluorescence labeling reagent, it can be used as a reagent containing the labeled biological substance having any one of the forms described above.

<Use application of labeled biological substance>

The labeled biological substance according to the embodiment of the present invention, which is obtained from the compound according to the embodiment of the present invention, can exhibit an excellent fluorescence intensity and stably detect fluorescence emitted from the labeled biological substance excited by light irradiation. As a result, the labeled biological substance according to the embodiment of the present invention can be applied to various techniques using the fluorescence labeling, and it can be suitably used, for example, as a fluorescence labeling reagent in a multicolor WB or dot blotting or as a reagent for in vivo fluorescence imaging.

The fluorescence detection carried out using the labeled biological substance according to the embodiment of the present invention usually includes the following processes (i) to (iii) or (iv) to (vii). The fluorescence detection including the processes (i) to (iii) corresponds to the direct method using a primary antibody fluorescently labeled with the compound according to the embodiment of the present invention, and the fluorescence detection including the processes (iv) to (vii) corresponds to the indirect method using a secondary antibody fluorescently labeled with the compound according to the embodiment of the present invention.

(i) The process of preparing each of the following (a) and (b)

(a) A sample containing a targeted biological substance (hereinafter, also referred to as a "target biological substance")

(b) A labeled biological substance according to the embodiment of the present invention (hereinafter, also referred to as a "labeled biological substance A according to the embodiment of the present invention") obtained by bonding the biological substance (hereinafter, also referred to as a "primary biological substance") capable of binding to the target biological substance in the above (a) to the compound according to the embodiment of the present invention

(ii) The process of preparing a conjugate (hereinafter, also referred to as a "fluorescently labeled conjugate A") in which the target biological substance in the above (a) is bonded to the primary biological substance in the labeled biological substance A according to the embodiment of the present invention in the above (b)
(iii) The process of irradiating the fluorescently labeled conjugate A with light having the range of the wavelength which is absorbed by the labeled biological substance A according to the embodiment of the present invention, and detecting the fluorescence emitted by the labeled biological substance A according to the embodiment of the present invention
(iv) The process of preparing each of the following (c) to (e)

(c) A sample containing a target biological substance
(d) A biological substance capable of binding to the target biological substance in the above (c) (hereinafter, also referred to as a "primary biological substance")
(e) A labeled biological substance according to the embodiment of the present invention (hereinafter, also referred to as a "labeled biological substance B according to the embodiment of the present invention") obtained by bonding the biological substance (hereinafter, also referred to as a "secondary biological substance") capable of binding to the primary biological substance in the above (d) to the compound according to the embodiment of the present invention (hereinafter, also referred to as a "labeled biological substance B according to the embodiment of the present invention")

(v) The process of preparing a conjugate (hereinafter, also referred to as a "conjugate b") in which the target biological substance in the above (c) is bonded to the primary biological substance of the above (d)
(vi) The process of preparing a conjugate (hereinafter, also referred to as a "fluorescently labeled conjugate B2") in which the primary biological substance in the conjugate b is bonded to the secondary biological substance in the labeled biological substance B according to the embodiment of the present invention
(vii) The process of irradiating the fluorescently labeled conjugate B2 with light having the range of the wavelength which is absorbed by the labeled biological substance B according to the embodiment of the present invention, and detecting the fluorescence emitted by the labeled biological substance B according to the embodiment of the present invention

Examples of the biological substance (the primary biological substance) capable of binding to the target biological substance and the biological substance (the secondary biological substance) capable of binding to the primary biological substance include the biological substances in the labeled biological substance according to the embodiment of the present invention. The above biological substance can be appropriately selected in accordance with the target biological substance (a biological substance in the test object) or the primary biological substance, and a biological substance capable of specifically binding to the biological substance in the test object or to the primary biological substance can be selected.

Examples of the protein among the target biological substances include a protein, which is a so-called disease marker. The disease marker is not particularly limited; however, examples thereof include α-fetoprotein (AFP), protein induced by vitamin K absence or antagonist II (PIVKA-II), breast carcinoma-associated antigen (BCA) 225, basic fetoprotein (BFP), carbohydrate antigen (CA) 15-3, CA19-9, CA72-4, CA125, CA130, CA602, CA54/61 (CA546), carcinoembryonic antigen (CEA), DUPAN-2, elastase 1, immunosuppressive acidic protein (IAP), NCC-ST-439, γ-seminoprotein (γ-Sm), prostate specific antigen (PSA), prostatic acid phosphatase (PAP), nerve specific enolase (NSE), Iba1, amyloid β, tau, flotillin, squamous cell carcinoma associated antigen (SCC antigen), sialyl LeX-i antigen (SLX), SPan-1, tissue polypeptide antigen (TPA), serial Tn antigen (STN), cytokeratin (CYFRA) pepsinogen (PG), C-reactive protein (CRP), serum amyloid A protein (SAA), myoglobin, creatine kinase (CK), troponin T, and ventricular muscle myosin light chain I.

The target biological substance may be a bacterium. Examples of the bacterium include a bacterium to be subjected to a cellular and microbiological test, which is not particularly limited. Specific examples thereof include Escherichia coli, Salmonella, Legionella, and bacteria causing problems in public health.

The target biological substance may be a virus. Although the virus is not particularly limited, examples of the virus antigen include hepatitis virus antigens such as hepatitis C and B virus antigens, p24 protein antigen of HIV virus, and pp65 protein antigen of cytomegalovirus (CMV), and E6 and E7 proteins of human papillomavirus (HPV).

In the above (i) or (iv), the sample containing the target biological substance is not particularly limited and can be prepared according to a conventional method.

In addition, the labeled biological substance according to the embodiment of the present invention is not particularly limited and can be prepared by bonding a biological substance capable of binding to a target biological substance to the compound according to the embodiment of the present invention, according to a conventional method. The form of the bond and the reaction to form the bond are as described above in the labeled biological substance according to the embodiment of the present invention.

In the above (v), the target biological substance may be directly bonded to the primary biological substance or may be bonded through another biological substance which is different from the target biological substance and the primary biological substance. In addition, in the above (vi), the primary biological substance in the conjugate b may be directly bonded to the secondary biological substance in the labeled biological substance B according to the embodiment of the present invention or may be bonded through another biological substance which is different from the primary biological substance and the secondary biological substance.

The labeled biological substance according to the embodiment of the present invention can be used as a fluorescently labeled antibody in both the direct method and the indirect method but is preferably used as a fluorescently labeled antibody in the indirect method.

In the above (ii) or (v) and the above (vi), the binding of the labeled biological substance or the like according to the embodiment of the present invention to the target biological substance is not particularly limited and can be carried out according to a conventional method.

In the above (iii) or (vii), the wavelength for exciting the labeled biological substance according to the embodiment of the present invention is not particularly limited as long as the wavelength is a luminescence wavelength (wavelength light) capable of exciting the labeled biological substance according to the embodiment of the present invention. Generally, the wavelength for excitation is preferably 300 to 1,000 nm and more preferably 400 to 800 nm.

The fluorescence excitation light source used in the present invention is not particularly limited as long as it emits light having a luminescence wavelength (wavelength light) capable of exciting the labeled biological substance according to the embodiment of the present invention, and for example, various laser light sources can be used. In addition, various optical filters can be used to obtain a preferred excitation wavelength or detect only fluorescence.

Other matters in the above (i) to (vii) are not particularly limited, and conditions of a method, a reagent, a device, and the like, which are generally used in the fluorescence detection using fluorescence labeling, can be appropriately selected.

In addition, also regarding the processes other than the above (i) to (vii), conditions of a method, a reagent, a device, and the like, which are generally used, can be appropriately selected in accordance with various methods using fluorescence labeling.

For example, in the multicolor WB using the labeled biological substance according to the embodiment of the present invention, it is possible to detect a target biological substance with excellent fluorescence intensity by preparing a blotted membrane according to a method generally used for a target biological substance (protein separation by electrophoresis, blotting to a membrane, and blocking of a membrane) and using the labeled biological substance according to the embodiment of the present invention as a labeled antibody (preferably, as a secondary antibody). In the dot blotting using the labeled biological substance according to the embodiment of the present invention, as in the case of the multicolor WB, it is possible to detect a target biological substance with excellent fluorescence intensity by preparing a blotted nitrocellulose membrane, a blotted PVDF (polyvinylidene fluoride) membrane, or the like according to a method generally used for a target biological substance and using the labeled biological substance according to the embodiment of the present invention as a labeled antibody (preferably, as a secondary antibody).

- Substituent group T -

In the present invention, the preferred substituents include those selected from the following substituent group T.

In addition, in the present invention, in a case of being simply described as a substituent, the substituent refers to this substituent group T, and in a case where an individual group, for example, an alkyl group is only described, a corresponding group in the substituent group T is preferably applied.

Further, in the present invention, in a case where an alkyl group is described separately from a cyclic (cyclo)alkyl group, the alkyl group is meant to include a linear alkyl group and a branched alkyl group. On the other hand, in a case where an alkyl group is not described separately from a cyclic alkyl group, and unless otherwise specified, the alkyl group is meant to include a linear alkyl group, a branched alkyl group, and a cycloalkyl group. The same applies to groups (alkoxy group, alkylthio group, alkenyloxy group, and the like) containing a group capable of having a cyclic structure (alkyl group, alkenyl group, alkynyl group, and the like) and compounds containing a group capable of having a cyclic structure. In a case where a group is capable of forming a cyclic skeleton, the lower limit of the number of atoms of the group forming the cyclic skeleton is 3 or more and preferably 5 or more, regardless of the lower limit of the number of atoms specifically described below for the group that can adopt this structure.

In the following description of the substituent group T, a group having a linear or branched structure and a

group having a cyclic structure, such as an alkyl group and a cycloalkyl group, are sometimes described separately for clarity.

The groups included in the substituent group T include the following groups.

An alkyl group (preferably having 1 to 30 carbon atoms, more preferably having 1 to 20 carbon atoms, still more preferably having 1 to 12 carbon atoms, still more preferably having 1 to 8 carbon atoms, still more preferably having 1 to 6 carbon atoms, and particularly preferably having 1 to 3 carbon atoms), an alkenyl group (preferably having 2 to 30 carbon atoms, more preferably having 2 to 20 carbon atoms, still more preferably having 2 to 12 carbon atoms, still more preferably having 2 to 6 carbon atoms, and even still more preferably having 2 to 4 carbon atoms), an alkynyl group (preferably having 2 to 30 carbon atoms, still more preferably having 2 to 20 carbon atoms, still more preferably having 2 to 12 carbon atoms, still more preferably having 2 to 6 carbon atoms, and even still more preferably having 2 to 4 carbon atoms), a cycloalkyl group (preferably having 3 to 20 carbon atoms), a cycloalkenyl group (preferably having 5 to 20 carbon atoms), an aryl group (it may be a monocyclic group or may be a fused ring group (preferably a fused ring group of 2 to 6 rings); in a case of a fused ring group, the fused ring group consists of a 5- to 7-membered ring; and the aryl group preferably has 6 to 40 carbon atoms, more preferably has 6 to 30 carbon atoms, still more preferably has 6 to 26 carbon atoms, and particularly preferably has 6 to 10 carbon atoms), a heterocyclic group (it has, as a ring-constituting atom, at least one of a nitrogen atom, oxygen atom, a sulfur atom, a phosphorus atom, a silicon atom, or selenium atom, may be a monocyclic ring, or may be a fused ring group (preferably a fused ring group of 2 to 6 rings); in a case of a monocyclic group, the monocyclic ring is preferably a 5- to 7-membered ring and more preferably a 5-membered or 6-membered ring; the heterocyclic group preferably has 2 to 40 carbon atoms and more preferably having 2 to 20 carbon atoms; and the heterocyclic group includes an aromatic heterocyclic group (a heteroaryl group) and an aliphatic heterocyclic group), an alkoxy group (preferably having 1 to 20 carbon atoms, and more preferably having 1 to 12 carbon atoms), an alkenyloxy group (preferably having 2 to 20 carbon atoms, and more preferably having 2 to 12 carbon atoms), and an alkynyloxy group (preferably having 2 to 20 carbon atoms, and more preferably having 2 to 12 carbon atoms), a cycloalkyloxy group (preferably having 3 to 20 carbon atoms), an aryloxy group (preferably having 6 to 40 carbon atoms, more preferably having 6 to 26 carbon atoms, and still more preferably having 6 to 14 carbon atoms), a heterocyclic oxy group (preferably having 2 to 20 carbon atoms), a polyalkyleneoxy group,

an alkoxycarbonyl group (preferably having 2 to 20 carbon atoms), a cycloalkoxycarbonyl group (preferably having 4 to 20 carbon atoms), an aryloxycarbonyl group (preferably having 6 to 20 carbon atoms), an amino group (preferably having 0 to 20 carbon atoms; the amino group includes an unsubstituted amino group ($-NH_2$), a (mono- or di-) alkylamino group, a (mono- or di-) alkenylamino group, a (mono- or di-) alkynylamino group, a (mono- or di-) cycloalkylamino group, a (mono- or di-) cycloalkenylamino group, a (mono- or di-) arylamino group, or a (mono- or di-) heterocyclic amino group, where each of the above groups substituting an unsubstituted amino group has the same definition as the corresponding group in the substituent group T), a sulfamoyl group (preferably having 0 to 20 carbon atoms; it is preferably an alkyl, cycloalkyl, or aryl sulfamoyl group), an acyl group (preferably having 1 to 20 carbon atoms, and more preferably having 2 to 15 carbon atoms; it includes -C(=O)H, an alkylcarbonyl group, a cycloalkylcarbonyl group, an arylcarbonyl group, and a heterocyclic carbonyl group), an acyloxy group (preferably having 1 to 20 carbon atoms), a carbamoyl group (preferably having 1 to 20 carbon atoms; it is preferably an alkyl, cycloalkyl, or aryl carbamoyl group),

an acylamino group (preferably having 1 to 20 carbon atoms), a sulfonamide group (preferably having 0 to 20 carbon atoms and preferably an alkyl, cycloalkyl, or aryl sulfonamide group), an alkylthio group (preferably having 1 to 20 carbon atoms and more preferably having 1 to 12 carbon atoms), a cycloalkylthio group (preferably having 3 to 20 carbon atoms), an arylthio group (preferably having 6 to 40 carbon atoms, more preferably having 6 to 26 carbon atoms, and still more preferably having 6 to 14 carbon atoms), a heterocyclic thio group (preferably having 2 to 20 carbon atoms), an alkyl, cycloalkyl, or aryl sulfonyl group (preferably having 1 to 20 carbon atoms),

a silyl group (preferably having 1 to 30 carbon atoms and more preferably having 1 to 20 carbon atoms; it is preferably a silyl group at which an alkyl, aryl, alkoxy, or aryloxy has been substituted), a silyloxy group (preferably having 1 to 20 carbon atoms: it is preferably a silyloxy group at which an alkyl, aryl, alkoxy, or aryloxy has been substituted), a hydroxy group, a cyano group, a nitro group, a halogen atom (for example, a fluorine atom, a chlorine atom, a bromine atom or an iodine atom), an oxygen atom (specifically, $>CH_2$ which constitutes a ring is replaced with $>C=O$), a carboxy group ($-CO_2H$), a phosphono group [$-PO(OH)_2$], a phosphonooxy group [$-O-PO(OH)_2$], a sulfo group ($-SO_3H$), a borate group [$-B(OH)_2$], an onio group (also referred to as a cationic group; it includes an ammonio group including a cyclic ammonio group, a sulfonio group, and a phosphonio group, and it preferably has 0 to 30 carbon atoms and more preferably has 1 to 20 carbon atoms), a sulfanyl group (-SH), a guanidino group ($-NHC(=NH)NH_2$), an amino acid residue, and a polyamino acid residue.

(Anionic group)

In the present invention, the anionic group may be any group having an anion. Examples of such an anionic group include a carboxy group, a phosphono group (a phosphonate group, $-PO(OH)_2$), a phosphonooxy group (a phosphate group, $-OPO(OH)_2$), and a sulfo group, where a phosphono group, a phosphonooxy group, or a sulfo group is preferable, and a phosphonooxy group or a sulfo group is more preferable.

The anionic group may dissociate a hydrogen ion to have an ionic structure, or it may have a salt structure. To the monovalent or polyvalent cation in a case where the anionic group has a salt structure, the description of the monovalent or polyvalent cation in the description of the salt structure described above can be preferably applied.

(Cationic group)

In the present invention, the cationic group may be any group having a cation. Examples of such a cationic group include a group having a quaternary ammonium ion and a group having a quaternary phosphonium ion, where a group having a quaternary ammonium ion is preferable.

The cationic group may have a salt structure in addition to the ionic structure. Examples of the monovalent or polyvalent anion in a case where the cationic group has a salt structure include halide ions such as $F^-$ and $Cl^-$, and monovalent or polyvalent organic anions such as $BF_4^-$, $PF_6^-$, and a bis(trifluoromethylsulfonyl)imide ion.

(Polyalkyleneoxy group)

In the present invention, examples of the polyalkyleneoxy group include a monovalent group represented by $-(L-O)_tR^E$ and a divalent group represented by $-(LL-O)_h-$.

L and LL described above represent an alkylene group obtained by removing one hydrogen atom from an alkyl group in the substituent group T described above, where it preferably has 2 to 4 carbon atoms, more preferably has 2 or 3 carbon atoms, and still more preferably has 2 carbon atoms. The number of carbon atoms contained in the shortest chain that links two carbon atoms which are bonding sites of the group is preferably 0 to 2, more preferably 0 or 1, and still more preferably 0. That is, L and LL described above are most preferably an ethylene group.

t and h described above mean an average repetition number (simply, also referred to as a repetition number), which is preferably 1 to 24, more preferably 1 to 12, and still more preferably 4 to 12. Even in a case where the repetition number is small, for example, even in a case where t and h are 1, a desired effect can be exhibited by preparing the ClogP value.

The $R^E$ represents a hydrogen atom or an alkyl group. For the alkyl group which can be adopted as $R^E$, the description of the alkyl group in the above-described substituent group T can be preferably applied, and among the above, a methyl group is preferable. $R^E$ is preferably a methyl group.

In addition, examples of the group obtained by combining a plurality of substituents selected from the substituent group T include the above-described alkyl group, alkenyl group, alkynyl group, cycloalkyl group, cycloalkenyl group, aryl group, heterocyclic group, alkoxy group, alkenyloxy group, alkynyloxy group, cycloalkyloxy group, aryloxy group, heterocyclic oxy group, alkoxycarbonyl group, cycloalkoxycarbonyl group, aryloxycarbonyl group, amino group, sulfamoyl group, acyl group, acyloxy group, carbamoyl group, acylamino group, sulfonamide group, alkylthio group, cycloalkylthio group, arylthio group, heterocyclic thio group, and an alkyl, cycloalkyl, and aryl sulfonyl group, which have, as a substituent, an anionic group (a carboxy group, a phosphono group, or a sulfo group), a cationic group (an onio group), an amino acid residue, a polyamino acid residue, or a polyalkyleneoxy group.

The substituent selected from the substituent group T is more preferably an alkyl group, an alkenyl group, a cycloalkyl group, an aryl group, a heterocyclic group, an alkoxy group, a cycloalkoxy group, an aryloxy group, an acyl group, an alkoxycarbonyl group, a cycloalkoxycarbonyl group, an amino group, an acylamino group, a carbamoyl group, a cyano group, a halogen atom, an anionic group, a cationic group, or a polyalkyleneoxy group, and particularly preferably an alkyl group, an alkenyl group, an aryl group, a heterocyclic group, an alkoxy group, an acyl group, an alkoxycarbonyl group, an amino group, an acylamino group, a carbamoyl group, a cyano group, an anionic group, a cationic group, or a polyalkyleneoxy group.

In addition to the group obtained by combining a plurality of substituents selected from the substituent group T described above, the substituent selected from the substituent group T also includes a group obtained by combining a plurality of the above groups, unless otherwise specified. For example, in a case where a compound, a substituent, or the like contains an alkyl group, an alkenyl group, or the like, the alkyl group, the alkenyl group, or the like may be substituted or unsubstituted. In addition, in a case where a compound, a substituent, or the like contains an aryl group, a heterocyclic group, or the like, the aryl group, the heterocyclic group, or the like may be a monocyclic ring or a fused ring moiety, and may be substituted or unsubstituted.

## Examples

Hereinafter, the present invention will be described in more detail based on Examples, but the present invention is not limited thereto. It is noted that room temperature means 25°C.

Compound (1) - NHS to compound (8) - NHS, comparative compound (1) - NHS, and a reference compound (2) - NHS, which are used in Examples, are shown below.

It is noted that Dye in each compound represents a structure represented by the following structural formula, and * represents a bonding site. In addition, in each compound, the sulfo group or the phosphonooxy group may include a salt structure (for example, a potassium salt, a sodium salt, a triethylammonium (TEA) salt, or an N,N-diisopropylethylammonium (DIPEA) salt), even unless otherwise specified. $mPEG_4$ means $-(CH_2CH_2O)_4CH_3$.

Hereinafter, the notation of Compound Z in the scheme means a compound (Z).

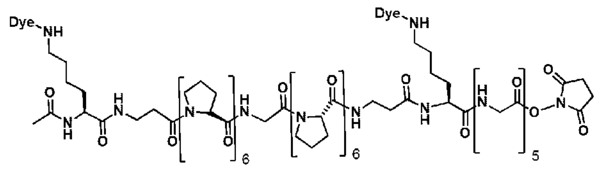

Example 1
Compound (1) – NHS

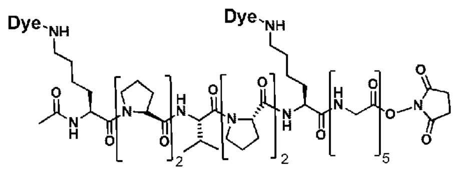

Example 2
Compound (2) – NHS

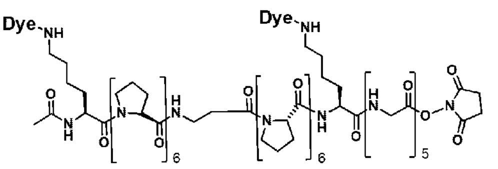

Example 3
Compound (3) – NHS

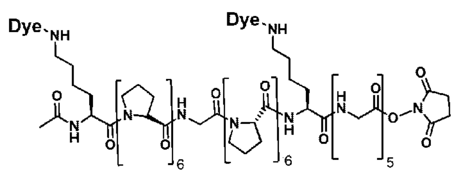

Example 4
Compound (4) – NHS

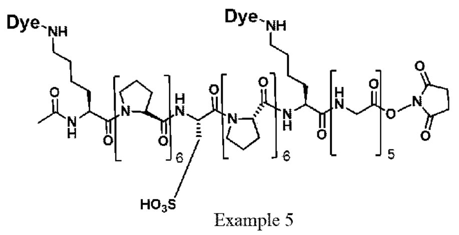

Example 5
Compound (5) – NHS

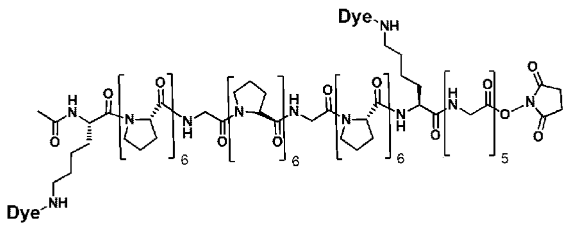

Example 6
Compound (6) – NHS

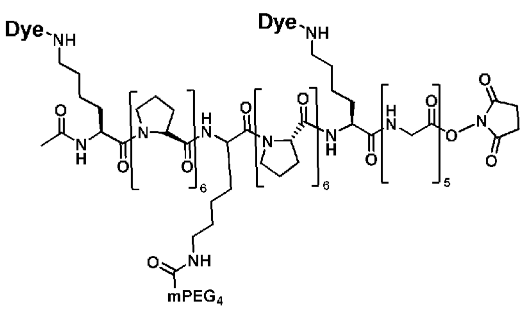

Example 7
Compound (7) – NHS

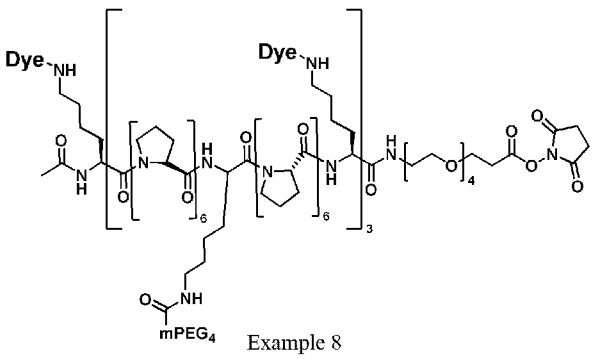

Example 8
Compound (8) – NHS

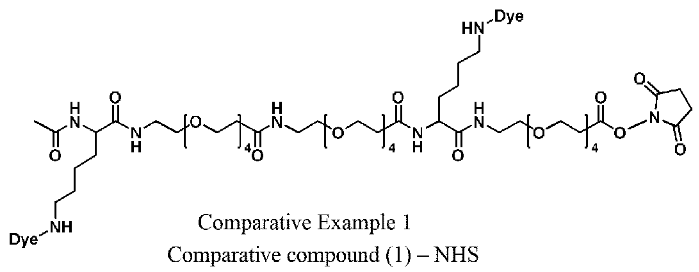

Comparative Example 1
Comparative compound (1) – NHS

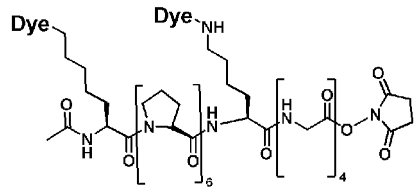

Reference Example 2
Reference compound (2) – NHS

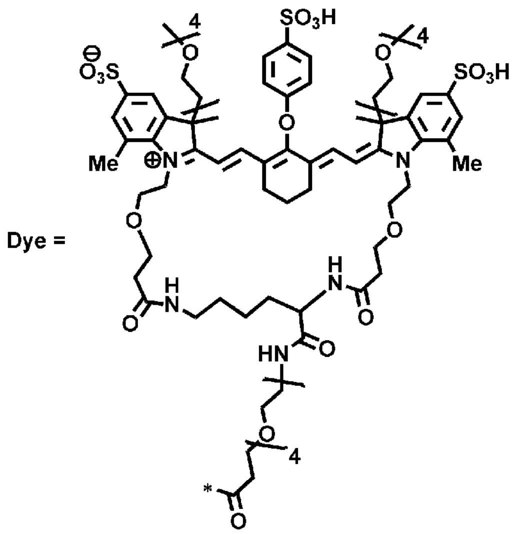

The method of synthesizing each compound and a labeled antibody will be described in detail below; however, the starting materials, the dye intermediates, and the synthetic routes are not limited thereto.

In the following synthetic route, room temperature means 25°C.

It is noted that abbreviations for a solvent, a compound, a substituent, and the like, which is used in each synthesis, respectively mean the same abbreviations for the solvent, the compound, the substituent, and the like in the description of the other portions. Resin means a 2-chlorotrityl resin. In addition, %v/v means a percentage in terms of volume.

Unless otherwise specified, SNAP Ultra C18 (product name, manufactured by Biotage, LLC) or Sfar C18 (product name, manufactured by Biotage, LLC) was used as the carrier in the reverse phase column chromatography, and Hi-Flash Column (product name, manufactured by Yamazen Corporation) was used as a carrier in the normal phase column chromatography.

The mixing ratio in the eluent used in the reverse phase column chromatography or the normal phase column chromatography is in terms of the volume ratio. For example, "acetonitrile:water = from 0:100 to 20:80" means that the eluent of "acetonitrile:water = 0:100" was changed to an eluent of "acetonitrile:water = 20:80".

For the preparative high performance liquid chromatography (HPLC), 2767 (product name, manufactured by Waters Corporation) was used.

The MS spectrum was measured by ACQUITY SQD LC/MS System [product name, manufactured by Waters Corporation, ionization method: electrospray Ionization (ESI)] or LCMS-2010EV [product name, manufactured by Shimadzu Corporation, ionization method: an ionization method simultaneously carrying out ESI and atomospheric pressure chemical ionization (APCI)].

Synthesis example

The synthesis of a peptide chain was carried out according to the general method of the peptide solid phase method described in WO2018/174078A.

[General method of solid phase peptide synthesis method using automatic peptide synthesizer]

Solid phase peptide synthesis was carried out using an automatic peptide synthesizer (product name: Syrol, manufactured by biotage, LLC). The synthesizer was set with Rink Amide-ChemMatrix (registered trade name) (manufactured by Biotage, LLC), an N-methyl-2-pyrrolidone (NMP) solution of Fmoc amino acid (0.5 mol/L), an NMP solution of cyano-hydroxyimino-acetic acid ethyl ester (1.0 mol/L) and diisopropylethylamine (0.1 mol/L), an NMP solution of diisopropylcarbodiimide (1.0 mol/L), an NMP solution of piperidine (20% v/v), and an NMP solution of acetic anhydride (20 %v/v), and synthesis was carried out according to the manual. A procedure of Fmoc deprotection (20 minutes), washing with NMP, condensation of Fmoc amino acids (1 hour), and washing with NMP was set as one cycle, and this cycle was repeated to elongate the peptide chain.

[Synthesis of compound (M1-1)]

A compound (M1-1) was synthesized based on the following scheme. The results of the MS measurement of the compound (5) were as follows.
MS (ESI m/z): $(M + H^+)^+ = 1,723$, $(M - H^+)^- = 1,721$

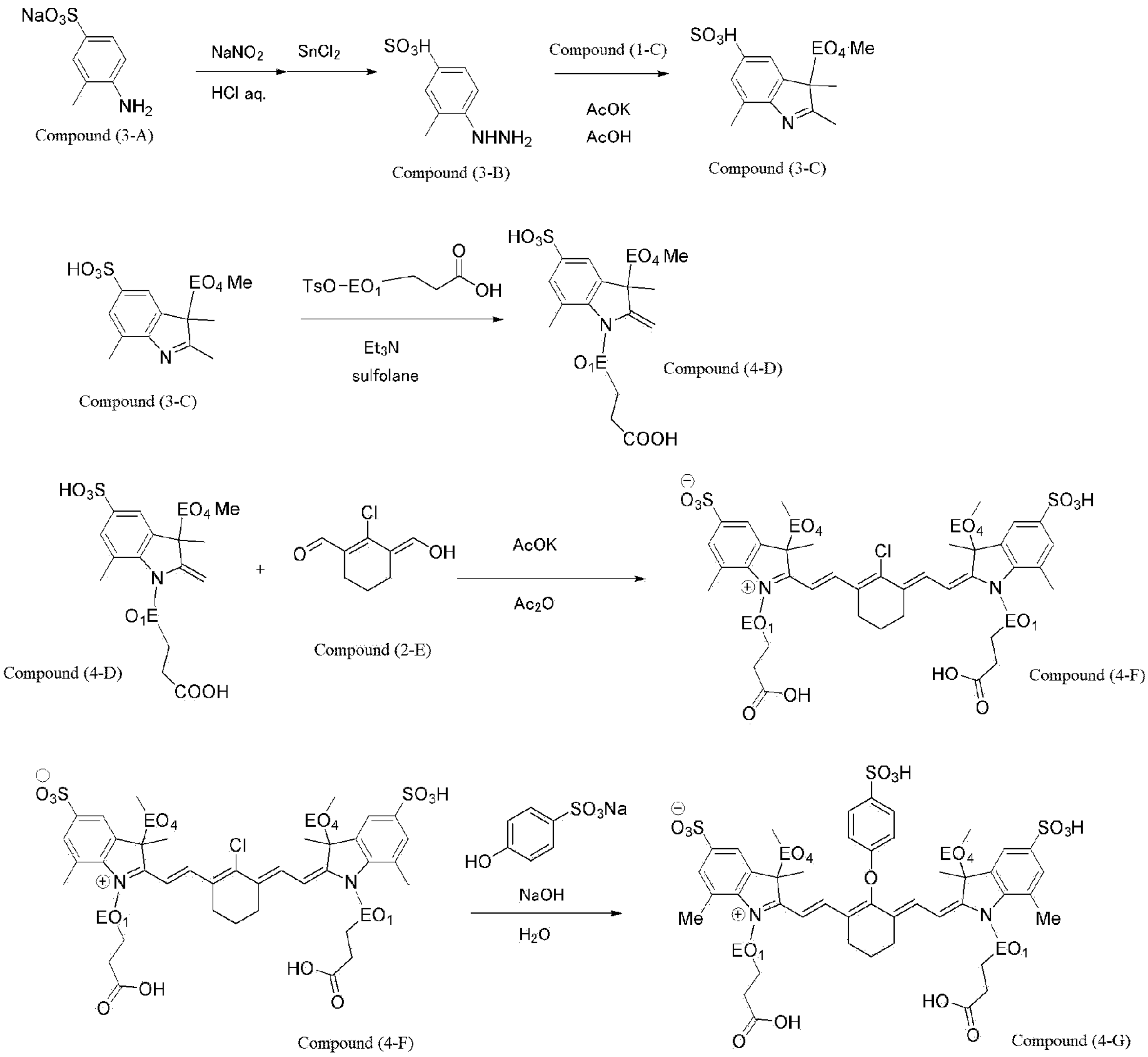

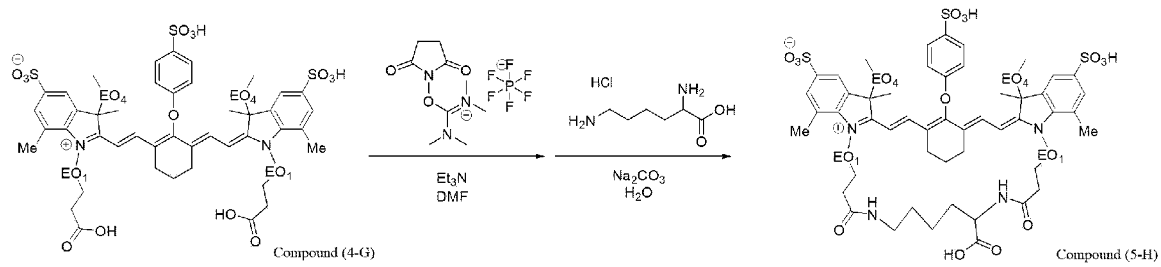

Compound (4-G)
Compound (5-H)

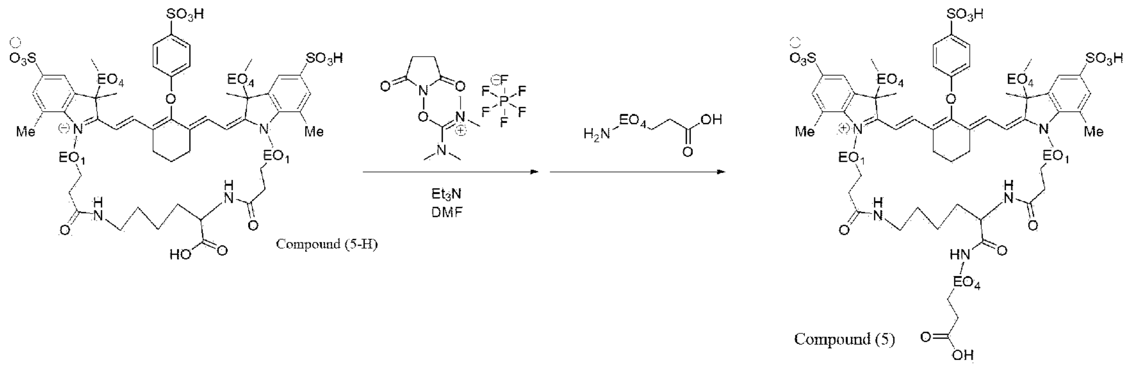

Compound (5-H)
Compound (5)

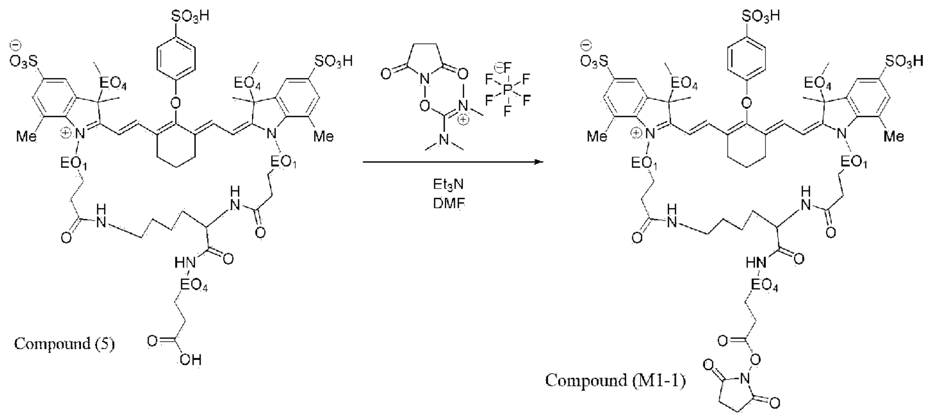

Compound (5)
Compound (M1-1)

[Synthesis of compound (1)]

A compound (1) was synthesized as follows.

1) Synthesis of compound (1-1)

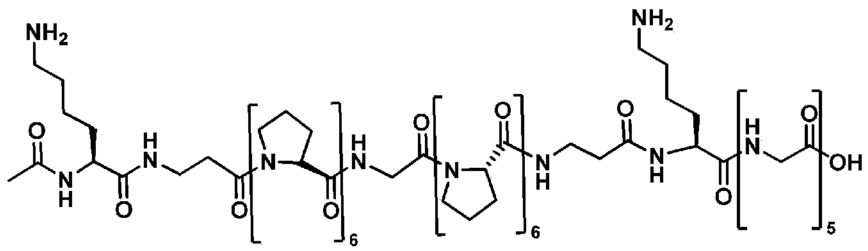

Compound 1-1

Solid phase peptide synthesis was carried out using H-Gly-Trt(2-Cl)-Resin (manufactured by Watanabe Chemical Industries, Ltd., 0.93 mmol/g, 53.8 mg) as a starting raw material. The elongation that was carried out by using N-[(9H-fluoren-9-ylmethoxy)carbonyl]glycine (Fmoc-Gly-OH) was repeated for 4 cycles. Nε-(tert-butoxycarbonyl)-Nα-[(9H-fluoren-9-ylmethoxy)carbonyl)-L-lysine (Fmoc-Lys(Boc)-OH) and subsequently N-[(9H-fluoren-9-ylmethoxy)carbonyl]-β-alanine (Fmoc-β-Ala-OH) were subjected to elongation. Subsequently, the elongation that was carried out by using N-(9-fluorenylmethoxycarbonyl)-L-proline (Fmoc-Pro-OH) was repeated for 6 cycles. Further, after subjecting N-[(9H-fluoren-9-ylmethoxy)carbonyl]glycine (Fmoc-Gly-OH) to elongation, the elongation that was carried out by using N-(9-fluorenylmethoxycarbonyl)-L-proline (Fmoc-Pro-OH) was repeated for 6 cycles, and then N-[(9H-fluoren-9-ylmethoxy)carbonyl]-β-alanine (Fmoc-β-Ala-OH) was subjected to elongation. Subsequently, Nε-(tert-butoxycarbonyl)-Nα-[(9H-fluoren-9-ylmethoxy)carbonyl)-L-lysine (Fmoc-Lys(Boc)-OH) was subjected to elongation, and then, an NMP solution of piperidine (20% v/v) was added thereto to carry out a reaction for 20 minutes, thereby carrying out the deprotection of the Fmoc group, and then, an NMP solution of acetic anhydride (20% v/v) was added thereto to carry out a reaction for 10 minutes, thereby subjecting the N-terminal amino group to acetylation. After completion of the elongation, the resin was washed with dichloromethane, and then the solvent was distilled off under reduced pressure. 2.0 mL of a mixed solution of trifluoroacetic acid (TFA):triisopropylsilane:water = 95:2.5:2.5 was added thereto, and the peptide was cut out and deprotected. After 2 hours, the resin was filtered out, and methyl-t-butyl ether (12 mL) was added to the filtrate to generate a solid. The solid was precipitated by centrifugation and then the supernatant was removed. The solid was washed with methyl-t-butyl ether, and then the solvent was distilled off under reduced pressure to obtain 73.2 mg of a white solid compound (1-1).

2) Synthesis of compound (1)

1.2 mg of the compound (1-1), 150 μL of N,N-dimethylformamide (DMF), 1 μL of triethylamine ($Et_3N$), and 3.5 mg of the compound (M1-1) were placed in an eggplant flask having a capacity of 10 mL, and stirring was carried out at room temperature for 1 hour. Thereafter, the reaction solution was concentrated and purified by preparative HPLC, and freeze drying was followed to obtain 2.2 mg of a compound (1).

The results of the MS measurement of the compound (1) were as follows.

MS (ESI m/z): $(M + H^+)^+$ = 5,373, $(M - H^+)^-$ = 5,271

3) Synthesis of compound (1) - NHS

220 μL of N,N-dimethylformamide (DMF), 1 mg of N,N,N',N'-tetramethyl-O-(N-succinimidyl)uronium hexafluorophosphate, and 1.3 μL of triethylamine ($Et_3N$) were added to 2.2 mg of the compound (1), and stirring was carried out for 1 hour. Then, the solvent was distilled off under reduced pressure, ethyl acetate was added, the supernatant was removed, and vacuum drying was carried out to obtain a compound (1) - NHS.

The synthesis scheme of the compound (1) and the compound (1) - NHS, in which the compound (1-1) and the compound (M1-1) are used as starting materials are as follows.

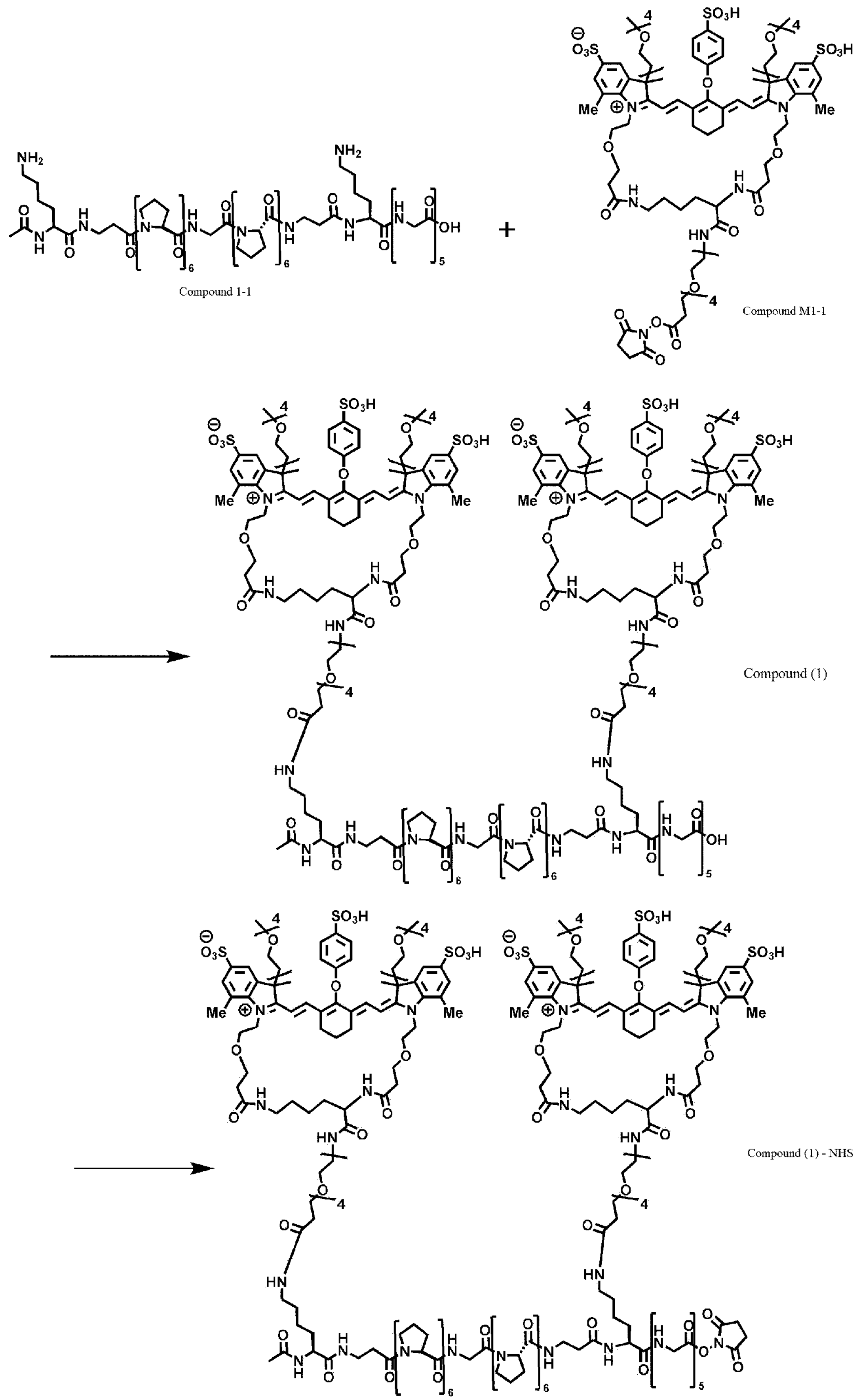

Compound 1-1

Compound M1-1

Compound (1)

Compound (1) - NHS

The compounds (2) to (7) and the reference compound (2), which are synthesized below are respectively compounds obtained by replacing the NHS ester structures in the above-described compounds (2) - NHS to (7) - NHS with a carboxy group and reference compound (2) - NHS. That is, the carboxy group in each of the compounds (2) to (7) and the reference compound (2) is converted into the corresponding NHS ester structure by carrying out the same chemical reaction as in the synthesis of the above-described compound (1) - NHS.

[Synthesis of compound (2) and compound (2) - NHS]

A compound (2) was synthesized in the same manner as in the synthesis of the compound (1) by using, as raw materials for solid phase peptide synthesis, N-[(9H-fluoren-9-ylmethoxy)carbonyl]glycine (Fmoc-Gly-OH), Nε-(tert-butoxycarbonyl)-Nα-[(9H-fluoren-9-ylmethoxy)carbonyl)-L-lysine (Fmoc-Lys(Boc)-OH), N-(9-fluorenylmethoxycarbonyl)-L-proline (Fmoc-Pro-OH), and N-[(9H-fluoren-9-ylmethoxy)carbonyl]-L-valine (Fmoc-L-Val-OH), except that the peptide chain was subjected to elongation so that the structure of the peptide chain described in the compound (2) - NHS was obtained.
The results of the MS measurement of the compound (2) were as follows.
MS (ESI m/z): $(M + H^+)^+$ = 5,273, $(M - H^+)^-$ = 5,271
The compound (2) - NHS was synthesized from the compound (2) in the same manner as in the synthesis of the compound (1) - NHS, except that the compound (2) was used.

[Synthesis of compound (3) and compound (3) - NHS]

A compound (3) was synthesized in the same manner as in the synthesis of the compound (1) by using, as raw materials for solid phase peptide synthesis, N-[(9H-fluoren-9-ylmethoxy)carbonyl]glycine (Fmoc-Gly-OH), Nε-(tert-butoxycarbonyl)-Nα-[(9H-fluoren-9-ylmethoxy)carbonyl)-L-lysine (Fmoc-Lys(Boc)-OH), N-(9-fluorenylmethoxycarbonyl)-L-proline (Fmoc-Pro-OH), and N-[(9H-fluoren-9-ylmethoxy)carbonyl]-β-alanine (Fmoc-β-Ala-OH), except that the peptide chain was subjected to elongation so that the structure of the peptide chain described in the compound (3) - NHS was obtained.
The results of the MS measurement of the compound (3) were as follows.
MS (ESI m/z): $(M + H^+)^+$ = 5,245, $(M - H^+)^-$ = 5,243
The compound (3) - NHS was synthesized from the compound (3) in the same manner as in the synthesis of the compound (1) - NHS, except that the compound (3) was used.

[Synthesis of compound (4) and compound (4) - NHS]

A compound (4) was synthesized in the same manner as in the synthesis of the compound (1) by using, as raw materials for solid phase peptide synthesis, N-[(9H-fluoren-9-ylmethoxy)carbonyl]glycine (Fmoc-Gly-OH), Nε-(tert-butoxycarbonyl)-Nα-[(9H-fluoren-9-ylmethoxy)carbonyl)-L-lysine (Fmoc-Lys(Boc)-OH) and N-(9-fluorenylmethoxycarbonyl)-L-proline (Fmoc-Pro-OH), except that the peptide chain was subjected to elongation so that the structure of the peptide chain described in the compound (4) - NHS was obtained.
The results of the MS measurement of the compound (4) were as follows.
MS (ESI m/z): $(M + H^+)^+$ = 5,231, $(M - H^+)^-$ = 5,229
The compound (4) - NHS was synthesized from the compound (4) in the same manner as in the synthesis of the compound (1) - NHS, except that the compound (4) was used.

[Synthesis of compound (5)]

A compound (5) was synthesized in the same manner as in the synthesis of the compound (1) by using, as raw materials for solid phase peptide synthesis, N-[(9H-fluoren-9-ylmethoxy)carbonyl]glycine (Fmoc-Gly-OH), Nε-(tert-butoxycarbonyl)-Nα-[(9H-fluoren-9-ylmethoxy)carbonyl)-L-lysine (Fmoc-Lys(Boc)-OH), N-(9-fluorenylmethoxycarbonyl)-L-proline (Fmoc-Pro-OH), and N-[(9H-fluoren-9-ylmethoxy)carbonyl]-L-cysteic acid, except that the peptide chain was subjected to elongation so that the structure of the peptide chain described in the compound (5) - NHS was obtained.
The results of the MS measurement of the compound (5) were as follows.
MS (ESI m/z): $(M + H^+)^+$ = 5,325, $(M - H^+)^-$ = 5,323
The compound (5) - NHS was synthesized from the compound (5) in the same manner as in the synthesis of the compound (1) - NHS, except that the compound (5) was used.

[Synthesis of compound (6) and compound (6) - NHS]

A compound (6) was synthesized in the same manner as in the synthesis of the compound (1) by using, as raw materials for solid phase peptide synthesis, N-[(9H-fluoren-9-ylmethoxy)carbonyl]glycine (Fmoc-Gly-OH), Nε-(tert-butoxycarbonyl)-Nα-[(9H-fluoren-9-ylmethoxy)carbonyl)-L-lysine (Fmoc-Lys(Boc)-OH) and N-(9-fluorenylmethoxycarbonyl)-L-proline (Fmoc-Pro-OH), except that the peptide chain was subjected to elongation so that the structure of the peptide chain described in the compound (6) - NHS was obtained.

The results of the MS measurement of the compound (6) were as follows.
MS (ESI m/z): $(M + H^+)^+$ = 5,870, $(M - H^+)^-$ = 5,868

The compound (6) - NHS was synthesized from the compound (6) in the same manner as in the synthesis of the compound (1) - NHS, except that the compound (6) was used.

[Synthesis of compound (7) and compound (7) - NHS]

Solid phase peptide synthesis was carried out using H-Gly-Trt(2-Cl)-Resin (0.93 mmol/g, 53.8 mg) as a starting raw material. The elongation that was carried out by using N-[(9H-fluoren-9-ylmethoxy)carbonyl]glycine (Fmoc-Gly-OH) was repeated for 4 cycles. The elongation that was carried out by using Nε-(tert-butoxycarbonyl)-Nα-[(9H-fluoren-9-ylmethoxy)carbonyl)-L-lysine (Fmoc-Lys(Boc)-OH) and subsequently N-(9-fluorenylmethoxycarbonyl)-L-proline (Fmoc-Pro-OH) was repeated for 6 cycles. Further, after subjecting Nε-(1-(4,4-dimethyl-2,6-dioxocyclohexa-1-ylidene)-3-methylbutyl)-Nα-[(9H-fluoren-9-ylmet hoxy)carbonyl)-L-lysine (Fmoc-Lys(ivDde)-OH) to elongation, the elongation that was carried out by using N-(9-fluorenylmethoxycarbonyl)-L-proline (Fmoc-Pro-OH) was repeated for 6 cycles, and then, Nε-(tert-butoxycarbonyl)-Nα-[(9H-fluoren-9-ylmethoxy)carbonyl)-L-lysine (Fmoc-Lys(Boc)-OH) was subjected to elongation. Thereafter, an NMP solution of piperidine (20% v/v) was added thereto to carry out a reaction for 20 minutes, thereby carrying out the deprotection of the Fmoc group, and then, an NMP solution of acetic anhydride (20% v/v) was added thereto to carry out a reaction for 10 minutes, thereby subjecting the N-terminal amino group to acetylation. After completion of the elongation, an NMP solution of hydrazine monohydrate (5% v/v) was added thereto and reacted for 1 hour to deprotect the ivDde group, and an NMP solution of N-succinimidyl 4,7,10,13-tetraoxatetradecanoate (2% v/v) was added thereto to carry out reaction, whereby a PEG group was introduced into the Lys side chain. After completion of the reaction, the resin was washed with dichloromethane, and then the solvent was distilled off under reduced pressure. 2.0 mL of a mixed solution of TFA:triisopropylsilane:water = 95:2.5:2.5 was added thereto, and the peptide was cut out and deprotected. After 2 hours, the resin was filtered out, and methyl-t-butyl ether (12 mL) was added to the filtrate to generate a solid. The solid was precipitated by centrifugation and then the supernatant was removed. The solid was washed with methyl-t-butyl ether, and then the solvent was distilled off under reduced pressure to obtain 69.5 mg of a white solid compound (7-1). Thereafter, a compound (7) was synthesized in the same manner as in the synthesis of the compound (1), except that the compound (7-1) was used instead of the compound (1-1).

The results of the MS measurement of the compound (7) were as follows.
MS (ESI m/z): $(M + H^+)^+$ = 5,329, $(M - H^+)^-$ = 5,327

The compound (7) - NHS was synthesized from the compound (7) in the same manner as in the synthesis of the compound (1) - NHS, except that the compound (7) was used.

[Synthesis method for compound (8)]

A compound (8) was synthesized according to the following scheme.

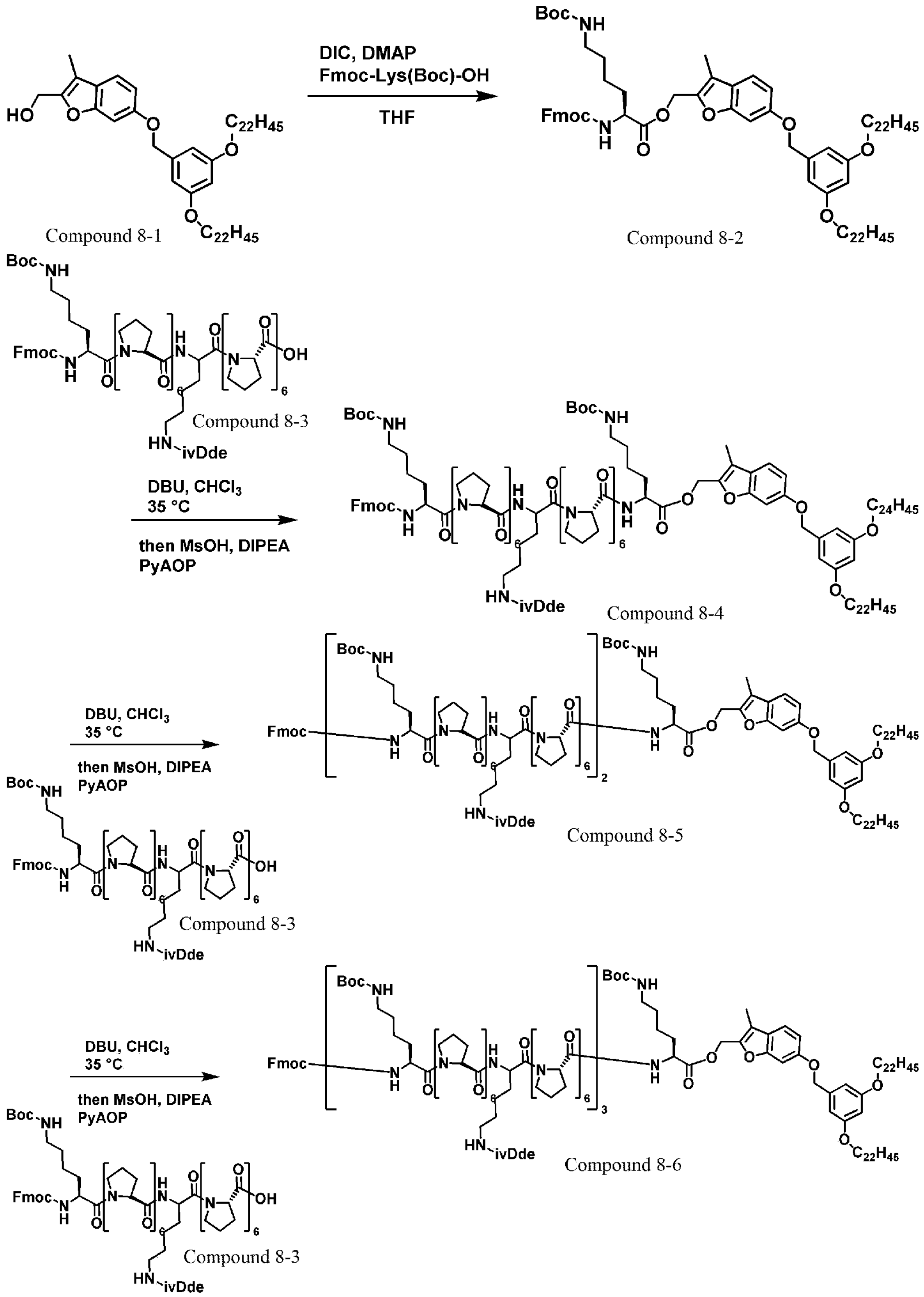


Compound 8-1

Compound 8-2

Compound 8-3

Compound 8-4

Compound 8-5

Compound 8-3

Compound 8-6

Compound 8-3

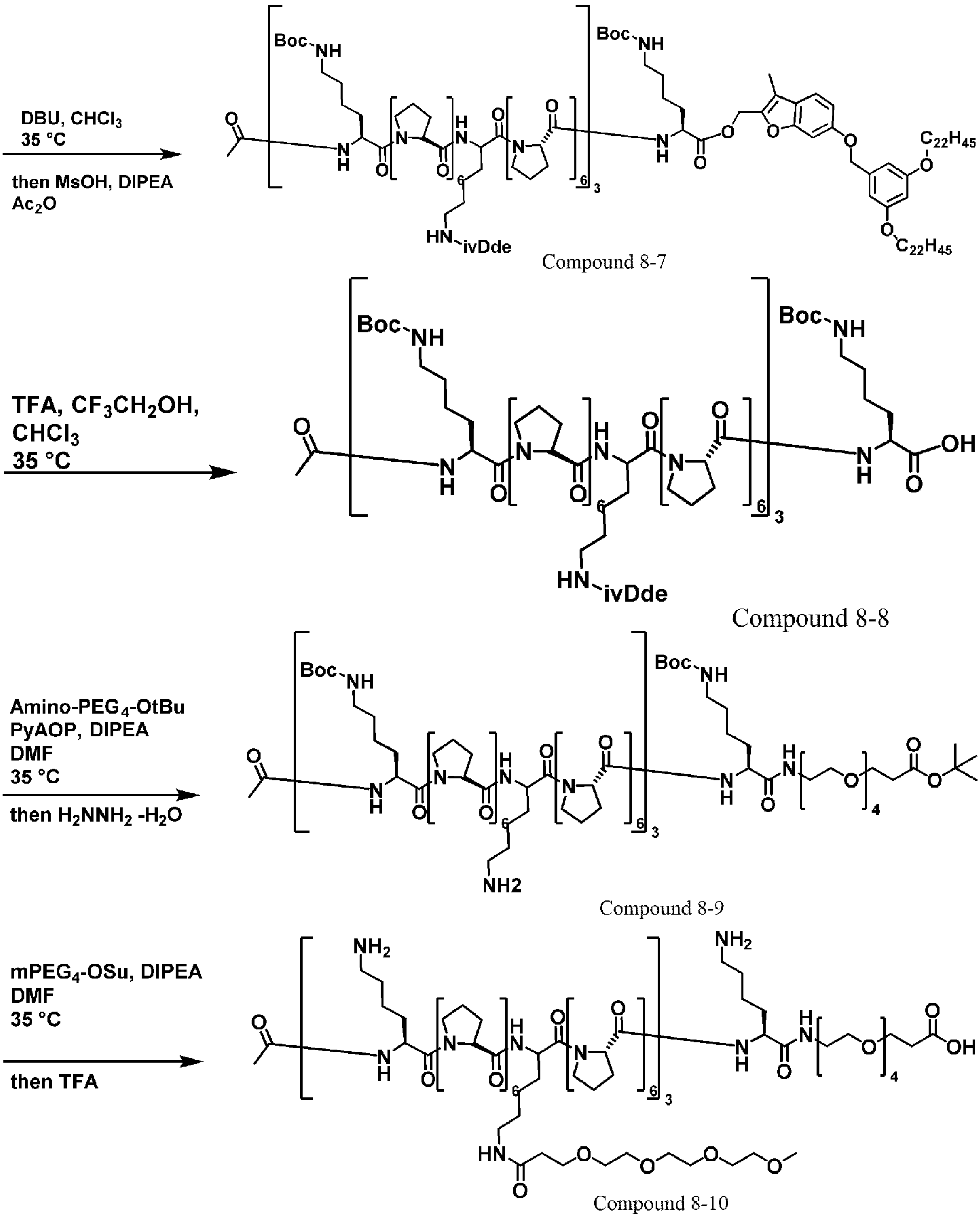


Compound 8-7

Compound 8-8

Compound 8-9

Compound 8-10

1) Synthesis of compound (8-2)

300 mg of the compound (8-1) (the compound (4-1) described in WO2020/175473A), 3 mL of tetrahydrofuran (THF), 229.8 mg of Nε-(tert-butoxycarbonyl)-Nα-[(9H-fluoren-9-ylmethoxy)carbonyl)-L-lysine (Fmoc-Lys(Boc)-OH), 76.8 μL of diisopropylcarbodiimide, and 8.0 mg of 4-dimethylaminopyridine were placed in an eggplant flask having a capacity of 50 mL, and stirring was carried out at room temperature for 2 hours. A solid precipitated by adding acetonitrile (30 mL) was subjected to filtration and drying under reduced pressure to obtain 350 mg of a compound (8-2).

2) Synthesis of compound (8-3)

Solid phase peptide synthesis was carried out using H-Pro-Trt(2-Cl)-Resin (0.94 mmol/g, 53.2 mg) as a starting raw material. The elongation that was carried out by using N-(9-fluorenylmethoxycarbonyl)-L-proline (Fmoc-Pro-OH) was repeated for 5 cycles, and after subjecting Nε-(1-(4,4-dimethyl-2,6-dioxocyclohexa-1-ylidene)-3-methylbutyl)-Nα-[(9H-fluoren-9-ylmet hoxy)carbonyl)-L-lysine (Fmoc-Lys(ivDde)-OH) to elongation, the elongation that was carried out by using N-(9-fluorenylmethoxycarbonyl)-L-proline (Fmoc-Pro-OH) was carried out for 6 cycles, and then Nε-(tert-butoxycarbonyl)-Nα-[(9H-fluoren-9-ylmethoxy)carbonyl)-L-lysine (Fmoc-Lys(Boc)-OH) was subjected to elongation. After completion of the elongation, the resin was washed with dichloromethane, and then the solvent was distilled off under reduced pressure. 2.0 mL of a mixed solution of hexafluoro-2-propanol (HFIP):dichloromethane ($CH_2Cl_2$) = 1:4 was added thereto, and the peptide was cut out. After 30 minutes, the resin was filtered out to concentrate the filtrate, which was subsequently purified by reverse phase column chromatography to obtain 45.2 mg of a white solid compound (8-3).

3) Synthesis of compound (8-4)

175 mg of the compound (8-2), 1.5 mL of chloroform ($CHCl_3$), and 38.2 μL of 1,8-diazabicyclo[5.4.0]-7-undecene (DBU) were placed in an eggplant flask having a capacity of 10 mL, and stirring was carried out at 35°C for 1 hour. 16.5 μL of methanesulfonic acid (MsOH), 133 μL of N,N-diisopropylethylamine (DIPEA), 300 mg of the compound (8-3), and 201 mg of (7-azabenzotriazole-1-yloxy)tripyrrolidinophosphonium hexafluorophosphate (PyAOP) were placed therein, and stirring was carried out at 35°C for 1 hour. Acetonitrile (15 mL) was added thereto to filter the precipitated solid, which was subsequently subjected to drying under reduced pressure to obtain 322 mg of a compound (8-4).

4) Synthesis of compound (8-5)

A compound (8-5) was synthesized in the same manner as in the synthesis of the compound (8-4), except that the compound (8-2) as a raw material was changed to the compound (8-4).

5) Synthesis of compound (8-6)

A compound (8-6) was synthesized in the same manner as in the synthesis of the compound (8-4), except that the compound (8-2) as a raw material was changed to the compound (8-5).

6) Synthesis of compound (8-7)

542 mg of the compound (8-6), 2.7 mL of chloroform ($CHCl_3$), and 25.1 μL of 1,8-diazabicyclo[5.4.0]-7-undecene (DBU) were placed in an eggplant flask having a capacity of 10 mL, and stirring was carried out at 35°C for 1 hour. 10.9 μL of methanesulfonic acid (MsOH), 117 μL of N,N-diisopropylethylamine (DIPEA), and 47.7 μL of acetic anhydride ($Ac_2O$) were placed therein, and stirring was carried out at 35°C for 1 hour. Acetonitrile (15 mL) was added thereto to filter the precipitated solid, which was subsequently subjected to drying under reduced pressure to obtain 322 mg of a compound (8-7).

7) Synthesis of compound (8-8)

48.5 mg of the compound (8-7), 500 μL of chloroform ($CHCl_3$), 50 μL of 2,2,2-trifluoroethanol (TFE), and 5 μL of trifluoroacetic acid (TFA) were placed in an eggplant flask having a capacity of 10 mL, and stirring was carried out at 35°C for 4 hours. Thereafter, the reaction solution was filtered, and a solid precipitated by adding 6 mL of methyl-tert-butyl ether (MTBE) to the filtrate was subjected to filtration and drying under reduced pressure to obtain 29.1 mg of a compound (8-8).

8) Synthesis of compound (8-9)

23 mg of the compound (8-8), 1.2 mL of N,N-dimethylformamide (DMF), 2.1 mg of tert-butyl 1-amino-3,6,9,12-tetraoxadecane-15-ate (Amino-$PEG_4$-OtBu), 6.4 mg of (7-azabenzotriazole-1-yloxy)tripyrrolidinophosphonium hexafluorophosphate (PyAOP), and 4.3 μL of N,N-diisopropylethylamine (DIPEA) were placed in an eggplant flask having a capacity of 10 mL, and stirring was carried out at 35°C for 2 hours. Thereafter, 24.2 μL of hydrazine monohydrate was added thereto, and stirring was carried out at room temperature for 3 hours. After completion of the reaction, the reaction solution was purified by reverse phase column chromatography to obtain 15.4 mg of a white solid compound (8-9).

9) Synthesis of compound (8-10)

34 mg of the compound (8-9), 670 µL of N,N-dimethylformamide (DMF), 11 mg of N-succinimidyl 4,7,10,13-tetraoxatetradecanoate (mPEG$_4$-OSu), and 9.1 µL of N,N-diisopropylethylamine (DIPEA) were placed in an eggplant flask having a capacity of 10 mL, and stirring was carried out at 35°C for 2 hours. After completion of the reaction, the solvent was removed by distillation under reduced pressure, and then 670 µL of trifluoroacetic acid (TFA) was added thereto, and stirring was carried out at room temperature for 2 hours. Thereafter, the reaction solution was concentrated and purified by preparative HPLC, and freeze drying was followed to obtain 13.3 mg of a compound (8-10).

10) Synthesis of compound (8)

A compound (8) was synthesized in the same manner as in the synthesis of the compound (1), except that the compound (8-10) was used instead of the compound (1-1).

The results of the MS measurement of the compound (8) were as follows.

MS (ESI m/z): $(M + H^+)^+$ = 11,610, $(M - H^+)^-$ = 11,608

11) Synthesis of compound (8) - NHS

Further, the compound (8) - NHS was synthesized from the compound (8) in the same manner as in the synthesis of the compound (1) - NHS, except that the compound (8) was used.

[Synthesis method for comparative example compound (1)]

A comparative compound (1) was synthesized according to the following scheme. t-Bu means t-butyl.

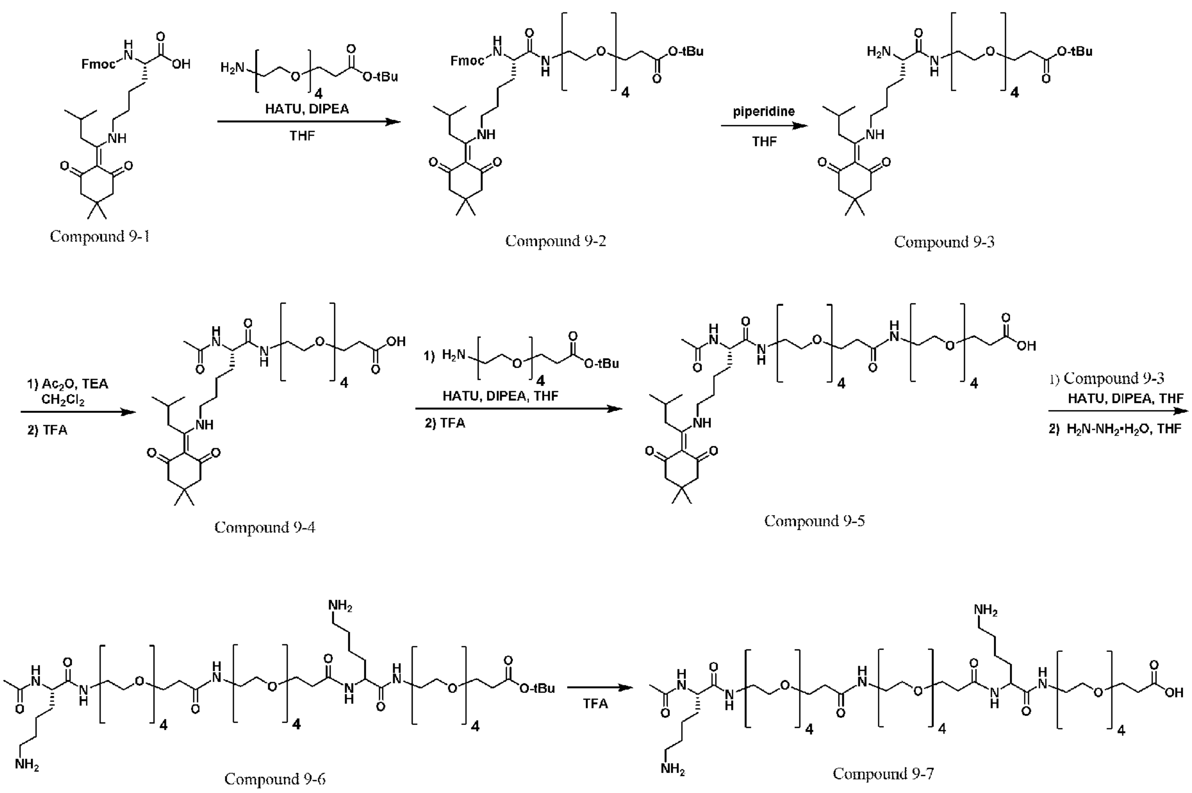

Compound 9-1 Compound 9-2 Compound 9-3 Compound 9-4 Compound 9-5 Compound 9-6 Compound 9-7

1) Synthesis of compound (9-3)

750 mg of Nε-(1-(4,4-dimethyl-2,6-dioxocyclohexa-1-ylidene)-3-methylbutyl)-Nα-[(9H-fluoren-9-ylmethoxy)carbonyl)-L-lysine (Fmoc-Lys(ivDde)-OH) (the compound (9-1)), 15 mL of tetrahydrofuran (THF), 461 mg of tert-butyl 1-amino-3,6,9,12-tetraoxadecane-15-ate, 595 mg of 1-[bis(dimethylamino)methylene]-1H-1,2,3-triazolo[4,5-b]pyridinium 3-oxide hexafluorophosphate (HATU), and 273 µL of N,N-diisopropylethylamine (DIPEA) were placed in an

eggplant flask having a capacity of 50 mL, and stirring was carried out at room temperature for 4 hours. Thereafter, liquid separation was carried out with ethyl acetate and saturated saline, and then the organic layer was distilled off under reduced pressure to obtain a compound (9-2) as a crude product.

1.18 g of the compound (9-2), 7.5 mL of tetrahydrofuran (THF), and 258 μL of piperidine were placed in an eggplant flask having a capacity of 50 mL, and stirring was carried out at room temperature for 12 hours. Thereafter, liquid separation was carried out with ethyl acetate and saturated saline, the organic layer was distilled off under reduced pressure, and the purification was carried out by normal phase column chromatography to obtain 855 mg of a compound (9-3).

2) Synthesis of compound (9-4)

855 mg of the compound (9-3), 7.7 mL of dichloromethane ($CH_2Cl_2$), 83.2 μL of acetic anhydride ($Ac_2O$), and 139 μL of triethylamine (TEA) were placed in an eggplant flask having a capacity of 50 mL, and stirring was carried out at room temperature for 1 hour. Thereafter, liquid separation was carried out with chloroform ($CHCl_3$) and saturated saline, and then the organic layer was distilled off under reduced pressure to obtain a crude product.

The obtained crude product and 3.5 mL of trifluoroacetic acid (TFA) were placed in an eggplant flask having a capacity of 50 mL, and stirring was carried out at room temperature for 30 minutes. Thereafter, the reaction solution was concentrated and purified by reverse phase column chromatography, and freeze drying was followed to obtain 287 mg of a compound (9-4).

3) Synthesis of compound (9-5)

287 mg of the compound (9-4), 5.7 mL of tetrahydrofuran (THF), 172 mg of tert-butyl 1-amino-3,6,9,12-tetraoxadecane-15-ate, 255 mg of 1-[bis(dimethylamino)methylene]-1H-1,2,3-triazolo[4,5-b]pyridinium 3-oxide hexafluorophosphate (HATU), and 117 μL of N,N-diisopropylethylamine (DIPEA) were placed in an eggplant flask having a capacity of 50 mL, and stirring was carried out at room temperature for 4 hours. Thereafter, liquid separation was carried out with ethyl acetate and saturated saline, and then the organic layer was distilled off under reduced pressure to obtain a crude product.

The obtained crude product and 2.5 mL of trifluoroacetic acid (TFA) were placed in an eggplant flask having a capacity of 50 mL, and stirring was carried out at room temperature for 30 minutes. Thereafter, the reaction solution was concentrated and purified by reverse phase column chromatography, and freeze drying was followed to obtain 281 mg of a compound (9-5).

4) Synthesis of compound (9-6)

140 mg of the compound (9-5), 2.8 mL of tetrahydrofuran (THF), 103 mg of the compound (9-3), 71.9 mg of 1-[bis(dimethylamino)methylene]-1H-1,2,3-triazolo[4,5-b]pyridinium 3-oxide hexafluorophosphate (HATU), and 33.0 μL of N,N-diisopropylethylamine (DIPEA), were placed in an eggplant flask having a capacity of 50 mL, and stirring was carried out at room temperature for 4 hours. Thereafter, liquid separation was carried out with ethyl acetate and saturated saline, and then the organic layer was distilled off under reduced pressure to obtain a crude product.

The obtained crude product, 2.8 mL of tetrahydrofuran (THF), and 30.6 μL of hydrazine monohydrate were placed in an eggplant flask having a capacity of 50 mL, and stirring was carried out at room temperature for 12 hours. Thereafter, the reaction solution was concentrated and purified by reverse phase column chromatography, and freeze drying was followed to obtain 152 mg of a compound (9-6).

5) Synthesis of compound (9-7)

108 mg of the compound (9-6) and 3 mL of trifluoroacetic acid (TFA) were placed in an eggplant flask having a capacity of 50 mL, and stirring was carried out at room temperature for 1.5 hours. Thereafter, the reaction solution was concentrated and purified by reverse phase column chromatography, and freeze drying was followed to obtain 100 mg of a compound (9-7).

6) Synthesis of comparative compound (1)

A comparative compound (1) was synthesized in the same manner as in the synthesis of the compound (1), except that the compound (9-7) was used instead of the compound (1-1).

The results of the MS measurement of the comparative compound (1) were as follows.

MS (ESI m/z): $(M + H^+)^+$ = 4,466, $(M - H^+)^-$ = 4,464

7) Synthesis of comparative compound (1) - NHS

A comparative compound (1) - NHS was synthesized from the comparative compound (1) in the same manner as in the synthesis of the compound (1) - NHS, except that the comparative compound (1) was used.

[Synthesis of reference compound (2)]

1) Synthesis of reference compound (2)

A reference compound (2) was synthesized in the same manner as in the synthesis of the compound (1) by using, as raw materials for solid phase peptide synthesis, N-[(9H-fluoren-9-ylmethoxy)carbonyl]glycine (Fmoc-Gly-OH), Nε-(tert-butoxycarbonyl)-Nα-[(9H-fluoren-9-ylmethoxy)carbonyl)-L-lysine (Fmoc-Lys(Boc)-OH) and N-(9-fluorenylmethoxycarbonyl)-L-proline (Fmoc-Pro-OH), except that the peptide chain was subjected to elongation so that the structure of the peptide chain described in the reference compound (2) - NHS was obtained.

The results of the MS measurement of the reference compound (2) were as follows.
MS (ESI m/z): $(M + H^+)^+$ = 4,592, $(M - H^+)^-$ = 4,590

2) Synthesis of reference compound (2) - NHS

A reference compound (2) - NHS was synthesized from the reference compound (2) in the same manner as in the synthesis of the compound (1) - NHS, except that the reference compound (2) was used.

<Example 1>

For each of the above-described compounds, the degree of fluorescence labeling, the solution fluorescence intensity, the fluorescence intensity on the membrane, and the dot blot fluorescence intensity was evaluated as an indicator of suitability in a usage form of an application to a solution and an application to a membrane in a case of being used as a labeling substance.

[0] Preparation of fluorescently labeled antibody

217 μL of an anti-rabbit IgG antibody (2.3 mg/ml) and 21.7 μL of a carbonate buffer were added to a microtube, the resultant mixture was shaken and stirred. Then, a dimethyl sulfoxide solution of the compound (1) - NHS was added thereto so that the equivalent thereof was 15 equivalents with respect to 1 equivalent of the antibody, and the resultant mixture further was shaken and stirred. After being allowed to stand at 4°C for 24 hours, the reaction solution was subjected to purification, by using a centrifugal ultrafiltration filter (product name: Amicon Ultra UFC 510096, manufactured by Merck KGaA) and a PBS solution (phosphate-buffered saline), to obtain an IgG antibody labeled with the compound (1) - NHS. Similarly, an antibody labeled with each of the compounds, the comparative compounds, and the reference compounds was obtained. Hereinafter, the notation of Compound (Z) - IgG, Comparative compound (Z) - IgG, or Reference compound (Z) - IgG respectively mean an IgG antibody labeled with the compound (Z) - NHS, an IgG antibody labeled with the comparative compound (Z) - NHS, or an IgG antibody labeled with the reference compound (Z) - NHS.

The degree of fluorescence labeling (DOL) of the obtained labeled antibody was calculated according to the method described below. The results are summarized in Table A.

As the method for calculating the degree of fluorescence labeling, such a general method as described below was used. The description in [ ] indicates a unit, and [-] means that there is no unit. In the present test, protein means an anti-rabbit IgG antibody.

$$\text{Degree of fluorescence labeling} = \text{fluorescent dye concentration}/\text{protein concentration}$$

The fluorescent dye concentration means the total molar concentration [M] of the labeled fluorescent dye, and the protein concentration means the molar concentration [M] of the fluorescently labeled protein. They are respectively calculated according to the following expressions.

$$\text{Fluorescent dye concentration} = \text{Dye}_{max}/\varepsilon_{dye}$$

$$\text{Protein concentration} = (IgG_{280} - (Dye_{max} \times CF))/\varepsilon_{protein}$$

Each symbol in the above expressions is as follows.

$Dye_{max}$: Absorption [-] of fluorescent dye at maximum absorption wavelength
$\varepsilon_{dye}$: Molar absorption coefficient [$M^{-1}cm^{-1}$] of fluorescent dye
$IgG_{280}$: Absorption [-] of fluorescently labeled protein at 280 nm
$Dye_{280}$: Absorption [-] of fluorescent dye at 280 nm
$\varepsilon_{protein}$. Molar absorption coefficient [$M^{-1}cm^{-1}$] of protein
Correction Factor (CF): $Dye_{280}/Dye_{max}$ [-]

[Table A]

| No. | Labeled antibody | DOL |
|---|---|---|
| 101 | Compound (1) - IgG | 4.3 |
| 102 | Compound (2) - IgG | 4.4 |
| 103 | Compound (3) - IgG | 4.4 |
| 104 | Compound (4) - IgG | 4.2 |
| 105 | Compound (5) - IgG | 4.5 |
| 106 | Compound (6) - IgG | 4.6 |
| 107 | Compound (7) - IgG | 4.4 |
| 108 | Compound (8) - IgG | 4.2 |
| c101 | Comparative compound (1) - IgG | 4.6 |
| r102 | Reference compound (2) - IgG | 4.6 |

[1] Evaluation of solution fluorescence intensity

A solution of the labeled antibody prepared in the section of [0] described above was prepared to have a protein concentration of 0.005 mg/mL, and the integrated value of the fluorescence intensity in a fluorescence wavelength range of 810 to 840 nm was calculated by using a spectroscopic fluorescence intensity meter (product name: RF-5300, manufactured by Shimadzu Corporation) with excitation light of 785 nm and unified the exposure conditions. Using the integrated value of the fluorescence intensity of the comparative compound (1) - IgG having DOL of 4.6 in a fluorescence wavelength range of 810 to 840 nm as the reference value, the ratio to this reference value (the integrated value of the fluorescence intensity of the labeled antibody in a fluorescence wavelength range of 810 to 840 nm/the reference value) was calculated, and evaluation was carried out based on the following evaluation standards. The results are summarized in Table 1.

- Evaluation standards for solution fluorescence intensity -

A: The ratio of fluorescence intensity to the reference value is 2.0 times or more.
B: The ratio of fluorescence intensity to the reference value is 1.5 times or more and less than 2.0 times.
C: The ratio of fluorescence intensity to the reference value is 1.3 times or more and less than 1.5 times.
D: The ratio of fluorescence intensity to the reference value is 1.1 times or more and less than 1.3 times.
E: The ratio of fluorescence intensity to the reference value is less than 1.1 times.

[2] Evaluation of fluorescence intensity on membrane

The anti-rabbit IgG solution was prepared to have a protein concentration of 5.0 ng/mL, and 2 μL thereof was carefully spotted on a nitrocellulose membrane. The membrane was dried and then blocked with a Fish Gelatin blocking

buffer solution in Tris Buffered Saline with Tween 20 (TBS-T). The membrane was incubated at room temperature for 1 hour with stirring. The blocking solution was removed, and the PBS solution of the labeled antibody (the solution of the labeled antibody prepared in the section of [0] described above, concentration before dilution: 1 mg/mL) was diluted 20,000 times with Tris Buffered Saline (TBS). The membrane was immersed in the diluted solution and incubated for 1 hour with stirring. The membrane was washed three times with TBS-T for 10 minutes and finally washed with TBS for 10 minutes. The obtained membrane was dried on a hot plate at 40°C for 1 hour and imaged using an Amersham Typhoon scanner (manufactured by GEHC) with excitation light of 785 nm under the uniform exposure conditions, thereby calculating the fluorescence intensity in a fluorescence wavelength range of 810 to 840 nm. Using the integrated value of the fluorescence intensity of the comparative compound (1) - IgG having DOL of 4.6 in a fluorescence wavelength range of 810 to 840 nm as the reference value, the ratio to this reference value (the integrated value of the fluorescence intensity of the labeled antibody in a fluorescence wavelength range of 810 to 840 nm/the reference value) was calculated, and evaluation was carried out based on the following evaluation standards. The results are summarized in Table 1.

- Evaluation standards for fluorescence intensity on membrane -

A: The ratio of fluorescence intensity to the reference value is 2.0 times or more.
B: The ratio of fluorescence intensity to the reference value is 1.5 times or more and less than 2.0 times.
C: The ratio of fluorescence intensity to the reference value is 1.3 times or more and less than 1.5 times.
D: The ratio of fluorescence intensity to the reference value is 1.1 times or more and less than 1.3 times.
E: The ratio of fluorescence intensity to the reference value is less than 1.1 times.

[3] Evaluation of dot blot fluorescence intensity

Transferrin (20 mg/mL) was prepared to 50 ng/mL with Phosphate-buffered saline with Tween 20 (TBS-T), and 2 μL thereof was carefully spotted on a nitrocellulose membrane. The membrane was dried and then blocked in TBS-T with a Fish Gelatin blocking buffer solution. Subsequently, 6 μL of a polyclonal rabbit anti-human transferrin antibody was added to 30 mL of PBS-T, the membrane was immersed therein, and shaking was carried out for 1 hour. Then, the membrane was taken out and washed with TBS-T four times. Thereafter, 15 μL of a solution obtained by adjusting the solution of the labeled antibody (anti-rabbit IgG) prepared in the section of [0] described above to a protein concentration of 0.1 mg/mL was added to 30 mL of TBS-T, the membrane was immersed therein, and incubation was carried out at room temperature for 1 hour with stirring. The membrane was washed three times with TBS-T for 10 minutes/washing and finally washed with TBS for 10 minutes. The obtained membrane was dried on a hot plate at 40°C for 1 hour and imaged using an Amersham Typhoon scanner (manufactured by GEHC) with excitation light of 785 nm under the uniform exposure conditions, thereby calculating the fluorescence intensity in a fluorescence wavelength range of 810 to 840 nm. Using the integrated value of the fluorescence intensity of the comparative compound (1) - IgG having DOL of 4.6 in a fluorescence wavelength range of 810 to 840 nm as the reference value, the ratio to this reference value (the integrated value of the fluorescence intensity of the labeled antibody in a fluorescence wavelength range of 810 to 840 nm/the reference value) was calculated, and evaluation was carried out based on the following evaluation standards. The results are summarized in Table 1.

- Evaluation standards for dot blot fluorescence intensity -

A: The ratio of fluorescence intensity to the reference value is 2.0 times or more.
B: The ratio of fluorescence intensity to the reference value is 1.5 times or more and less than 2.0 times.
C: The ratio of fluorescence intensity to the reference value is 1.3 times or more and less than 1.5 times.
D: The ratio of fluorescence intensity to the reference value is 1.1 times or more and less than 1.3 times.
E: The ratio of fluorescence intensity to the reference value is less than 1.1 times.

[Table 1]

| NO. | | Solution fluorescence intensity | Fluorescence intensity on membrane | Dot blot fluorescence intensity |
|---|---|---|---|---|
| 101 | Compound (1) - IgG | D | D | D |

(continued)

| NO. | | Solution fluorescence intensity | Fluorescence intensity on membrane | Dot blot fluorescence intensity |
|---|---|---|---|---|
| 102 | Compound (2) - IgG | D | D | C |
| 103 | Compound (3) - IgG | D | C | C |
| 104 | Compound (4) - IgG | C | C | C |
| 105 | Compound (5) - IgG | B | C | C |
| 106 | Compound (6) - IgG | B | B | C |
| 107 | Compound (7) - IgG | B | B | B |
| 108 | Compound (8) - IgG | A | A | A |
| c101 | Comparative compound (1) - IgG | E (reference) | E (reference) | E (reference) |
| r102 | Reference compound (2) - IgG | D | D | E |

From the results in Table 1 above, the following facts can be seen.

The comparative compound (1) - NHS is not the compound defined in the present invention in that it does not have $A^1$ and $A^2$ of the structure (-$A^1$-B-$A^2$-) represented by General Formula (I), and the reference compound (2) - NHS is not the compound defined in the present invention in that it does not have B of the structure (-$A^1$-B-$A^2$-) represented by General Formula (I).is not the compound defined in the present invention. The comparative compound (1) - NHS made it possible to obtain only a labeled antibody having a low fluorescence intensity in the solution, a low fluorescence intensity on the membrane, and a low fluorescence intensity in the dot blot (No. c101). The reference compound (2) - NHS, made it possible to obtain only a labeled antibody having a fluorescence intensity in a dot blot state of less than 1.1 times with respect to the fluorescence intensity of the antibody labeled with the comparative compound (1) - NHS (No. r102).

On the other hand, all of the compounds (1) - NHS to (8) - NHS defined in the present invention made it possible to prepare a labeled antibody that has a fluorescence intensity of 1.1 times or more with respect to the fluorescence intensity of the antibody labeled with the comparative compound (1) - NHS, in any state of being in the solution, the membrane, or the dot blot and exhibits an excellent fluorescence intensity (Nos. 101 to 108 with respect to No. c101). In particular, in a case of comparing the compound (2) -NHS with the reference compound (2) - NHS having a structure similar to the structure (-$A^1$-B-$A^2$-) represented by General Formula (I) except that B of the structure (-$A^1$-B-$A^2$-) is not contained, it has been found that the fluorescence intensity in the dot blot state is less than 1.1 times for the reference compound (2) - NHS, whereas it is 1.3 times or more for the compound (2) - NHS, and thus an excellent fluorescence intensity is exhibited in the dot blot state due to having the structure of B in General Formula (I) (No. 102 with respect to No. r102).

Hereinafter, a speculation will be given focusing on the compounds (the compound (2) - NHS to the compound (8) - NHS) having structures derived from lysine on both sides of the structure represented by General Formula (I).

The compound (3) - NHS to the compound (8) - NHS in which l or n in General Formula (I) is 6 made it possible to prepare a labeled antibody that exhibits a fluorescence intensity of 1.3 times or more in states of being in a membrane and a dot blot (Nos. 103 to 108 with respect to No. 102).

The compound (4) -NHS to the compound (8) -NHS, in which l or n in General Formula (I) is 6 and $L^1$ in General Formula (b) is a linking group in which the number of shortest-distance atoms that connect >$NY^1$ to >C=O is 1, made it possible to prepare a labeled antibody that exhibits a fluorescence intensity of 1.3 times or more in any states of being in a solution, a membrane, and a dot blot (Nos. 104 to 108 with respect to No. 103).

The compound (5) - NHS having an anionic group made it possible to prepare a labeled antibody that exhibits a fluorescence intensity of 1.5 times or more in a state of being a solution (No. 105 with respect to No. 104).

The compound (6) - NHS in which p is 2 in General Formula (I) made it possible to prepare a labeled antibody that exhibits a fluorescence intensity of 1.5 times or more in states of being in a solution and a membrane (No. 106 with respect to No. 104).

The compound (7) - NHS having a polyalkyleneoxy group made it possible to prepare a labeled antibody that exhibits a fluorescence intensity of 1.5 times or more in any of a solution, a membrane, and a dot blot (No. 107 with respect to No. 104).

The compound (8) - NHS having a polyalkyleneoxy group and four phosphor moieties has phosphor moieties that is twice as more as the compound (7) - NHS having two phosphor moieties. The compound (8) - NHS makes it possible to prepare a labeled antibody that exhibits an intensity of about 1.5 times with respect to the compound (7) - NHS. In general, the association is more likely to occur as the number (concentration) of phosphor moieties increases, and thus it is difficult to obtain a fluorescence intensity proportional to the number of phosphor moieties. However, it has been found that even in a case where the number of phosphor moieties in the compound is increased, the association of phosphor moieties is effectively suppressed in the compound according to the embodiment of the present invention as in the case where the number of phosphor moieties is small, whereby an excellent fluorescence intensity is exhibited.

The present invention has been described together with the embodiments of the present invention. However, the inventors of the present invention do not intend to limit the present invention in any part of the details of the description unless otherwise specified, and it is conceived that the present invention should be broadly construed without departing from the spirit and scope of the invention shown in the attached "WHAT IS CLAIMED IS".

This application claims priority based on JP2021-141996 filed in Japan on August 31, 2021, which is incorporated herein by reference as a part of the description of the present specification.

## Claims

1. A compound comprising:

    a phosphor moiety; and
    a structure represented by General Formula (I),

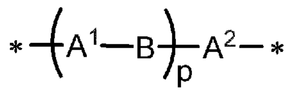
General Formula (I)

in the formula, $A^1$ represents a structure represented by General Formula (a1),
$A^2$ represents a structure represented by General Formula (a2), and
B represents a structure represented by General Formula (b),

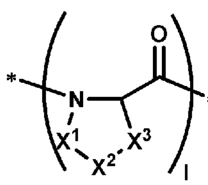
General Formula (a1)
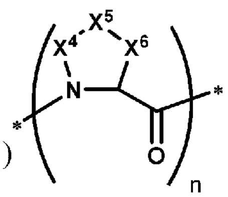
General Formula (a2)

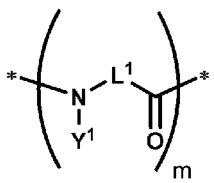
General Formula (b)

in the formulae, $X^1$ to $X^6$ represent -O-, -S-, >$NR^1$, or >$CR^2R^3$,
$R^1$ to $R^3$ represent a hydrogen atom, an alkyl group, an alkenyl group, an alkynyl group, an acyl group, an aryl group, a heteroaryl group, -$NR^9R^{10}$, -$OR^{11}$, or an anionic group,
$R^9$ to $R^{11}$ represent a hydrogen atom, an alkyl group, an alkenyl group, an alkynyl group, an acyl group, an aryl group, or a heteroaryl group,
$Y^1$ represents a hydrogen atom, an alkyl group, an alkenyl group, an alkynyl group, an aryl group, or a heteroaryl group,
$L^1$ represents a divalent linking group,
l, m, n, and p are an integer of 1 or more, and
* represents a bonding site.

2. The compound according to claim 1,

wherein the compound is represented by General Formula (II),

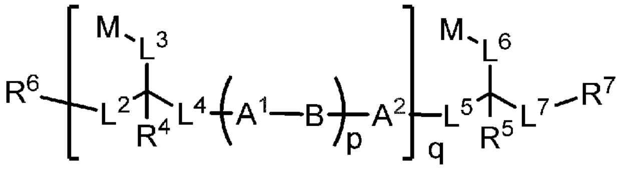

General Formula (II)

in the formula, $R^4$ and $R^5$ represent a hydrogen atom, an alkyl group, an alkenyl group, an alkynyl group, an aryl group, or a heteroaryl group,
$R^6$ and $R^7$ represent a hydrogen atom, a hydroxy group, a sulfanyl group, an alkyl group, an alkenyl group, an alkynyl group, an aryl group, an alkoxy group, an amino group, an acyl group, a heteroaryl group, an anionic group, a cationic group, $-(L-O)_tR^E$, or Q, where L represents an alkylene group, $R^E$ represents a hydrogen atom or an alkyl group, t is 1 to 24, and Q represents a carboxy group, a substituent capable of being bonded to a biological substance, or a substituent capable of being bonded to a solid support,
$L^2$ to $L^7$ represents a single bond or a divalent linking group,
M represents a phosphor moiety, a physiologically active substance moiety, a prodrug moiety, or a radioactive isotope-containing moiety,
q is an integer of 1 or more, and
$A^1$, $A^2$, B, and p have the same meanings as $A^1$, $A^2$, B, and p described above,
provided that at least one of M's represents a phosphor moiety.

**3.** The compound according to claim 1 or 2,
wherein at least one of l or n described above is an integer of 3 or more.

**4.** The compound according to any one of claims 1 to 3,
wherein the $L^1$ is a linking group in which the number of shortest-distance atoms that connect $>NY^1$ to $>C=O$ in General Formula (b) is 1 to 4.

**5.** The compound according to any one of claims 1 to 4,
wherein the $L^1$ is a linking group including a ring structure.

**6.** The compound according to claim 5,
wherein the ring structure is a group having a polyalkyleneoxy group as a substituent.

**7.** The compound according to any one of claims 1 to 6,
wherein a ClogP value of the structure represented by General Formula (b) is 1.0 or less.

**8.** The compound according to any one of claims 1 to 7,

wherein the B is a structure represented by General Formula (c),

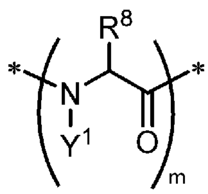

General Formula (c)

in the formula, $R^8$ represents a hydrogen atom or an alkyl group,
$Y^1$ and m respectively have the same meanings as $Y^1$ and m described above, and
* represents a bonding site.

**9.** The compound according to claim 8,
wherein $R^8$ is an alkyl group having, as a substituent, any one of an anionic group, a cationic group, or a polyalkyleneoxy group.

**10.** The compound according to any one of claims 1 to 9, wherein the p is an integer of 1.

**11.** The compound according to claim 9, wherein the $R^8$ is an alkyl group having a polyalkyleneoxy group as a substituent.

**12.** A labeled biological substance that is obtained by bonding the compound according to any one of claims 1 to 11 to a biological substance.

**13.** The labeled biological substance according to claim 12, wherein the biological substance is any one of a protein, an amino acid, a nucleic acid, a nucleotide, a sugar chain, or a phospholipid.

# INTERNATIONAL SEARCH REPORT

International application No.
**PCT/JP2022/032639**

**A. CLASSIFICATION OF SUBJECT MATTER**

***C07K 2/00***(2006.01)i; ***A61K 47/54***(2017.01)i; ***A61K 49/00***(2006.01)i; ***C07K 16/00***(2006.01)i; ***C09K 11/06***(2006.01)i; ***G01N 33/533***(2006.01)i
FI: C07K2/00; A61K47/54; A61K49/00; G01N33/533; C09K11/06; C07K16/00

According to International Patent Classification (IPC) or to both national classification and IPC

**B. FIELDS SEARCHED**

Minimum documentation searched (classification system followed by classification symbols)

C07K2/00; A61K47/54; A61K49/00; C07K16/00; C09K11/06; G01N33/533

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/MEDLINE/EMBASE/BIOSIS (STN); UniProt/GeneSeq; SwissProt/GeneSeq

**C. DOCUMENTS CONSIDERED TO BE RELEVANT**

| Category* | Citation of document, with indication, where appropriate, of the relevant passages | Relevant to claim No. |
|---|---|---|
| X | JP 2003-185655 A (WARNER LAMBERT CO., LLC) 03 July 2003 (2003-07-03) claims, paragraphs [0004], [0058]-[0061], [0073], seq. ID no. 3 | 1-5, 7-10, 12, 13 |
| Y | claims, paragraphs [0004], [0058]-[0061], [0073], SEQ ID no. 3 | 6, 11 |
| Y | JP 2019-531474 A (LIFE TECHNOLOGIES CORP.) 31 October 2019 (2019-10-31) paragraphs [0004], [0023], [0049] | 6, 11 |
| Y | WO 2013/005603 A1 (PUBLIC UNIVERSITY CORPORATION YOKOHAMA CITY UNIV.) 10 January 2013 (2013-01-10) paragraph [0034] | 6, 11 |
| X | JP 2000-506522 A (CYTOGEN CORP.) 30 May 2000 (2000-05-30) claims, pages 19, 46, seq. ID no. 178, 181 | 1-5, 7-10, 12, 13 |
| Y | claims, pages 19, 46, seq. ID no. 178, 181 | 6, 11 |
| X | JP 2003-517300 A (ACGT PROGENOMICS AG) 27 May 2003 (2003-05-27) claims, paragraphs [0015], [0037], seq. ID no. 15 | 1-5, 7-10, 12, 13 |
| Y | claims, paragraphs [0015], [0037], seq. ID no. 15 | 6, 11 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **01 November 2022** | **15 November 2022** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**
**Information on patent family members**

International application No.
**PCT/JP2022/032639**

| Patent document cited in search report | Publication date (day/month/year) | Patent family member(s) | Publication date (day/month/year) |
|---|---|---|---|
| JP 2003-185655 A | 03 July 2003 | US 2003/0108975 A1<br>claims, paragraphs [0006], [0108]-[0111], [0123], seq. ID no. 3<br>EP 1281962 A1 | |
| JP 2019-531474 A | 31 October 2019 | US 2018/0092993 A1<br>paragraphs [0005], [0050], [0076]<br>WO 2018/045278 A1<br>EP 3507603 A1<br>CN 109844538 A | |
| WO 2013/005603 A1 | 10 January 2013 | (Family: none) | |
| JP 2000-506522 A | 30 May 2000 | US 2002/0091085 A1<br>claims, paragraphs [0002], [0096], seq. ID no. 178, 181<br>WO 1997/030074 A1<br>EP 772773 A1 | |
| JP 2003-517300 A | 27 May 2003 | US 2006/0252130 A1<br>claims, paragraphs [0020], [0028], seq. ID no. 15<br>WO 2001/032684 A2<br>EP 1227848 A2<br>CN 1390140 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2017512763 A
- JP 2010512400 A
- WO 2019230963 A
- WO 2021100814 A
- JP 2021020956 A
- JP 2020105187 A
- JP 2021011483 A
- WO 2002026891 A
- WO 2018174078 A
- JP 2019172826 A
- WO 2020175473 A
- JP 2021141996 A


### Non-patent literature cited in the description


- **GREG T. HERMANSON.** Bioconjugate Techniques
- **LUCAS C. D. DE REZENDE ; FLAVIO DA SILVA EMERY.** A Review of the Synthetic Strategies for the Development of BODIPY Dyes for Conjugation with Proteins. *Orbital: The Electronic Journal of Chemistry,* 2013, vol. 5 (1), 62-83